# EUROPEAN PATENT APPLICATION

(11) **EP 4 692 074 A1**
(43) Date of publication of application: **11.02.2026**
(21) Application number: 24784413.7
(22) Date of filing: 03.04.2024
(51) Int. Cl.: C07D 401/14, C07D 471/04, A61K 31/502, A61K 31/4427, A61P 35/00, A61P 35/02

(54) **CRBN LIGAND, PREPARATION METHOD THEREFOR, AND APPLICATION**

(30) Priority: 04.04.2023 CN 202310392633
(71) Applicant: Shenzhen Zhongge Biological Technology Co., Ltd., Shenzhen, Guangdong 518017 (CN)
(72) Inventor: MA, Junjun, Shenzhen, Guangdong 518017 (CN); JI, Fanyang, Shenzhen, Guangdong 518017 (CN); DU, Xinming, Shenzhen, Guangdong 518017 (CN); CHEN, Zhaoqiang, Shenzhen, Guangdong 518017 (CN); DU, Lifei, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Rückerl, Florian
(86) International application number: PCT/CN2024/086069
(87) International publication number: WO 2024/208331

(57) **Abstract**

Provided are a compound or an enantiomer, diastereoisomer, racemate, tautomer, stereoisomer, geometric isomer, nitrogen oxide, or metabolite thereof, or a pharmaceutically acceptable salt, ester, solvate, hydrate, isotope-labeled compound, or prodrug thereof, as well as an application thereof in the treatment of a CRBN-mediated disease or disorder.

## Description

### CROSS-REFERENCE TO RELATED APPLICATIONS

The present application claims priority to Chinese Patent Application No. 2023103926330 filed on April 4, 2023, the entire content of which is hereby incorporated by reference as part of the present application.

### TECHNICAL FIELD

The present disclosure relates to a CRBN ligand, a preparation method therefor, and use thereof.

### BACKGROUND

The ubiquitin-proteasome system (UPS) is the primary way for cells to regulate protein levels. It is involved in the degradation of more than 80% of intracellular proteins and is responsible for degrading damaged, misfolded, or excessive proteins in cells. In this system, multiple ubiquitin molecules mark substrate proteins through covalent linkage of E3 ubiquitin ligase to terminal lysine residues, forming ubiquitination chains, thereby enabling the substrate proteins to be recognized and hydrolyzed by the proteasome.

PROTAC (proteolysis targeting chimera) is a drug development technology that utilizes the ubiquitin-proteasome system (UPS) to degrade target proteins. Structurally, PRTOTAC consists of three parts: a target protein ligand (POI), a linker, and an E3 ubiquitin ligase ligand. Currently, over 600 E3 ubiquitin ligases have been identified in the human genome, primarily belonging to four families: RING (really interesting new gene) finger, HECT (homologous to E6-AP carboxyl terminus), RBR (RING-between-RING), and RCR (RING-Cys-Relay). Among them, the RING family is the most widely expressed ubiquitin ligase in the human body. At present, the types of E3 ligase ligands used in clinical practice for PROTACs are limited, primarily confined to CRBN and VHL ligands. Moreover, the E3 ubiquitinase CRBN, which is relatively easier to achieve oral administration, has few types of ligands available for clinical application. Therefore, it is necessary to develop novel CRBN ligands. The present disclosure focuses on the development of novel smallmolecule ligands targeting CRBN.

Cereblon (CRBN) is a multifunctional protein consisting of 442 amino acids, which is highly conserved from plants to humans. It primarily interacts with damaged DNA binding protein-1 (DDB1), Cullin 4 (Cul4A or Cul4B), and regulator of Cullins 1 (RoC1) to form a functional E3 ubiquitin ligase complex (CRL4-CRBN-E3 ubiquitin ligase complex). CRBN functions as a substrate receptor of the E3 ubiquitin ligase complex, mediating the ubiquitination and degradation of proteins. As an important target for anti-tumor drugs and immunomodulatory drugs (IMiDs), CRBN has been confirmed to exhibit clear therapeutic efficacy in various hematological malignancies such as multiple myeloma (MM) and chronic lymphocytic leukemia (CLL), as well as autoimmune diseases such as systemic lupus erythematosus (SLE). Currently, there are three CRBN-targeting compounds on the market, including thalidomide, lenalidomide, and pomalidomide. Compounds in clinical research and development include CC-122, CC-220, CC-90009, CC-92480, and the like, all of which are derivatives of domide compounds. However, domide compounds may cause side effects such as neutropenia, thrombocytopenia, and peripheral neuropathy. Therefore, there is an urgent need to develop structurally novel CRBN ligands. Furthermore, since CRBN is a commonly used ubiquitinase in PROTACs, developing structurally novel CRBN ligands can further enhance the clinical therapeutic ability of PROTAC molecules, offering excellent application prospects.

### SUMMARY

In order to solve the aforementioned technical problems existing in the prior art, the present disclosure provides a CRBN ligand for forming PROTAC molecules for the treatment of CRBN-mediated diseases or disorders. This ligand has a high activation rate and shows good druggability.

In one aspect, the present disclosure provides a compound having a structure represented by formula I, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 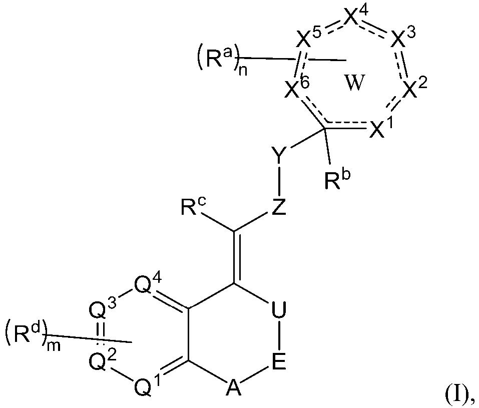 wherein
X¹, X², X³, X⁴, X⁵, and X⁶ are each independently a bond, carbon, nitrogen, oxygen, or sulfur; ring W is a 5-, 6-, or 7-membered ring;
the - - - - - - bonds connected to X¹, X², X³, X⁴, X⁵, and X⁶ each independently represent a single bond or a double bond; two - - - - - - bonds connected to the same atom are not simultaneously double bonds; when X¹, X², X³, X⁴, X⁵, or X⁶ is oxygen or sulfur, the - - - - - - bonds connected thereto are single bonds;
each R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3-to 6-membered heterocyclylamino; optionally, two R^{a} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, and the 3- to 7-membered monocyclic carbocycle or 3- to 7-membered monocyclic heterocycle formed by the connection of two R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
n is 0, 1, 2, 3, 4, 5, 6, 7, 8, 9, 10, 11, or 12;
R^{b} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, or 3- to 6-membered heterocyclyl; optionally, R^{b} and R^{a} are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, and the 3- to 7-membered monocyclic carbocycle or 3-to 7-membered monocyclic heterocycle formed by the connection of R^{b} and R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Y is a bond, NH, CH₂, CH₂CH₂, NHCH₂, or CH₂NH; optionally, Y is substituted with 1, 2, 3, or 4 R^{Y}; each R^{Y} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, and 5- to 10-membered heteroaryl are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Z is NH, O, S, or CH₂; optionally, Z is substituted with 1 or 2 R^{Z}; each R^{Z} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, R^{Z} and R^{a}, or R^{Z} and R^{b}, are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, 5- to 10-membered heteroaryl, and the 3- to 7-membered monocyclic carbocycle or 3- to 7-membered monocyclic heterocycle formed by the connection of R^{Z} and R^{b} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R^{c} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
U is CH₂, NH, O, or S;
E and A are each independently a bond, CH₂, NH, O, or S, wherein E and A are not simultaneously a bond, NH, or O;
optionally, U, E, and A are each independently substituted with 1 or 2 R^{e}; each R^{e} is independently hydrogen, deuterium, halogen, cyano, hydroxyl, amino, oxo (O=), thio (S=), C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, two R^{e} attached to the same carbon atom, together with the atom to which they are attached, form a 3- to 7-membered monocyclic carbocycle or a 3- to 7-membered monocyclic heterocycle; the C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
Q¹, Q², Q³, and Q⁴ are each independently CH or N;
each R^{d} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-; optionally, two adjacent R^{d} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, C₆₋₁₀ aryl S(=O)₂NH-, and the 3- to 7-membered monocyclic carbocycle or 3- to 7-membered monocyclic heterocycle formed by the connection of two adjacent R^{d} are each independently optionally substituted with 1, 2, 3, 4, or 5 R^{d1}, each R^{d1} being independently selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, 3- to 6-membered heterocyclyl, and C₁₋₆ alkyl OC(=O)-; optionally, two R^{d1} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle formed by the connection of two R^{d1} is optionally substituted with 1, 2, 3, 4, or 5 substituents selected from hydrogen, deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
m is 0, 1, 2, 3, or 4.

In another aspect, the present disclosure provides a PROTAC molecule, which comprises a ligand capable of binding to CRBN, wherein the ligand comprises or is based on the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure.

In another aspect, the present disclosure provides a pharmaceutical composition, which comprises: the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure; and at least one pharmaceutically acceptable carrier.

In another aspect, the present disclosure provides the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure, or the pharmaceutical composition of the present disclosure for use in treating a CRBN-mediated disease or disorder.

In another aspect, the present disclosure provides use of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure, or the pharmaceutical composition of the present disclosure in the preparation of a medicament, wherein the medicament is used for treating a CRBN-mediated disease or disorder.

In another aspect, the present disclosure provides a method for treating a CRBN-mediated disease or disorder, which comprises administering to a human in need thereof a therapeutically effective amount of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure, or the pharmaceutical composition of the present disclosure.

Another aspect of the present disclosure relates to methods for the preparation, separation, and purification of compounds represented by formulas I, II-1, II-2, and III.

Any embodiment of any aspect of the present disclosure may be combined with other embodiments, provided that they do not contradict each other. Furthermore, in any embodiment of any aspect of the present disclosure, any technical feature may be applicable to the same technical feature in other embodiments, provided that they do not contradict each other.

The foregoing description merely summarizes certain aspects of the present disclosure, but is not intended to limit such aspects. A more detailed and complete description of these and other aspects will be provided below. All references in this specification are incorporated herein by reference in their entirety.

### DETAILED DESCRIPTION

In order to make the objectives, technical solutions, and advantages of the present disclosure more apparent, the present disclosure is further described in detail with reference to the following examples. The specific examples described herein are merely illustrative of the present disclosure and do not constitute any limitation thereon. Furthermore, in the following description, descriptions of well-known structures and techniques are omitted so as to avoid unnecessarily obscuring the concepts of the present disclosure. Such structures and techniques are also described in numerous publications.

### Definitions

Certain embodiments of the present disclosure will now be described in detail, and examples of the embodiments are illustrated by the accompanying structural and chemical formulas. The present disclosure is intended to cover all alternatives, modifications, and equivalent technical solutions, which are included within the scope of the present disclosure as defined by the claims. Those skilled in the art should recognize that many methods and materials similar or equivalent to those described herein can be used in the practice of the present disclosure. The present disclosure is in no way limited to the methods and materials described herein. In the event that one or more of the incorporated literature, patents, and similar materials differ from or contradict the present application (including but not limited to defined terms, term usage, described techniques, etc.), the present application prevails.

It should be further recognized that certain features of the present disclosure, which are, for clarity, described in the context of separate embodiments, may alternatively be provided in combination in a single embodiment. Conversely, various features of the present disclosure, which are, for brevity, described together in a single embodiment, may instead be provided separately or in any suitable sub-combination.

Unless otherwise stated, all scientific and technical terms as used herein have the same meaning as commonly understood by those skilled in the art to which the present disclosure pertains. All patents and publications referred to herein are incorporated herein by reference in their entirety.

Unless otherwise stated, the following definitions as used herein should be applied. For the purposes of the present disclosure, chemical elements are in accordance with the Periodic Table of Elements, CAS version, and Handbook of Chemistry and Physics, 75th edition, 1994. In addition, for general principles of organic chemistry, reference can be made to the descriptions in Organic Chemistry, Thomas Sorrell, University Science Books, Sausalito: 1999, and March's Advanced Organic Chemistry by Michael B. Smith and Jerry March, John Wiley & Sons, New York: 2007, which are incorporated herein by reference in their entirety.

Unless otherwise specified or clearly contradicted by the context, the articles "a", "an", and "the" as used herein are intended to include "at least one" or "one or more". Thus, these articles as used herein refer to articles for one or more than one (i.e., at least one) object. For example, "a component" means one or more components, meaning that more than one component may be contemplated for adoption or use in the implementation of the stated embodiment.

The term "subject" refers to an animal. Typically, the animal is a mammal. The subject also refers to, for example, primates (e.g., humans, male or female), cattle, sheep, goats, horses, dogs, cats, rabbits, rats, mice, fish, birds, and the like. In certain embodiments, the subject is a primate. In other embodiments, the subject is a human.

The term "patient" refers to humans (including adults and children) or other animals. In some embodiments, "patient" refers to a human.

The term "comprise", "comprises", or "comprising" is an open-ended expression, i.e., including what is meant by the present disclosure, but not excluding other aspects.

When a substituent is described by a general formula written from left to right, the substituent also includes chemically equivalent substituents that are obtained when the structural formula is written from right to left. For example, -CH₂O- is equivalent to -OCH₂-.

The term "enantiomer" refers to two isomers of a compound that are non-superimposable but are mirror images of each other.

The term "diastereoisomer" refers to stereoisomers that have two or more chiral centers and whose molecules are not mirror images of one another. Diastereoisomers have different physical properties, such as melting points, boiling points, spectral properties, and reactivities. A mixture of diastereoisomers can be resolved through high-resolution analytical procedures such as electrophoresis and chromatography (e.g., HPLC).

The term "racemate" or "racemic mixture" refers to an equimolar mixture of two enantiomers that lacks optical activity.

The term "tautomer" or "tautomeric form" refers to structural isomers having different energies that are interconvertible via a low energy barrier. If tautomerism is possible (e.g., in a solution), a chemical equilibrium of tautomers can be achieved. For example, proton tautomers (also known as prototropic tautomers) involve interconversion through proton migration, such as keto-enol isomerization and imine-enamine isomerization. Valence tautomers involve interconversion through reorganization of some bonding electrons. A specific example of keto-enol tautomerization is the interconversion between pentane-2,4-dione and 4-hydroxypent-3-en-2-one tautomers. Another example of tautomerization is phenol-keto tautomerization. A specific example of phenol-keto tautomerization is the interconversion between pyridin-4-ol and pyridin-4(1H)-one tautomers. Unless otherwise stated, all tautomeric forms of the compound of the present disclosure are within the scope of the present disclosure.

The term "stereoisomer" refers to compounds that have the same chemical constitution but differ in the spatial arrangements of atoms or groups. Stereoisomers include enantiomers, diastereoisomers, conformational isomers (rotamers), geometric isomers (cis/trans isomers), atropisomers, etc.

The term "geometric isomers", also known as "cis-trans isomers", refers to isomers caused by the inability of double bonds (including alkene double bonds, C=N double bonds, and N=N double bonds) or single bonds of ring carbon atoms to rotate freely.

The stereochemical definitions and rules as used herein generally follow S.P. Parker, Ed., McGraw-Hill Dictionary of Chemical Terms (1984), McGraw-Hill Book Company, New York; and Eliel, E. and Wilen, S., Stereochemistry of Organic Compounds, John Wiley & Sons, Inc., New York, 1994. Many organic compounds exist in optically active forms-that is, they have the ability to rotate the plane of plane-polarized light. When describing an optically active compound, the prefixes D and L or R and S are used to denote the absolute configuration of the molecule about one or more of its chiral centers. The prefixes D and L or (+) and (-) are the symbols used to designate the rotation of plane-polarized light caused by a compound, where (-) or L indicates that the compound is levorotatory. Compounds with the prefix (+) or D are dextrorotatory. A specific type of stereoisomer is an enantiomer, and a mixture of such isomers is referred to as an enantiomeric mixture. A 50:50 mixture of enantiomers is referred to as a racemic mixture or racemate, which may occur when there is no stereoselectivity or stereospecificity in a chemical reaction or process.

Any asymmetric atom (e.g., carbon) of the compound disclosed herein may exist in a racemic or enantiomerically enriched form, such as in an (R)-, (S)-, or (R,S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in terms of the (R)- or (S)-configuration.

Depending on the choice of starting materials and methods, the compound of the present disclosure may exist in the form of one of the possible isomers or a mixture thereof, such as a racemate and a diastereoisomeric mixture (depending on the number of asymmetric carbon atoms). Optically active (R)- or (S)-isomers can be prepared using chiral synthons or chiral reagents, or resolved using conventional techniques. If the compound contains a double bond, the substituents may be in an E or Z configuration; if the compound contains a disubstituted cycloalkyl group, the substituents of the cycloalkyl group may have a cis- or trans-configuration.

Any resulting mixture of stereoisomers may be separated into pure or substantially pure geometric isomers, enantiomers, or diastereoisomers depending on differences in the physicochemical properties of the components, for example, by chromatography and/or fractional crystallization.

The racemate of any resulting final product or intermediate can be resolved into optical enantiomers using known methods familiar to those skilled in the art, for example, by separation of the resulting diastereomeric salt. Racemic products can also be resolved by chiral chromatography, for example, by high-performance liquid chromatography (HPLC) using a chiral adsorbent. Particularly, enantiomers can be prepared by asymmetric synthesis. For reference, see, for example, Jacques, et al., Enantiomers, Racemates and Resolutions (Wiley Interscience, New York, 1981); Principles of Asymmetric Synthesis (2nd Ed., Robert E. Gawley, Jeffrey Aubé, Elsevier, Oxford, UK, 2012); Eliel, E.L., Stereochemistry of Carbon Compounds (McGraw-Hill, NY, 1962); Wilen, S.H., Tables of Resolving Agents and Optical Resolutions, p. 268 (E.L. Eliel, Ed., Univ. of Notre Dame Press, Notre Dame, IN, 1972); and Chiral Separation Techniques: A Practical Approach (Subramanian, G., Ed., Wiley-VCH Verlag GmbH & Co. KGaA, Weinheim, Germany, 2007).

The term "nitrogen oxide" refers to an N-oxide formed by oxidizing one or more nitrogen atoms when the compound contains several amine functional groups. Specific examples of N-oxides are N-oxides of tertiary amines or N-oxides of nitrogen atoms in nitrogen-containing heterocycles. The corresponding amines can be treated with an oxidant, such as hydrogen peroxide or peracid (e.g., peroxycarboxylic acid), to form N-oxides (see Advanced Organic Chemistry, Wiley Interscience, 4th edition, Jerry March, pages). In particular, N-oxides can be prepared by the method of L.W. Deady (Syn. Comm. 1977, 7, 509-514), wherein an amine compound is reacted with m-chloroperbenzoic acid (MCPBA) in an inert solvent such as dichloromethane.

The term "metabolite" refers to a product obtained *in vivo* through the metabolism of a specific compound or a salt thereof. Metabolites of a compound can be identified by techniques well known in the art, and their activities can be characterized by assays as described herein. Such products may be obtained by the oxidation, reduction, hydrolysis, amidation, deamidation, esterification, deesterification, enzymatic cleavage, and the like of the administered compound. Accordingly, the present disclosure includes metabolites of the compound, including those produced by sufficient exposure of the compound disclosed herein to a mammal over a period of time.

The term "pharmaceutically acceptable" means that the substance or composition must be chemically and/or toxicologically compatible with other ingredients contained in the formulation and/or the mammal to be treated therewith. Preferably, the term "pharmaceutically acceptable" as used herein refers to those approved by federal regulatory agencies or national governments, or listed in the *United States Pharmacopeia* or other generally recognized pharmacopeias for use in animals, particularly in humans.

The term "pharmaceutically acceptable salt" refers to both organic and inorganic salts of the compound of the present disclosure. Pharmaceutically acceptable salts are well known in the art to which they pertain, for example, those documented in the literature: S.M. Berge et al., J. Pharmaceutical Sciences, 66: 1-19, 1977. Pharmaceutically acceptable salts include salts formed by the compound with acids, including, but not limited to, inorganic acid salts (e.g., hydrochloride, hydrobromide, phosphate, sulfate, nitrate, and perchlorate) and organic acid salts (e.g., acetate, glycolate, oxalate, maleate, tartrate, citrate, succinate, fumarate, mandelate, and sulfosalicylate), or salts obtained by other methods documented in the literature, such as ion exchange. Additional pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecyl sulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, malonate, methanesulfonate, 2-naphthalenesulfonate, nicotinate, oleate, palmitate, pamoate, pectinate, persulfate, 3-phenylpropionate, picrate, pivalate, propionate, stearate, thiocyanate, p-toluenesulfonate, undecanoate, valerate, etc. Pharmaceutically acceptable salts also include salts formed by the compound with bases, including, but not limited to, inorganic base salts (e.g., alkali metal salts, alkaline earth metal salts, ammonium salts, and N⁺(C₁₋₄ alkyl)₄ salts). Alkali metal or alkaline earth metal salts include sodium, lithium, potassium, calcium, magnesium, etc. The present disclosure also contemplates quaternary ammonium salts formed by any compound having a N-containing group. Water-soluble or oil-soluble or dispersible products can be obtained by quaternization. Pharmaceutically acceptable salts further include appropriate and non-toxic ammonium, quaternary ammonium salts, and amine cations formed with counterions, such as halides, hydroxides, carboxylates, sulfates, phosphates, nitrates, C₁₋₈ sulfonates, and aromatic sulfonates. Organic base salts include, for example, salts of primary, secondary, and tertiary amines, and salts of substituted amines (including naturally occurring substituted amines, cyclic amines, and basic ion exchange resins). Certain organic amine salts include, for example, isopropylamine salts, benzathine salts, cholinate salts, diethanolamine salts, diethylamine salts, lysine salts, meglumine salts, piperazine salts, and tromethamine salts.

Pharmaceutically acceptable acid addition salts can be formed by reacting the compound of the present disclosure with inorganic or organic acids, and pharmaceutically acceptable base addition salts can be formed by reacting the compound of the present disclosure with inorganic or organic bases. The pharmaceutically acceptable salts of the present disclosure can be synthesized by conventional chemical methods from the parent compound, basic moiety, or acidic moiety. Generally, such salts can be prepared by reacting the free acid forms of these compounds with a stoichiometric amount of a suitable base (e.g., hydroxide, carbonate, bicarbonate, etc. of Na, Ca, Mg, or K), or by reacting the free base forms of these compounds with a stoichiometric amount of a suitable acid. Such reactions are typically carried out in water or an organic solvent or a mixture of the two. Generally, where appropriate, non-aqueous media such as diethyl ether, ethyl acetate, ethanol, isopropanol, or acetonitrile may be required. Additional lists of suitable salts may be found, for example, in Remington's Pharmaceutical Sciences, 20th Edition, Mack Publishing Company, Easton, Pa. (1985); and Handbook of Pharmaceutical Salts: Properties, Selection, and Use, Stahl and Wermuth (Wiley-VCH, Weinheim, Germany, 2002).

The term "solvate" refers to an association compound formed from one or more solvent molecules and the compound of the present disclosure. The solvent may be water, acetic acid, diethyl ether, isopropyl ether, petroleum ether, ethyl formate, ethyl acetate, isopropyl acetate, n-propyl acetate, isobutyl acetate, n-butyl acetate, methyl tert-butyl ether (MTBE), n-heptane, a mixed solvent of ethanol and water in a volume ratio of 10:90 to 90:10, acetone, methyl isobutyl ketone, acetonitrile, benzene, chloroform, carbon tetrachloride, dichloromethane, dimethyl sulfoxide, 1,4-dioxane, ethanol, ethyl acetate, ethylene glycol, n-butanol, tert-butanol, sec-butanol, N,N-dimethylacetamide, N,N-dimethylformamide, formamide, formic acid, n-hexane, cyclohexane, n-heptane, a mixed solvent of n-heptane and ethyl acetate in a volume ratio of 1:5 to 5:1, isopropanol, methanol, butanone, 1-methyl-2-pyrrolidone, mesitylene, nitromethane, polyethylene glycol, n-propanol, 2-propanone, 4-methyl-2-pentanone, pyridine, tetrahydrofuran, methyl ethyl ketone, toluene, xylene, cumene, or a mixture thereof, etc.

The term "hydrate" refers to an association compound formed from one or more water molecules and the compound of the present disclosure.

In addition, the compounds disclosed herein, including their salts, may also be obtained in the form of their hydrates or in the form of solvates containing solvents (e.g., ethanol, DMSO, etc.) for their crystallization. The compounds disclosed herein may inherently or by design form solvates with pharmaceutically acceptable solvents (including water); therefore, the present disclosure is intended to encompass both solvated and unsolvated forms.

The term "ester" is represented by the formula -OC(O)R or -C(O)OR, wherein R may be alkyl, cycloalkyl, alkenyl, cycloalkenyl, alkynyl, cycloalkynyl, aryl, or heteroaryl as described in the present disclosure.

The term "isotope-labeled compound" refers to a compound of the present disclosure that is labeled with an isotope. It is identical to those compounds described in the present disclosure except for the fact that one or more atoms are replaced by an atom having an atomic mass or mass number different from the atomic mass or mass number naturally predominant. Exemplary isotopes that may also be incorporated into the compound of the present disclosure include isotopes of hydrogen, carbon, nitrogen, oxygen, phosphorus, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F, and ³⁷Cl.

Compounds of the present disclosure that contain the aforementioned isotopic labels and/or other isotopic labels of other atoms, as well as pharmaceutically acceptable salts of the compounds, are all encompassed within the scope of the present disclosure. Isotope-labeled compounds of the present disclosure, such as those into which radioisotopes (e.g., ³H and ¹⁴C) are incorporated, may be used in drug and/or substrate tissue distribution assays. Tritium (i.e., ³H) and carbon-14 (i.e., ¹⁴C) isotopes are particularly preferred due to their ease of preparation and detection. Additionally, substitution with isotopes with larger mass numbers, such as deuterium (i.e., ²H), may provide some therapeutic advantages due to greater metabolic stability, for example, an increased *in vivo* half-life or reduced dosage requirements. Thus, in some cases, it may be preferred.

Furthermore, substitution with heavier isotopes, particularly deuterium (i.e., ²H or D), may provide certain therapeutic advantages resulting from greater metabolic stability. For example, these advantages may include increased *in vivo* half-life or reduced dosage requirements or improved therapeutic index. It should be understood that deuterium in the present disclosure is considered as a substituent of the compounds of formulas I to VI. The concentration of such heavier isotopes, particularly deuterium, may be defined using an isotopic enrichment factor. The term "isotopic enrichment factor" as used herein refers to the ratio of the isotopic abundance of a specified isotope to its natural abundance. Where a substituent in the compound of the present disclosure is designated as deuterium, the compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). The pharmaceutically acceptable solvates of the present disclosure include those in which the crystallization solvent may be isotopically substituted, e.g., D₂O, acetone-d₆, or DMSO-d₆.

The term "prodrug" as used herein represents a compound that is converted *in vivo* to a compound represented by formula I. Such conversion is influenced by hydrolysis of the prodrug in blood or by enzymatic conversion of the prodrug into the parent structure in blood or tissues. The prodrug compounds of the present disclosure may be esters, and in the prior art, esters that can serve as prodrugs include phenyl esters, aliphatic (C1-24) esters, acyloxymethyl esters, carbonates, carbamates, and amino acid esters. For example, a compound of the present disclosure containing hydroxyl may be acylated to obtain a prodrug form of the compound. Other prodrug forms include phosphate esters, such as those phosphate ester compounds obtained through phosphorylation of the hydroxyl on the parent structure. For a complete discussion on prodrugs, reference can be made to the following literature: Higuchi et al., Pro-drugs as Novel Delivery Systems, Vol. 14, A.C.S. Symposium Series; Roche et al., Bioreversible Carriers in Drug Design, American Pharmaceutical Association and Pergamon Press, 1987; Rautio et al., Prodrugs: Design and Clinical Applications, Nature Reviews Drug Discovery, 2008, 7, 255-270; and Hecker et al., Prodrugs of Phosphates and Phosphonates, J. Med. Chem., 2008, 51, 2328-2345.

Unless otherwise explicitly indicated, the descriptions "each... be independently" and "... be each independently" and "... be independently" in the present disclosure are used interchangeably and should be understood in a broad sense, and the descriptions may mean that specific options expressed by the same symbol in different groups do not affect each other, or that specific options expressed by the same symbol in the same group do not affect each other.

The term "optional" or "optionally" means that the subsequently described event or circumstance may, but does not necessarily, occur, and the description includes instances where the event or circumstance occurs and instances where it does not. For example, "optionally substituted with..." means that the substitution may or may not be present.

The term "each independently", when used in combination with "optionally", such as in "each independently optionally substituted with...", means that the specific options do not affect each other in the case of either substitution or non-substitution with "...".

The term "unsaturated" means that the moiety contains one or more degrees of unsaturation.

Throughout various sections of this specification, substituents of the compound of the present disclosure are disclosed according to group types or ranges. It is specifically noted that the present disclosure includes each and every individual sub-combination of the members of these group types and ranges. For example, the term "C₁₋₆ alkyl" specifically refers to independently disclosed methyl, ethyl, C₃ alkyl, C₄ alkyl, C₅ alkyl, and C₆ alkyl.

Throughout various sections of the present disclosure, linking substituents are described. When the structure clearly requires a linking group, the Markush variables listed for the group should be understood as linking groups. For example, if the structure requires a linking group and "alkyl" or "aryl" are listed for the Markush group definition of the variable, it should be understood that the "alkyl" or "aryl" respectively represents a linked alkylene group or arylene group.

The term "heteroatom" refers to O, S, N, P, and Si, including any oxidation states of S, N, and P; the forms of primary, secondary, and tertiary amines, and quaternary ammonium salts; or the form where hydrogen on a nitrogen atom in a heterocycle is substituted, for example, N (such as N in 3,4-dihydro-2H-pyrrolyl), NH (such as NH in pyrrolidinyl), or NRT (such as NRT in N-substituted pyrrolidinyl, where RT is a substituent on N). In the compounds involved in the present disclosure, when containing multiple heteroatoms, the resulting compounds conform to the covalent rules and composition rules of organic compounds; that is, the compounds containing multiple heteroatoms should exclude compounds that do not conform to the covalent rules and composition rules of organic compounds.

The term "heterocyclyl" refers to a saturated (i.e., "heterocycloalkyl") or partially unsaturated monovalent monocyclic or bicyclic group having 2, 3, 4, 5, 6, 7, 8, or 9 carbon atoms and one or more (e.g., one, two, three, or four) heteroatom-containing groups selected from C(=O), O, S, S(=O), S(=O)₂, and NR' in the ring, where R' represents a hydrogen atom or C1-6 alkyl or C1-6 haloalkyl. The heterocyclyl may be attached to the rest of the molecule through any one of the carbon atoms or the nitrogen atom (if present). In particular, 3- to 10-membered heterocyclyl is a group having 3-10 (e.g., 3-7, 4-6, or 5-6) ring atoms in the ring, wherein the ring atoms include carbon atoms and heteroatoms; examples thereof include, but are not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl, or trithianyl.

The term "heterocyclyl" encompasses fused ring structures, wherein the point of attachment to other groups may be on any ring within the fused ring structure. Thus, the heterocyclyl of the present disclosure also includes, but is not limited to, heterocyclyl-fused heterocyclyl, heterocyclyl-fused cycloalkyl, monoheterocyclyl-fused monoheterocyclyl, and monoheterocyclyl-fused monocycloalkyl, such as 3- to 7-membered (mono)heterocyclyl-fused 3- to 7-membered (mono)heterocyclyl, 3- to 7-membered (mono)heterocyclyl-fused (mono)cycloalkyl, and 3- to 7-membered (mono)heterocyclyl-fused C4-6 (mono)cycloalkyl, examples of which include, but are not limited to, pyrrolidinyl-fused cyclopropyl, cyclopentyl-fused aziridinyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

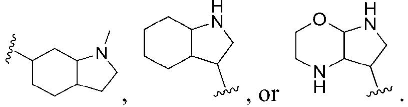

The term "heterocyclyl" encompasses bridged heterocyclyl and spiroheterocyclyl.

The term "bridged heterocycle" refers to a cyclic structure containing one or more (e.g., 1, 2, 3, or 4) heteroatoms (e.g., oxygen atoms, nitrogen atoms, and/or sulfur atoms) formed by two saturated rings sharing two ring atoms that are not directly linked, including, but not limited to, 7- to 10-membered bridged heterocycle, 8- to 10-membered bridged heterocycle, 7- to 10-membered nitrogen-containing bridged heterocycle, 7- to 10-membered oxygen-containing bridged heterocycle, 7- to 10-membered sulfur-containing bridged heterocycle, and the like, such as 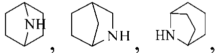 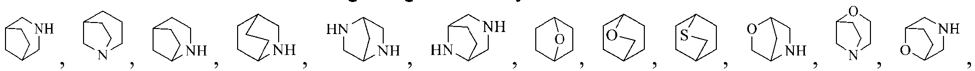 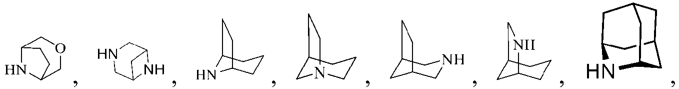 etc. The "nitrogen-containing bridged heterocycle", "oxygen-containing bridged heterocycle", or "sulfur-containing bridged heterocycle" optionally further contains one or more additional heteroatoms selected from oxygen, nitrogen, and sulfur.

The term "monospiroheterocyclyl" refers to a cyclic structure containing one or more (e.g., 1, 2, 3, or 4) heteroatoms (e.g., oxygen atoms, nitrogen atoms, or sulfur atoms) formed by two or more saturated or partially unsaturated rings sharing one ring atom. "Monospiroheterocycloalkyl" is monospiroheterocyclyl in which each ring forming the spirocycle is a saturated ring. Monospiroheterocycloalkyl includes, but is not limited to, 5- to 11-membered monospiroheterocycloalkyl, 6- to 10-membered monospiroheterocycloalkyl, 7- to 10-membered monospiroheterocycloalkyl, 6- to 10-membered nitrogen-containing spiroheterocycloalkyl, 6- to 10-membered oxygen-containing spiroheterocycloalkyl, 6- to 10-membered sulfur-containing spiroheterocycloalkyl, and the like. Monospiroheterocycloalkyl may include, e.g., 3-membered/5-membered ring systems, 4-membered/4-membered ring systems, 4-membered/5-membered ring systems, 4-membered/6-membered ring systems, 5-membered/5-membered ring systems, 5-membered/6-membered ring systems, and 6-membered/6-membered ring systems, wherein the numbering of each ring includes the spiro atom. Examples include, but are not limited to, 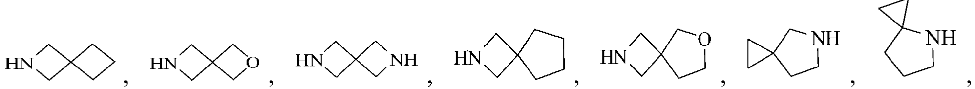 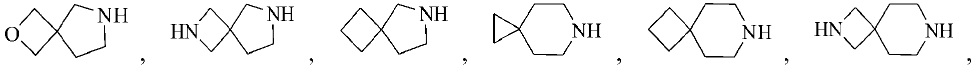 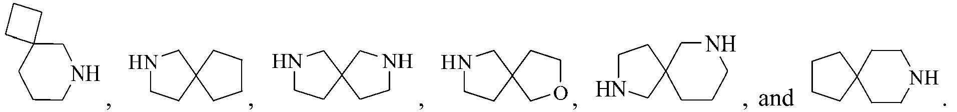 The "nitrogen-containing monospiroheterocycloalkyl", "oxygen-containing monospiroheterocycloalkyl", or "sulfur-containing monospiroheterocycloalkyl" optionally further contains one or more additional heteroatoms selected from oxygen, nitrogen, and sulfur. The term "6- to 10-membered nitrogen-containing monospiroheterocycloalkyl" refers to spiroheterocyclyl containing a total of 6-10 ring atoms, wherein at least one ring atom is a nitrogen atom.

The term "monocyclic heterocycle" refers to monocyclic heterocyclyl containing carbon atoms and a heteroatom, which may be fully saturated or contain one or more degrees of unsaturation, but cannot contain any aromatic ring.

The term "cycloalkyl" refers to a monovalent or polyvalent monocyclic (e.g., cyclopropyl, cyclobutyl, cyclopentyl, cyclohexyl, cycloheptyl, cyclooctyl, or cyclononyl), bicyclic (including spiro, fused, or bridged systems (e.g., bicyclo[1.1.1]pentyl, bicyclo[2.2.1]heptyl, bicyclo[3.2.1]octyl or bicyclo[5.2.0]nonyl, decahydronaphthalenyl, etc.)), or tricyclic system containing carbon atoms, which may be fully saturated or contain one or more degrees of unsaturation, but cannot contain any aromatic ring. In one embodiment, the cycloalkyl contains 3-6 carbon atoms, such as C₃₋₆ saturated or partially unsaturated cycloalkyl. Examples of cycloalkyl groups include, but are not limited to, cyclopropyl, cyclobutyl, cyclopentyl, cyclohexyl, cyclopentenyl, cyclohexenyl, etc. In one embodiment, the saturated or partially unsaturated cycloalkyl is selected from: saturated monocyclic cycloalkyl, saturated bicyclic cycloalkyl, saturated tricyclic cycloalkyl, partially unsaturated monocyclic cycloalkyl, partially unsaturated bicyclic cycloalkyl, and partially unsaturated tricyclic cycloalkyl. C₄₋₇ cycloalkyl refers to cycloalkyl having 4-7 ring atoms. C₃₋₆ cycloalkyl refers to cycloalkyl having 3-6 ring atoms.

The term "monocyclic carbocycle" refers to a monocyclic ring containing carbon atoms, which may be fully saturated or contain one or more degrees of unsaturation, but cannot contain any aromatic ring. Examples include, but are not limited to, cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, cycloheptyl, cyclooctyl, cyclononyl, cyclodecyl, cycloundecyl, cyclododecyl, etc.

The term "heteroaryl" or "heteroaromatic ring" refers to a monocyclic, bicyclic, or tricyclic aromatic system containing a heteroatom. The term "heteroaryl" is used interchangeably with the term "heteroaromatic ring" or "heteroaromatic compound". The heteroatom has the definition as described in the present disclosure. In some embodiments, the heteroaryl is heteroaryl consisting of 5-10 atoms containing 1, 2, 3, or 4 heteroatoms independently selected from O, S, and N, i.e., 5- to 10-membered heteroaryl; the heteroaryl is heteroaryl consisting of 5-8 atoms containing 1, 2, 3, or 4 heteroatoms independently selected from O, S, and N, i.e., 5- to 8-membered heteroaryl; in some embodiments, the heteroaryl is heteroaryl consisting of 5-7 atoms containing 1, 2, 3, or 4 heteroatoms independently selected from O, S, and N, i.e., 5- to 7-membered heteroaryl; in some embodiments, the heteroaryl is heteroaryl consisting of 5-6 atoms containing 1, 2, 3, or 4 heteroatoms independently selected from O, S, and N, i.e., 5- to 6-membered heteroaryl; in some embodiments, the heteroaryl is heteroaryl consisting of 5 atoms containing 1, 2, 3, or 4 heteroatoms independently selected from O, S, and N, i.e., 5-membered heteroaryl; in some embodiments, the heteroaryl is heteroaryl consisting of 6 atoms containing 1, 2, 3, or 4 heteroatoms independently selected from O, S, and N, i.e., 6-membered heteroaryl. Examples of heteroaryl groups include, but are not limited to, furanyl (e.g., 2-furanyl or 3-furanyl), imidazolyl (e.g., N-imidazolyl, 2-imidazolyl, 4-imidazolyl, or 5-imidazolyl), isoxazolyl (e.g., 3-isoxazolyl, 4-isoxazolyl, or 5-isoxazolyl), oxazolyl (e.g., 2-oxazolyl, 4-oxazolyl, or 5-oxazolyl), pyrrolyl (e.g., N-pyrrolyl, 2-pyrrolyl, or 3-pyrrolyl), pyridinyl (e.g., 2-pyridinyl, 3-pyridinyl, or 4-pyridinyl), pyrimidinyl (e.g., 2-pyrimidinyl, 4-pyrimidinyl, or 5-pyrimidinyl), pyridazinyl (e.g., 3-pyridazinyl), thiazolyl (e.g., 2-thiazolyl, 4-thiazolyl, or 5-thiazolyl), tetrazolyl (e.g., 5H-tetrazolyl or 2H-tetrazolyl), triazolyl (e.g., 2-triazolyl, 5-triazolyl, 4H-1,2,4-triazolyl, 1H-1,2,4-triazolyl, or 1,2,3-triazolyl), thienyl (e.g., 2-thienyl or 3-thienyl), pyrazolyl (e.g., 2-pyrazolyl or 3-pyrazolyl), isothiazolyl, oxadiazolyl (e.g., 1,2,3-oxadiazolyl, 1,2,5-oxadiazolyl, 1,2,4-oxadiazolyl, or 1,3,4-oxadiazolyl), thiadiazolyl (e.g., 1,2,3-thiadiazolyl, 1,3,4-thiadiazolyl, or 1,2,5-thiadiazolyl), pyrazinyl, and 1,3,5-triazinyl; examples thereof also include the following bicyclic or tricyclic groups, but are in no way limited to the following groups: benzimidazolyl, benzofuranyl, benzothienyl, indolyl (e.g., 2-indolyl), purinyl, quinolinyl (e.g., 2-quinolinyl, 3-quinolinyl, or 4-quinolinyl), isoquinolinyl (e.g., 1-isoquinolinyl, 3-isoquinolinyl, or 4-isoquinolinyl), imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, indolinyl, 1,2,3,4-tetrahydroisoquinolinyl, etc.

The term "aryl" or "aromatic ring" refers to a monocyclic, bicyclic, or tricyclic aromatic carbocyclic system. The term "aryl" is used interchangeably with the term "aromatic ring". 6- to 10-membered aryl represents an aryl group containing 6-10 ring atoms; examples thereof include, but are not limited to, phenyl, naphthyl, etc.

The term "hydrogen" refers to ¹H; "deuterium" refers to ²H.

The terms "halogen" and "halo" refer to fluorine (F), chlorine (Cl), bromine (Br), or iodine (I).

The term "amino" refers to -NH₂.

The term "hydroxyl" refers to -OH.

The term "sulfhydryl" refers to -SH.

The term "cyano" refers to -CN.

The term "nitro" refers to -NO₂.

The term "carboxyl" refers to HO(C=O)-.

The terms "oxo" and "O=" are used interchangeably, that is, when the substituent is O=, the O is connected to the substituted group through a double bond.

The terms "thio" and "S=" are used interchangeably, that is, when the substituent is S=, the S is connected to the substituted group through a double bond.

The term "alkyl" or "alkyl group" refers to a saturated, linear or branched hydrocarbon group containing carbon atoms. In one embodiment, the alkyl group contains 1-6 carbon atoms, i.e., C₁₋₆ alkyl; in another embodiment, the alkyl group contains 1-4 carbon atoms, i.e., C₁₋₄ alkyl; in yet another embodiment, the alkyl group contains 1-3 carbon atoms, i.e., C₁₋₃ alkyl. Examples of alkyl include, but are not limited to, methyl, ethyl, n-propyl, isopropyl, n-butyl, tert-butyl, isobutyl, secbutyl, n-pentyl, n-hexyl, and the like.

When two groups are jointly used, it means that one of the groups is attached to the rest of the molecule through the other group. For example, arylalkyl means that the aryl is attached to the molecule through the alkyl; C₆₋₁₀ aryl C₁₋₆ alkyl means that the aryl has 6-10 carbon atoms and the alkyl has 1-6 carbon atoms.

The term "alkenyl" refers to a linear or branched monovalent hydrocarbon group containing carbon atoms, wherein there is at least one site of unsaturation, i.e., one carbon-carbon sp² double bond, which includes "cis" and "trans" configurations, or "E" and "Z" configurations. In one embodiment, the alkenyl group contains 2-6 carbon atoms, i.e., C₂-C₆ alkenyl; in another embodiment, the alkenyl group contains 2-4 carbon atoms, i.e., C₂-C₄ alkenyl. Examples of alkenyl groups include, but are not limited to, vinyl (-CH=CH₂), allyl (-CH₂CH=CH₂), etc.

The term "alkynyl" refers to a linear or branched monovalent hydrocarbon group containing carbon atoms, wherein there is at least one site of unsaturation, i.e., one carbon-carbon sp triple bond. In one embodiment, the alkynyl group contains 2-6 carbon atoms, i.e., C₂-C₆ alkynyl; in another embodiment, the alkynyl group contains 2-4 carbon atoms, i.e., C₂-C₄ alkynyl. Examples of alkynyl groups include, but are not limited to, ethynyl (-C=CH), propargyl (-CH₂C≡CH), 1-propynyl (-C=C-CH₃), etc.

The term "heteroalkyl" refers to a group formed by incorporating one or more heteroatoms into alkyl. The alkyl and heteroatom have the definitions as described in the present disclosure. The heteroalkyl may be attached to the rest of the molecule through a carbon atom or heteroatom.

The term "alkoxy" refers to an alkyl group attached to the rest of the molecule through an oxygen atom, wherein the alkyl group has the meaning as described in the present disclosure. In one embodiment, the alkoxy group contains 1-6 carbon atoms, i.e., C₁₋₆ alkoxy; in another embodiment, the alkoxy group contains 1-4 carbon atoms, i.e., C₁₋₄ alkoxy; in yet another embodiment, the alkoxy group contains 1-3 carbon atoms, i.e., C₁₋₃ alkoxy. Examples of alkoxy groups include, but are not limited to, methoxy (MeO, -OCH₃), ethoxy (EtO, -OCH₂CH₃), 1-propoxy (n-PrO, n-propoxy, - OCH₂CH₂CH₃), 2-propoxy (i-PrO, i-propoxy, -OCH(CH₃)₂), 1-butoxy (n-BuO, n-butoxy, - OCH₂CH₂CH₂CH₃), 2-methyl-1-propoxy (i-BuO, i-butoxy, -OCH₂CH(CH₃)₂), 2-butoxy (s-BuO, s-butoxy, -OCH(CH₃)CH₂CH₃), 2-methyl-2-propoxy (t-BuO, t-butoxy, -OC(CH₃)₃), 1-pentyloxy (n-pentyloxy, -OCH₂CH₂CH₂CH₂CH₃), 2-pentyloxy (-OCH(CH₃)CH₂CH₂CH₃), 3-pentyloxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-1-butoxy (-OCH₂CH₂CH(CH₃)₂), 2-methyl-1-butoxy (-OCH₂CH(CH₃)CH₂CH₃), etc.

The term "alkylamino" refers to an alkyl group attached to the rest of the molecule through a nitrogen atom, wherein the alkyl group has the meaning as described in the present disclosure. In one embodiment, the alkylamino group contains 1-6 carbon atoms, i.e., C₁₋₆ alkylamino, including (C₁₋₆ alkyl)NH- and (C₁₋₆ alkyl)₂N-; in another embodiment, the alkylamino group contains 1-4 carbon atoms, i.e., C₁₋₄ alkylamino; in yet another embodiment, the alkylamino group contains 1-3 carbon atoms, i.e., C₁₋₃ alkylamino. Examples include, but are not limited to, methylamino (N-methylamino), ethylamino (N-ethylamino), N,N-dimethylamino, N,N-diethylamino, etc.

When a group (e.g., aryl, heteroaryl, cycloalkyl, or heterocyclyl) is jointly used with oxy or amino, it means that the group (e.g., aryl, heteroaryl, cycloalkyl, or heterocyclyl) is attached to the rest of the molecule through an oxygen atom or a nitrogen atom; for example, aryloxy means that the aryl is attached to the rest of the molecule through an oxygen atom.

The term "two substituents are connected to form a ring" means that the positions of the two substituents are connected through one bond or one atom or a long chain of multiple atoms, forming a ring together with the atoms between the positions of the two substituents on the parent molecule.

The term "two R^{a} are connected to form a 3- to 7-membered monocyclic carbocycle or a 3- to 7-membered monocyclic heterocycle" includes: two R^{a}, together with the atoms to which they are attached, forming a 3- to 7-membered monocyclic carbocycle or 3- to 7-membered monocyclic heterocycle fused with ring W (e.g., 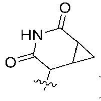 ); two R^{a}, together with the atoms to which they are attached, forming a 3- to 7-membered monocyclic carbocycle or 3- to 7-membered monocyclic heterocycle that constitutes a spiro ring system with ring W (e.g., 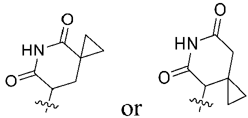 ); or two R^{a}, together with the atoms to which they are attached, forming a 3- to 7-membered monocyclic carbocycle or 3- to 7-membered monocyclic heterocycle that constitutes a bridged ring system with ring W (e.g., 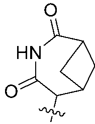 ).

The term "R^{b} and R^{a} are connected to form a 3- to 7-membered monocyclic carbocycle or a 3-to 7-membered monocyclic heterocycle" means that R^{b} connected to R^{a}, together with the atoms on ring W, form a 3- to 7-membered monocyclic carbocycle or 3- to 7-membered monocyclic heterocycle that constitutes a bridged ring system with ring W (e.g., 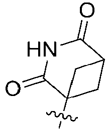 ).

The term "comprise" is synonymous with "include", "contain", or "be characterized by", is inclusive or open-ended, and does not exclude additional, unmentioned elements or ingredients from the drug (or, in the case of a method, steps). The phrase "consist of..." excludes any elements, steps, or ingredients not specified in the drug (or, in the case of a method, steps). The phrase "consist essentially of..." refers to the specified materials and those materials that do not substantially affect the basic and novel characteristics of the drug (or, in the case of a method, steps).

As described herein, a ring system formed by substituent R being connected to the center of a ring through a bond (as shown below) represents that substitution with substituent R occurs at any substitutable or any reasonable position on ring A. For example, formula f denotes any potentially substituted position on ring A, as represented by formulas f1-f5:

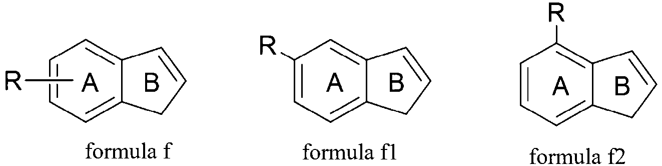

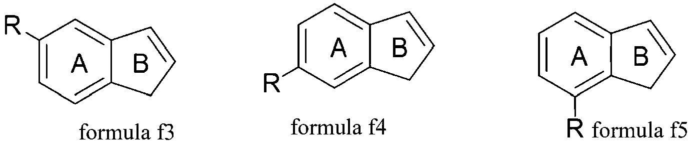

As described herein, a ring system formed by a substituent being connected to the center of a ring through a bond, such as (R^{x})ₙ, represents that substitution with n substituents R^{x} may occur at any substitutable position on the ring where they are located. For example, formula a represents that the benzene ring may be substituted with n R^{x}

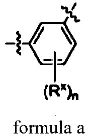

As described herein, *(R,S) means that the marked chiral center is in either the R or S configuration, but does not specify whether it is the R configuration or the S configuration.

The term "substituted" means that one or more hydrogen atoms on a specific group are replaced by a specific substituent. The specific substituents are those described correspondingly in the preceding text or those appearing in the various examples. Unless otherwise specified, a substituted group may have, at any substitutable position on the group, a substituent selected from a specific group, and the substituents at various positions may be identical or different, i.e., each substitution is independent of the others. It should be understood by those skilled in the art that combinations of substituents contemplated by the present disclosure are those that are stable or chemically achievable.

In addition, for the purpose of page simplification, when it is mentioned that a certain group may be substituted, it means that among the multiple options of that group, those substitutable groups may be substituted. For example, when R' is hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, or C₆₋₁₀ aryl C₁₋₆ alkyl, "R' is optionally substituted with 1, 2, 3, 4, or 5 substituents selected from hydrogen, deuterium, oxo (O=), thio (S=), halogen, cyano, and hydroxyl" means that the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, or C₆₋₁₀ aryl C_{1- 6} alkyl in R' is optionally substituted with 1, 2, 3, 4, or 5 substituents selected from hydrogen, deuterium, oxo (O=), thio (S=), halogen, cyano, and hydroxyl.

Unless otherwise specified, substituents or combinations of groups involving Markush structures herein are those that are stable or chemically achievable.

### Description of Compound of the Present Disclosure

The present disclosure provides a compound, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof, which plays a positive role in the treatment of CRBN-mediated diseases or disorders.

Specifically, the present disclosure provides a compound having a structure represented by formula I, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 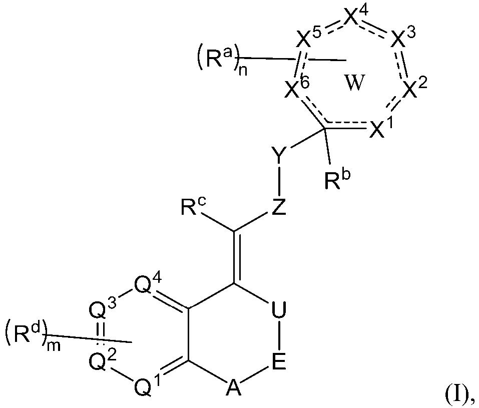 wherein
X¹, X², X³, X⁴, X⁵, and X⁶ are each independently a bond, carbon, nitrogen, oxygen, or sulfur; ring W is a 5-, 6-, or 7-membered ring;
the - - - - - - bonds connected to X¹, X², X³, X⁴, X⁵, and X⁶ each independently represent a single bond or a double bond; two - - - - - - bonds connected to the same atom are not simultaneously double bonds; when X¹, X², X³, X⁴, X⁵, or X⁶ is oxygen or sulfur, the - - - - - - bonds connected thereto are single bonds;
each R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3-to 6-membered heterocyclylamino; optionally, two R^{a} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, and the 3- to 7-membered monocyclic carbocycle or 3- to 7-membered monocyclic heterocycle formed by the connection of two R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
n is 0, 1, 2, 3, 4, 5, 6, 7, 8, 9, 10, 11, or 12;
R^{b} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, or 3- to 6-membered heterocyclyl; optionally, R^{b} and R^{a} are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, and the 3- to 7-membered monocyclic carbocycle or 3-to 7-membered monocyclic heterocycle formed by the connection of R^{b} and R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Y is a bond, NH, CH₂, CH₂CH₂, NHCH₂, or CH₂NH; optionally, Y is substituted with 1, 2, 3, or 4 R^{Y}; each R^{Y} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, and 5- to 10-membered heteroaryl are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Z is NH, O, S, or CH₂; optionally, Z is substituted with 1 or 2 R^{Z}; each R^{Z} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, R^{Z} and R^{a}, or R^{Z} and R^{b}, are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, 5- to 10-membered heteroaryl, and the 3- to 7-membered monocyclic carbocycle or 3- to 7-membered monocyclic heterocycle formed by the connection of R^{Z} and R^{b} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R^{c} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
U is CH₂, NH, O, or S;
E and A are each independently a bond, CH₂, NH, O, or S, wherein E and A are not simultaneously a bond, NH, or O;
optionally, U, E, and A are each independently substituted with 1 or 2 R^{e}; each R^{e} is independently hydrogen, deuterium, halogen, cyano, hydroxyl, amino, oxo (O=), thio (S=), C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, two R^{e} attached to the same carbon atom, together with the atom to which they are attached, form a 3- to 7-membered monocyclic carbocycle or a 3- to 7-membered monocyclic heterocycle; the C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
Q¹, Q², Q³, and Q⁴ are each independently CH or N;
each R^{d} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-; optionally, two adjacent R^{d} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, C₆₋₁₀ aryl S(=O)₂NH-, and the 3- to 7-membered monocyclic carbocycle or 3- to 7-membered monocyclic heterocycle formed by the connection of two adjacent R^{d} are each independently optionally substituted with 1, 2, 3, 4, or 5 R^{d1}, each R^{d1} being independently selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, 3- to 6-membered heterocyclyl, and C₁₋₆ alkyl OC(=O)-; optionally, two R^{d1} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle formed by the connection of two R^{d1} is optionally substituted with 1, 2, 3, 4, or 5 substituents selected from hydrogen, deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
m is 0, 1, 2, 3, or 4.

In some embodiments, 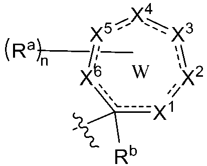 is 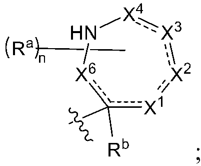 X¹, X², X³, X⁴, X⁶, R^{b}, W, R^{a}, and n have the definitions as described in the present disclosure.

In some embodiments, 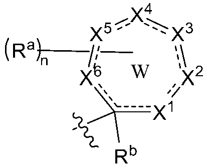 is selected from: 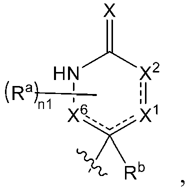 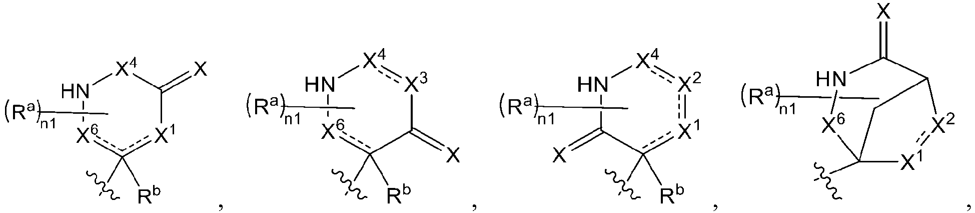 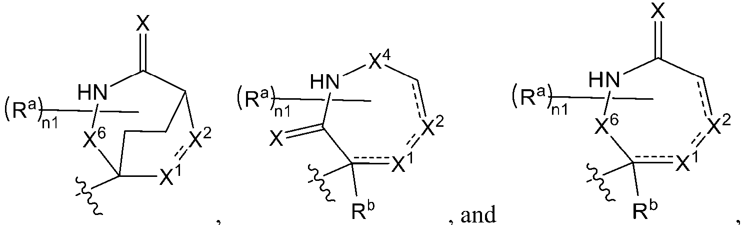 wherein X is O or S; n1 is 0, 1, 2, 3, or 4; X¹, X², X³, X⁴, X⁶, R^{b}, and R^{a} have the definitions as described in the present disclosure.

In some embodiments, 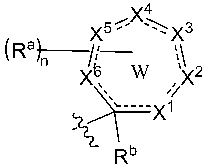 is selected from: 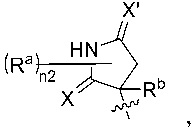 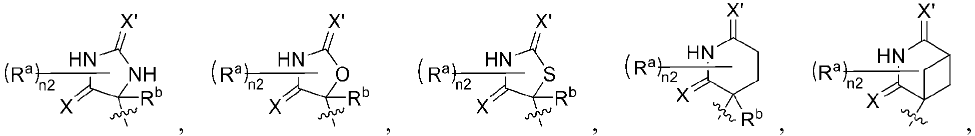 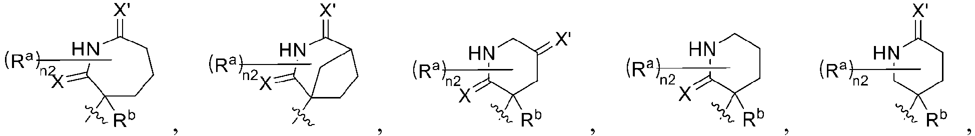 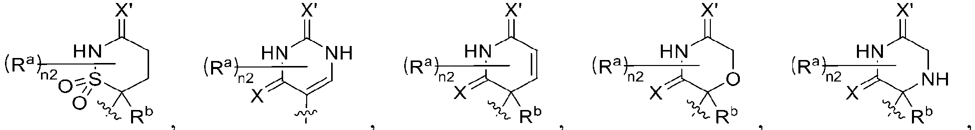 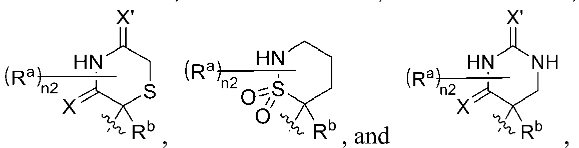 wherein X and X' are each independently O or S; n2 is 0, 1, 2, 3, or 4; R^{b} and R^{a} have the definitions as described in the present disclosure.

Preferably, 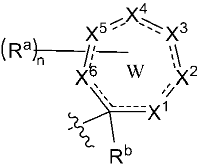 is selected from: 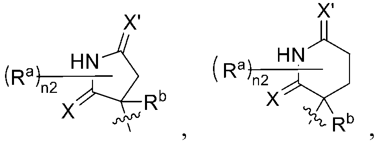 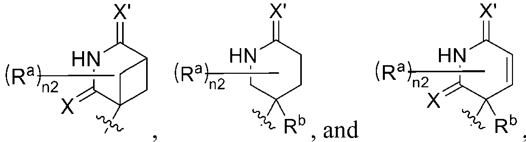 wherein X and X' are each independently O or S; n2 is 0, 1, 2, 3, or 4, R^{b} and R^{a} have the definitions as described in the present disclosure. Preferably, n2 is 0, 1, 2, or 3.

In some embodiments, 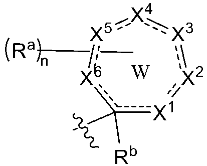 is selected from 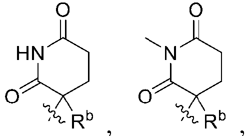 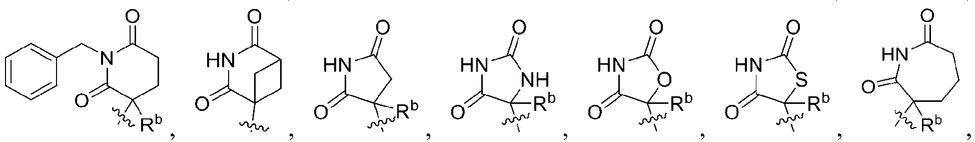 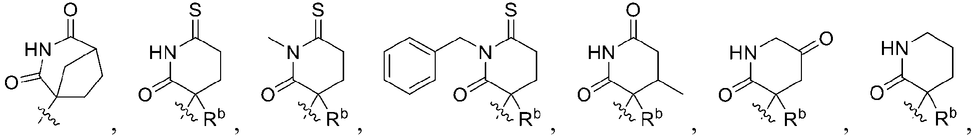 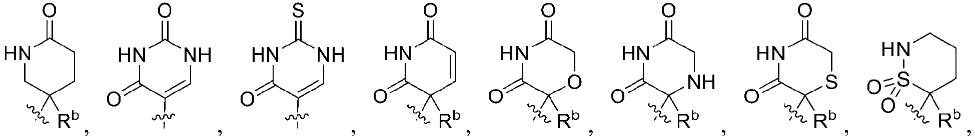 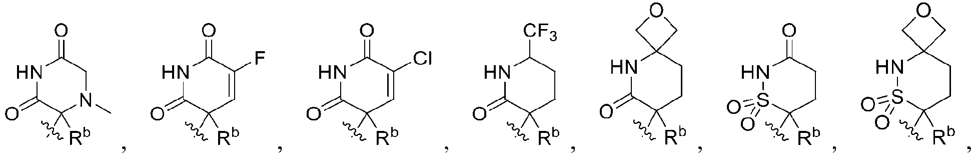 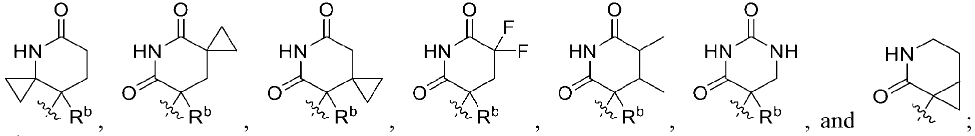 R^{b} has the definition as described in the present disclosure.

Preferably, 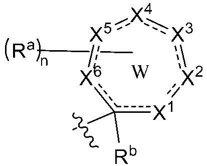 is selected from 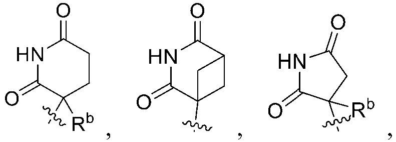 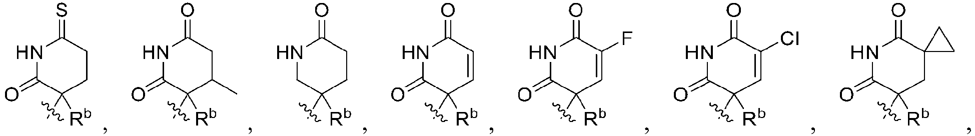 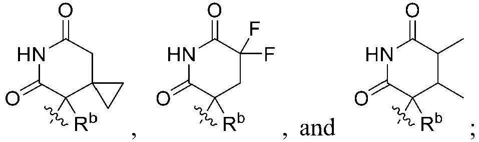 R^{b} has the definition as described in the present disclosure.

In some embodiments, 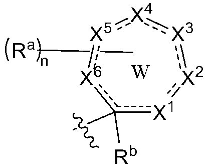 is selected from 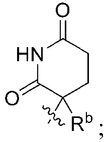 R^{b} has the definition as described in the present disclosure.

In some embodiments, 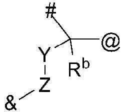 is selected from 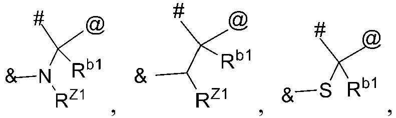 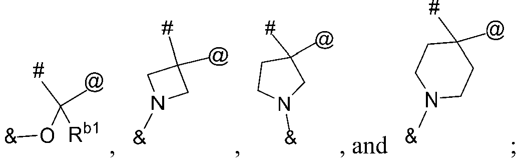 # is connected to X⁶, @ is connected to X¹, and & is connected to the double bond;
each R^{b1} is independently hydrogen, deuterium, halogen, cyano, hydroxyl, amino, or C₁₋₆ alkyl, wherein the C₁₋₆ alkyl is optionally substituted with 1, 2, 3, 4, or 5 substituents selected from deuterium, oxo (O=), halogen, cyano, hydroxyl, amino, C₁₋₄ alkyl, C₁₋₄ alkoxy, C₁₋₄ alkylamino, and C₃₋₆ cycloalkyl;
each R^{Z1} is independently hydrogen, deuterium, halogen, cyano, hydroxyl, amino, or C₁₋₆ alkyl, wherein the C₁₋₆ alkyl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, and C₁₋₄ alkylamino.

In some embodiments, preferably, each R^{b1} is independently hydrogen, deuterium, F, Cl, Br, cyano, hydroxyl, or C₁₋₄ alkyl, wherein the C₁₋₄ alkyl is optionally substituted with 1, 2, 3, 4, or 5 substituents selected from deuterium, F, Cl, Br, cyano, hydroxyl, amino, C₁₋₄ alkyl, C₁₋₄ alkoxy, and C₁₋₄ alkylamino.

In some embodiments, each R^{b1} is independently hydrogen, deuterium, F, Cl, Br, cyano, hydroxyl, methyl, ethyl, propyl, or isopropyl, wherein the methyl, ethyl, propyl, and isopropyl are each independently optionally substituted with 1, 2, or 3 substituents selected from deuterium, F, Cl, Br, cyano, hydroxyl, amino, methyl, ethyl, propyl, isopropyl, methoxy, ethoxy, isopropoxy, methylamino, and dimethylamino.

In some embodiments, each R^{b1} is independently hydrogen, deuterium, F, Cl, Br, cyano, or methyl.

In some embodiments, each R^{b1} is hydrogen.

In some embodiments, each R^{Z1} is independently hydrogen, deuterium, or C₁₋₄ alkyl, wherein the C₁₋₄ alkyl is optionally substituted with 1, 2, or 3 substituents selected from deuterium, F, Cl, Br, cyano, hydroxyl, amino, C₁₋₄ alkyl, C₁₋₄ alkoxy, and C₁₋₄ alkylamino.

In some embodiments, each R^{Z1} is independently hydrogen, deuterium, methyl, ethyl, propyl, or isopropyl.

In some embodiments, 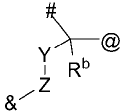 is selected from 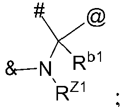 R^{Z1} and R^{b1} have the definitions as described in the present disclosure.

In some embodiments, R^{c} is hydrogen, deuterium, halogen, cyano, hydroxyl, amino, C₁₋₆ alkyl, C₁₋₆ alkoxy, C₁₋₆ alkylamino, C₃₋₆ cycloalkyl, 3- to 6-membered heterocyclyl, C₆₋₁₀ aryl, or 5- to 10-membered heteroaryl, wherein the C₁₋₆ alkyl, C₁₋₆ alkoxy, C₁₋₆ alkylamino, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, halogen, cyano, hydroxyl, amino, C₁₋₄ alkyl, C₁₋₄ alkoxy, and C₁₋₄ alkylamino.

In some embodiments, R^{c} is halogen, cyano, C₁₋₆ alkyl, C₆₋₁₀ aryl, or 5- to 10-membered heteroaryl, wherein the C₁₋₆ alkyl, C₆₋₁₀ aryl, or 5- to 10-membered heteroaryl is optionally substituted with 1, 2, 3, 4, or 5 substituents selected from deuterium, halogen, cyano, hydroxyl, amino, C₁₋₄ alkyl, C₁₋₄ alkoxy, and C₁₋₄ alkylamino.

In some embodiments, R^{c} is halogen, cyano, C₁₋₆ alkyl, or C₆₋₁₀ aryl, wherein the C₁₋₆ alkyl or C₆₋₁₀ aryl is optionally substituted with 1, 2, 3, 4, or 5 substituents selected from deuterium, halogen, cyano, hydroxyl, amino, and C₁₋₄ alkyl.

In some embodiments, R^{c} is halogen, cyano, C₁₋₄ alkyl, or phenyl, wherein the C₁₋₄ alkyl or phenyl is optionally substituted with 1, 2, 3, 4, or 5 substituents selected from deuterium, halogen, cyano, hydroxyl, amino, and C₁₋₄ alkyl.

In some embodiments, R^{c} is F, Cl, Br, cyano, methyl, ethyl, propyl, isopropyl, difluoromethyl, trifluoromethyl, difluoroethyl, trifluoroethyl, or phenyl.

In some embodiments, 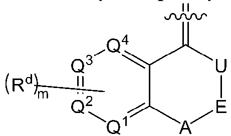 is selected from 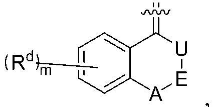 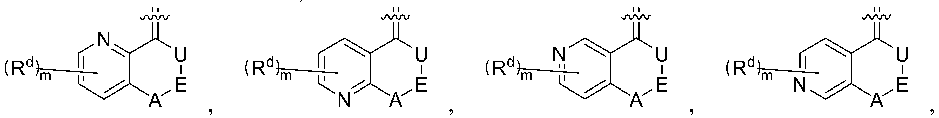 and 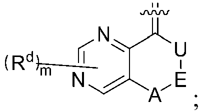 A, E, U, R^{d}, and m have the definitions as described in the present disclosure.

In some embodiments, 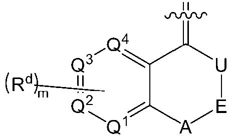 is selected from 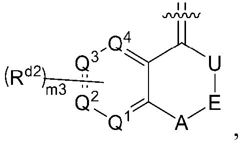 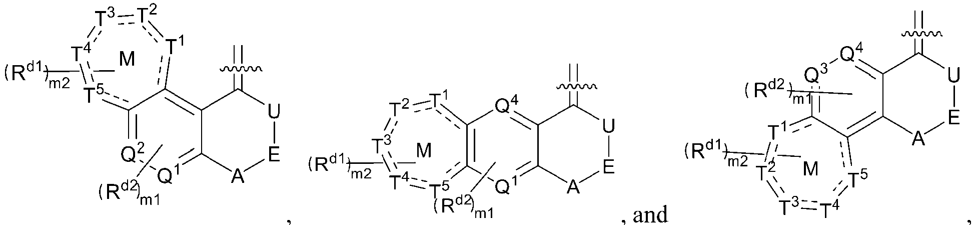
wherein T¹, T², T³, T⁴, and T⁵ are each independently a bond, carbon, nitrogen, oxygen, or sulfur; ring M is a 3-, 4-, 5-, 6-, or 7-membered ring;
the - - - - - - bonds connected to T¹, T², T³, T⁴, and T⁵ each independently represent a single bond or a double bond; two - - - - - - bonds connected to the same atom are not simultaneously double bonds; when T¹, T², T³, T⁴, and T⁵ are oxygen or sulfur, the - - - - - - bonds connected thereto are single bonds;
each R^{d2} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₆ alkyl, C₂₋₆ alkenyl, C₂₋₆ alkynyl, C₆₋₁₀ aryl, 5-to 10-membered heteroaryl, C₃₋₆ cycloalkyl, 3- to 6-membered heterocyclyl, C₆₋₁₀ aryl C₁₋₆ alkyl, 5-to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
m1 is 0, 1, or 2;
m2 is 0, 1, 2, 3, 4, or 5;
m3 is 0, 1, 2, 3, or 4; preferably, m3 is 0, 1, or 2;
A, E, U, Q1, Q2, Q3, Q4, and R^{d1} have the definitions as described in the present disclosure.

In some embodiments, each R^{d2} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R^{d2} is independently hydrogen, deuterium, F, Cl, Br, cyano, hydroxyl, sulfhydryl, nitro, amino, carboxyl, methyl, ethyl, propyl, isopropyl, tert-butyl, vinyl, ethynyl, phenyl, pyridinyl, pyrimidinyl, imidazolyl, oxazolyl, thiazolyl, cyclopropyl, cyclopentyl, cyclohexyl, oxetanyl, azetidinyl, pyrrolidinyl, piperidinyl, piperazinyl, 1,2,3,6-tetrahydropyridinyl, benzyl, 8-azabicyclo[3.2.1]octanyl, cyclopropylmethyl, piperidinylmethyl, methoxy, ethoxy, methylamino, dimethylamino, phenoxy, pyridinyloxy, cyclopropyloxy, phenylamino, cyclopropylamino, piperidinylamino, NH₂C(=O)-, methyl NHC(=O)-, ethyl NHC(=O)-, methyl C(=O)NH-, ethyl C(=O)NH-, methyl C(=O)-, ethyl C(=O)-, methyl S(=O)-, ethyl S(=O)-, methyl S(=O)₂-, methyl OC(=O)-, ethyl S(=O)₂-, ethyl OC(=O)-, methyl S(=O)NH-, ethyl S(=O)NH-, methyl S(=O)₂NH-, ethyl S(=O)₂NH-, phenyl C(=O)-, phenyl S(=O)-, phenyl S(=O)₂-, phenyl OC(=O)-, phenyl S(=O)NH-, or phenyl S(=O)₂NH-, wherein the methyl, ethyl, propyl, isopropyl, tert-butyl, vinyl, ethynyl, phenyl, pyridinyl, pyrimidinyl, imidazolyl, oxazolyl, thiazolyl, cyclopropyl, cyclopentyl, cyclohexyl, oxetanyl, azetidinyl, pyrrolidinyl, piperidinyl, piperazinyl, 1,2,3,6-tetrahydropyridinyl, benzyl, 8-azabicyclo[3.2.1]octanyl, cyclopropylmethyl, piperidinylmethyl, methoxy, ethoxy, methylamino, dimethylamino, phenoxy, pyridinyloxy, cyclopropyloxy, phenylamino, cyclopropylamino, piperidinylamino, NH₂C(=O)-, methyl NHC(=O)-, ethyl NHC(=O)-, methyl C(=O)NH-, ethyl C(=O)NH-, methyl C(=O)-, ethyl C(=O)-, methyl S(=O)-, ethyl S(=O)-, methyl S(=O)₂-, methyl OC(=O)-, ethyl S(=O)₂-, ethyl OC(=O)-, methyl S(=O)NH-, ethyl S(=O)NH-, methyl S(=O)₂NH-, ethyl S(=O)₂NH-, phenyl C(=O)-, phenyl S(=O)-, phenyl S(=O)₂-, phenyl OC(=O)-, phenyl S(=O)NH-, or phenyl S(=O)₂NH- is optionally substituted with 1, 2, 3, 4, or 5 substituents selected from deuterium, oxo (O=), thio (S=), F, Cl, Br, cyano, hydroxyl, amino, methyl, ethyl, methoxy, ethoxy, methylamino, and dimethylamino.

In some embodiments, 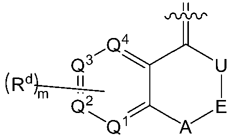 is selected from 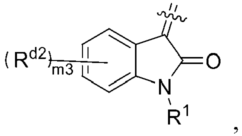 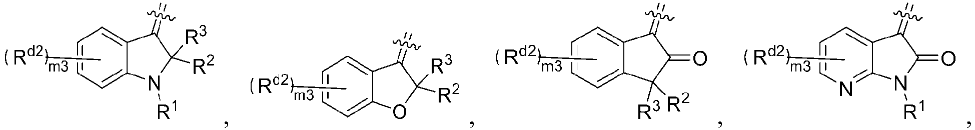 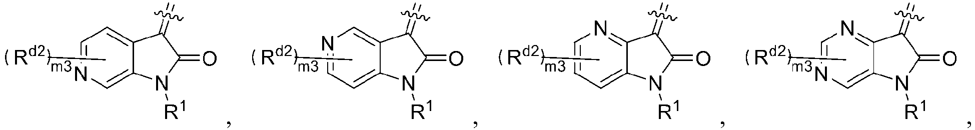 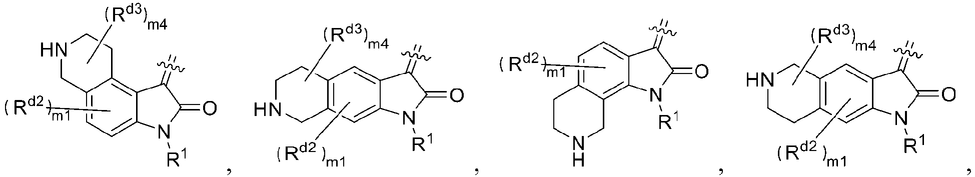 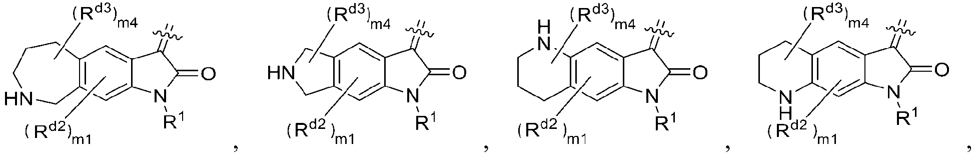 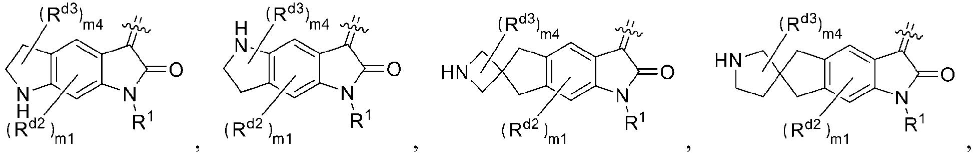 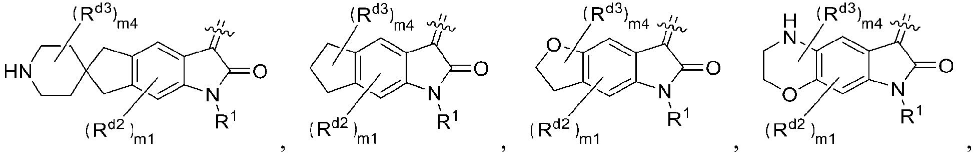 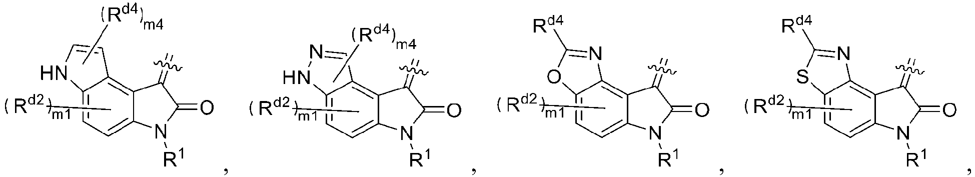 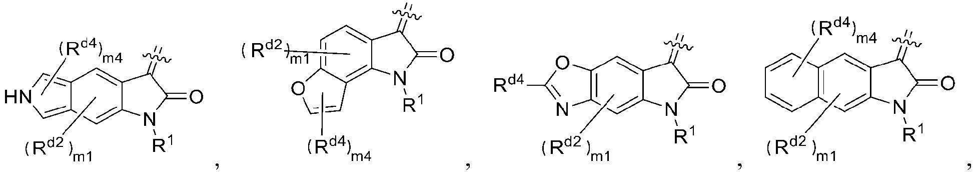 and 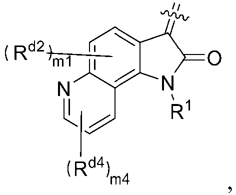 wherein
each R¹ is independently hydrogen, deuterium, C₁₋₆ alkyl, NH₂C(=O)-, C₁₋₆ alkyl C(=O)-, C₁₋₆ alkyl S(=O)-, C₁₋₆ alkyl S(=O)₂-, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl, wherein the C₁₋₆ alkyl, NH₂C(=O)-, C₁₋₆ alkyl C(=O)-, C₁₋₆ alkyl S(=O)-, C₁₋₆ alkyl S(=O)₂-, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R² and R³ are each independently hydrogen, deuterium, halogen, cyano, hydroxyl, amino, C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; or R² and R³ are connected to form oxo (O=) or thio (S=); or R² and R³, together with the C atom to which they are attached, form a 3- to 6-membered monocyclic carbocycle or a 3- to 6-membered monocyclic heterocycle; wherein the C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
each R^{d3} is independently hydrogen, deuterium, halogen, oxo (O=), thio (S=), cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3-to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
each R^{d4} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₁₋₆ alkyl NHC(=O)-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
m4 is 0, 1, or 2;
m1 and m3 have the definitions as described in the present disclosure.

In some embodiments, each R¹ is independently hydrogen, deuterium, C₁₋₄ alkyl, NH₂C(=O)-, C₁₋₄ alkyl C(=O)-, C₁₋₄ alkyl S(=O)-, C₁₋₄ alkyl S(=O)₂-, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, NH₂C(=O)-, C₁₋₄ alkyl C(=O)-, C₁₋₄ alkyl S(=O)-, C₁₋₄ alkyl S(=O)₂-, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R¹ is independently hydrogen, deuterium, methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, or imidazolyl, wherein the methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, or imidazolyl is optionally substituted with 1, 2, 3, 4, or 5 substituents selected from deuterium, oxo (O=), thio (S=), F, Cl, Br, cyano, hydroxyl, amino, methyl, ethyl, methoxy, ethoxy, methylamino, and dimethylamino.

In some embodiments, each R¹ is independently hydrogen, deuterium, methyl, ethyl, isopropyl, NH₂C(=O)-, methyl C(=O)-, ethyl C(=O)-, methyl S(=O)-, methyl S(=O)₂-, ethyl S(=O)-, ethyl S(=O)₂-, cyclopropyl, cyclobutyl, oxetanyl, azetidinyl, pyrrolyl, or imidazolyl, wherein the methyl, ethyl, isopropyl, NH₂C(=O)-, methyl C(=O)-, ethyl C(=O)-, methyl S(=O)-, methyl S(=O)₂-, ethyl S(=O)-, ethyl S(=O)₂-, cyclopropyl, cyclobutyl, oxetanyl, azetidinyl, pyrrolyl, or imidazolyl is optionally substituted with 1, 2, 3, 4, or 5 substituents selected from deuterium, F, Cl, Br, cyano, hydroxyl, amino, methyl, ethyl, methoxy, ethoxy, methylamino, and dimethylamino.

In some embodiments, R² and R³ are each independently hydrogen, deuterium, F, Cl, Br, cyano, hydroxyl, amino, C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; or R² and R³ are connected to form oxo (O=) or thio (S=); or R² and R³, together with the C atom to which they are attached, form a 3- to 6-membered monocyclic carbocycle or a 3- to 6-membered monocyclic heterocycle; wherein the C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R^{d3} is independently hydrogen, deuterium, F, Cl, Br, oxo (O=), thio (S=), cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-.

In some embodiments, each R^{d3} is independently hydrogen, deuterium, F, Cl, Br, oxo (O=), thio (S=), cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-.

In some embodiments, each R^{d3} is independently hydrogen, deuterium, F, Cl, Br, oxo (O=), thio (S=), cyano, hydroxyl, sulfhydryl, nitro, amino, carboxyl, methyl, ethyl, vinyl, ethynyl, NH₂C(=O)-, methyl NHC(=O)-, methyl C(=O)NH-, methyl C(=O)-, methyl S(=O)-, methyl S(=O)₂-, methyl OC(=O)-, methyl S(=O)NH-, methyl S(=O)₂NH-, ethyl NHC(=O)-, ethyl C(=O)NH-, ethyl C(=O)-, ethyl S(=O)-, ethyl S(=O)₂-, ethyl OC(=O)-, ethyl S(=O)NH-, ethyl S(=O)₂NH-, isopropyl NHC(=O)-, isopropyl C(=O)NH-, isopropyl C(=O)-, isopropyl S(=O)-, isopropyl S(=O)₂-, isopropyl OC(=O)-, isopropyl S(=O)NH-, isopropyl S(=O)₂NH-, tert-butyl NHC(=O)-, tert-butyl C(=O)NH-, tert-butyl C(=O)-, tert-butyl S(=O)-, tert-butyl S(=O)₂-, tert-butyl OC(=O)-, tert-butyl S(=O)NH-, or tert-butyl S(=O)₂NH-.

In some embodiments, each R^{d4} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-.

In some embodiments, each R^{d4} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-.

In some embodiments, each R^{d4} is independently hydrogen, deuterium, F, Cl, Br, cyano, hydroxyl, sulfhydryl, nitro, amino, carboxyl, methyl, ethyl, vinyl, ethynyl, NH₂C(=O)-, methyl NHC(=O)-, methyl C(=O)NH-, methyl C(=O)-, methyl S(=O)-, methyl S(=O)₂-, methyl OC(=O)-, methyl S(=O)NH-, methyl S(=O)₂NH-, ethyl NHC(=O)-, ethyl C(=O)NH-, ethyl C(=O)-, ethyl S(=O)-, ethyl S(=O)₂-, ethyl OC(=O)-, ethyl S(=O)NH-, ethyl S(=O)₂NH-, isopropyl NHC(=O)-, isopropyl C(=O)NH-, isopropyl C(=O)-, isopropyl S(=O)-, isopropyl S(=O)₂-, isopropyl OC(=O)-, isopropyl S(=O)NH-, isopropyl S(=O)₂NH-, tert-butyl NHC(=O)-, tert-butyl C(=O)NH-, tert-butyl C(=O)-, tert-butyl S(=O)-, tert-butyl S(=O)₂-, tert-butyl OC(=O)-, tert-butyl S(=O)NH-, or tert-butyl S(=O)₂NH-.

In some embodiments, 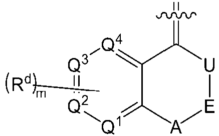 is selected from 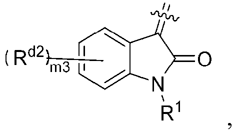 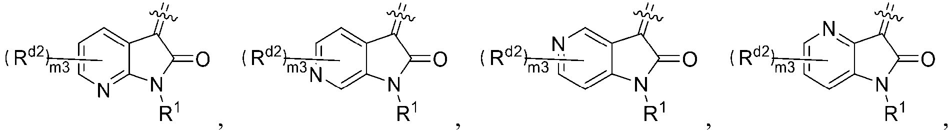 and 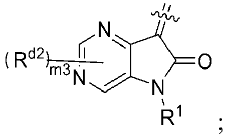 m3 is 0, 1, 2, 3, or 4; R^{d2} and R¹ have the definitions as described in the present disclosure.

In some embodiments, 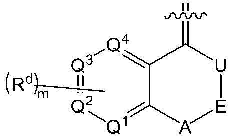 is selected from 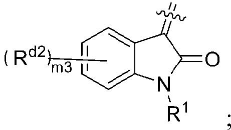 m3 is 0, 1, 2, 3, or 4; R^{d2} and R¹ have the definitions as described in the present disclosure.

In some embodiments, the compound has a structure of formula I-1, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 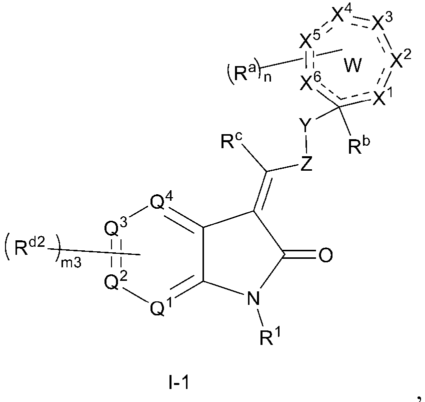 wherein X¹, X², X³, X⁴, X⁵ Q¹, Q², Q³, Q⁴, R^{a}, R^{b}, R^{c}, R^{d2}, X, Y, n, m3, and R¹ have the definitions as described in the present disclosure.

In some embodiments, the compound has a structure of formula II-1 or II-2, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof; 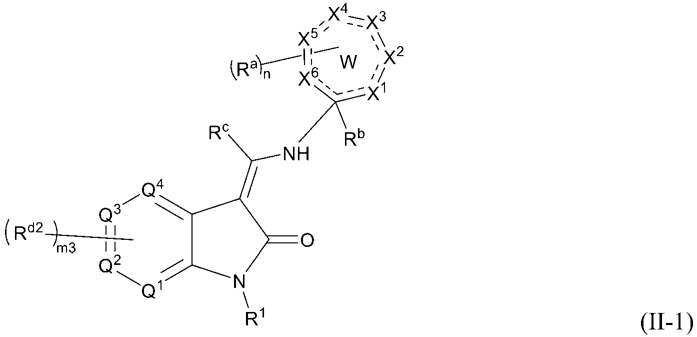 or 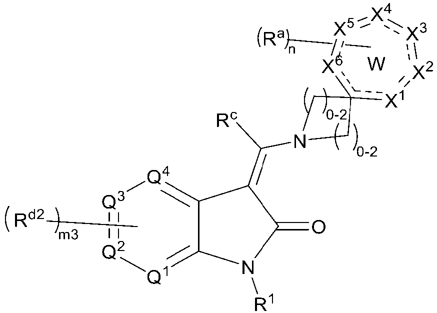 wherein X¹, X², X³, X⁴, X⁵, Q¹, Q², Q¹, Q⁴, R^{a}, R^{b}, R^{c}, R^{d2}, n, m³, and R¹ have the definitions as described in the present disclosure; 0-2 represents 0, 1, or 2.

In some embodiments, the compound has a structure of formula II-1-1, II-1-2, II-1-3, II-1-4, II-1-5, II-2-1, II-2-2, II-2-3, or II-2-4, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 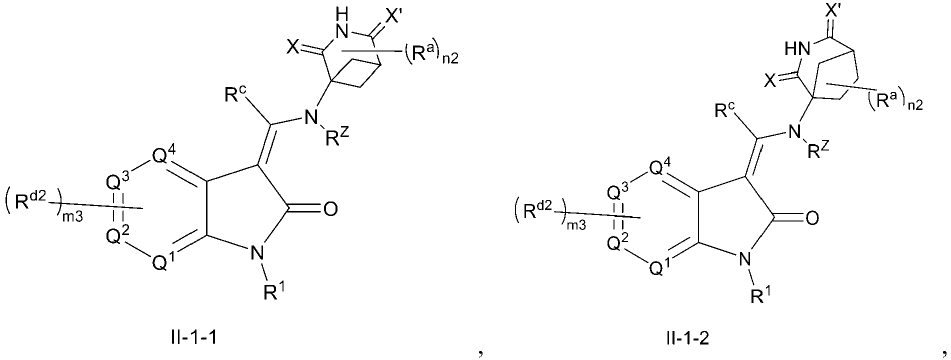 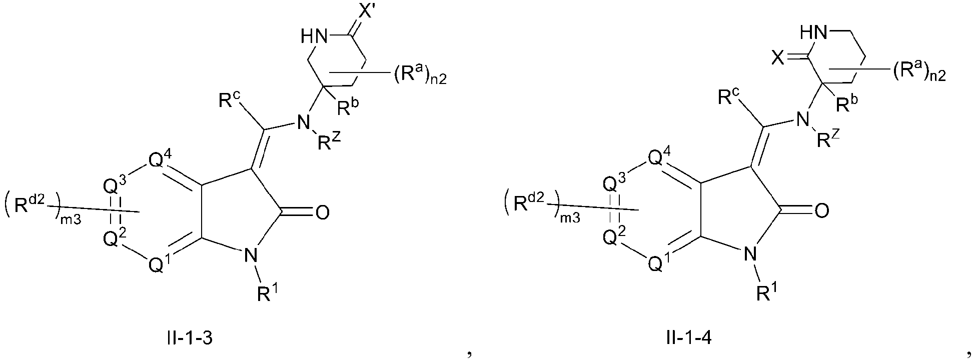 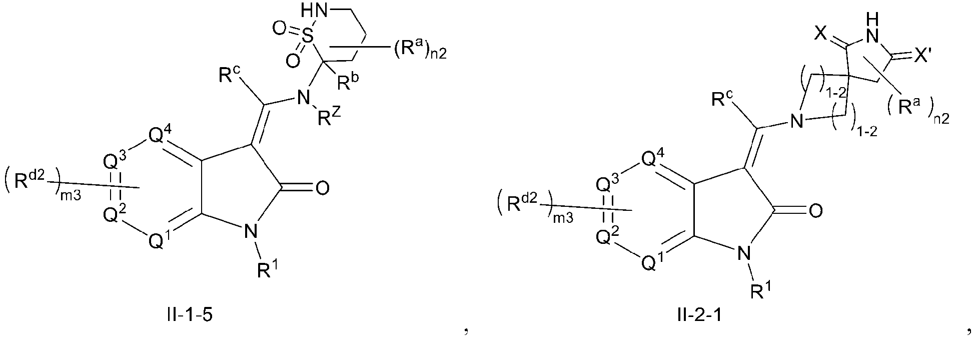 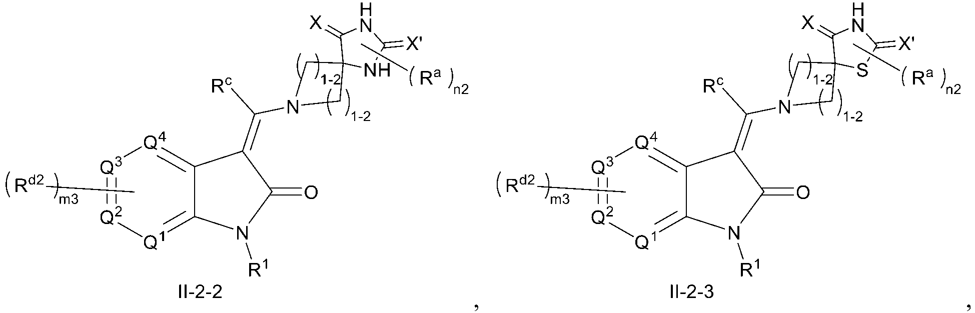 or 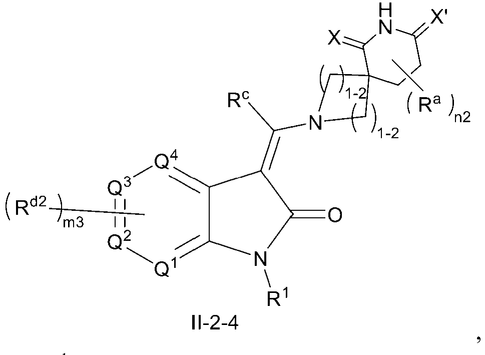 wherein Q¹, Q², Q³, Q⁴, R^{c}, R^{Z}, X, X', n₂, R^{d2}, m3, and R¹ have the definitions as described in the present disclosure;
in general formulas II-1-1, II-1-2, II-2-1, II-2-2, II-2-3, and II-2-4, each R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
in general formulas II-1-3, II-1-4, and II-1-5, there is at least one occurrence where two R^{a} on the same carbon atom are connected to form a 3- to 7-membered monocyclic carbocycle or a 3- to 7-membered monocyclic heterocycle, or there is at least one occurrence where R^{b} is connected to one R^{a} on an adjacent carbon atom to form a 3- to 7-membered monocyclic carbocycle or a 3- to 7-membered monocyclic heterocycle;
when two R^{a} on the same carbon atom are connected to form a 3- to 7-membered monocyclic carbocycle or a 3- to 7-membered monocyclic heterocycle, each remaining R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R^{b} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, or 3- to 6-membered heterocyclyl, wherein the C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, and 3- to 6-membered heterocyclyl are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
when R^{b} is connected to one R^{a} on an adjacent carbon atom to form a 3- to 7-membered monocyclic carbocycle or a 3- to 7-membered monocyclic heterocycle, each remaining R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, the compound has a structure of formula III, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 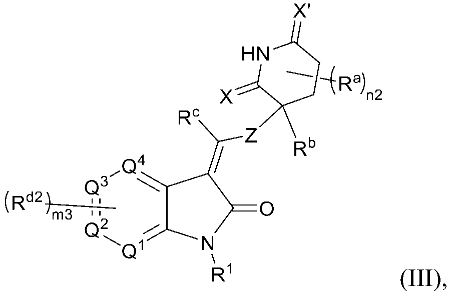 wherein Q¹, Q², Q³, Q⁴, R^{a}, R^{b}, R^{c}, Z, X, X', n2, R^{d2}, m3, and R¹ have the definitions as described in the present disclosure.

In some embodiments, the compound has a structure of formula III-1, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 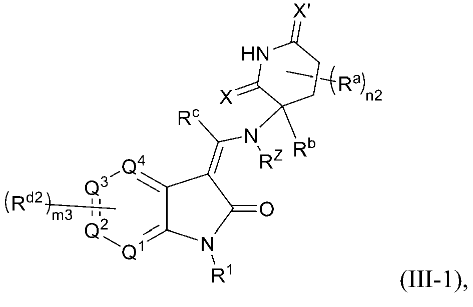 wherein X', X, Q¹, Q², Q³, Q⁴, R^{a}, R^{b}, R^{c}, R^{d2}, n2, m3, and R¹ have the definitions as described in the present disclosure.

In some embodiments, the compound has a structure of formula III-2, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 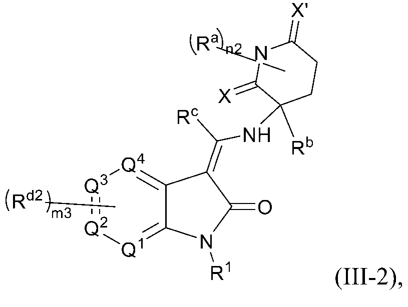 wherein X', X, Q¹, Q², Q³, Q⁴, R^{a}, R^{b}, R^{c}, R^{d2}, n2, m3, and R¹ have the definitions as described in the present disclosure.

In some embodiments, the compound has a structure represented by formula IV-1, IV-2, IV-3, IV-4, IV-5, or IV-6, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 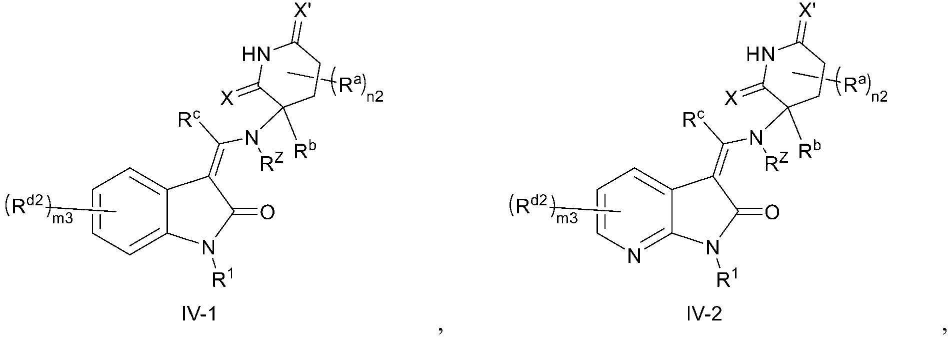 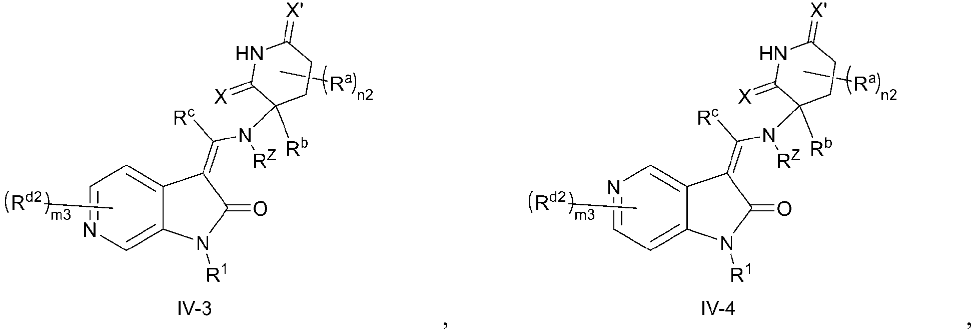 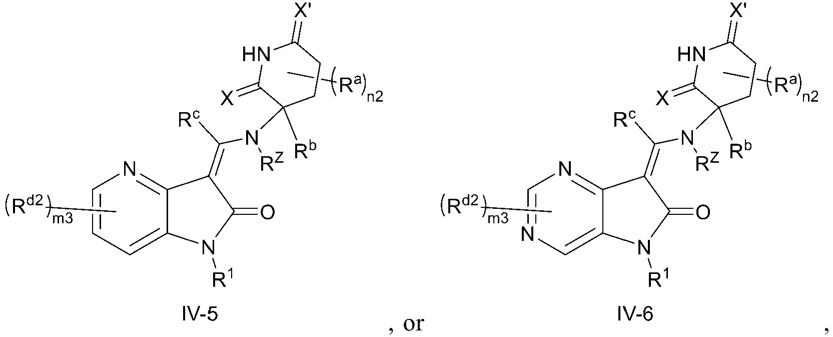 wherein R^{a}, R^{b}, R^{c}, R^{Z}, X, X', n2, R^{d2}, m3, and R¹ have the definitions as described in the present disclosure.

In some embodiments, each R^{a} is independently hydrogen, deuterium, oxo (O=), thio (S=), F, Cl, Br, cyano, hydroxyl, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3-to 6-membered heterocyclyloxy, C₆₋₁₀ arylamino, 5- to 10-membered heteroarylamino, C₃₋₆ cycloalkylamino, or 3- to 6-membered heterocyclylamino; optionally, two R^{a} are connected to form a 3- to 7-membered monocyclic carbocycle or a 3- to 7-membered monocyclic heterocycle; wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, and the 3- to 7-membered monocyclic carbocycle or 3- to 7-membered monocyclic heterocycle formed by the connection of two R^{a} are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R^{a} is independently hydrogen, deuterium, F, Cl, Br, -NH₂, -NH(C₁₋₆ alkyl), or -N(C₁₋₆ alkyl)(C₁₋₆ alkyl), or two R^{a} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle is optionally substituted with 1-3 substituents selected from deuterium, F, Cl, Br, and C₁₋₄ alkyl.

In some embodiments, n is 0, 1, 2, 3, 4, 5, or 6; in some embodiments, n is 0, 1, 2, or 3.

In some embodiments, n2 is 0, 1, 2, 3, or 4.

In some embodiments, n2 is 0, 1, or 2.

In some embodiments, X and X' are each independently O or S.

In some embodiments, X and X' are each independently O.

In some embodiments, R^{b} is hydrogen, deuterium, F, Cl, Br, cyano, hydroxyl, amino, C₁₋₄ alkyl, C₁₋₄ alkoxy, C₁₋₄ alkylamino, C₂₋₄ alkenyl, C₂₋₄ alkynyl, C₆₋₁₀ aryl, 5- to 10-membered heteroaryl, C₃₋₆ cycloalkyl, or 3- to 6-membered heterocyclyl; optionally, R^{b} and R^{a} are connected to form a 3- to 7-membered monocyclic carbocycle or a 3- to 7-membered monocyclic heterocycle; wherein the C₁₋₄ alkyl, C₁₋₄ alkoxy, C₁₋₄ alkylamino, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, and the 3- to 7-membered monocyclic carbocycle or 3- to 7-membered monocyclic heterocycle formed by the connection of R^{b} and R^{a} are each independently optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, R^{b} is H, deuterium, F, Cl, Br, or C₁₋₄ alkyl; optionally, R^{b} and R^{a} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle is optionally substituted with 1, 2, or 3 substituents selected from D and C₁₋₄ alkyl.

In some embodiments, Y is a bond, NH, CH₂, CH₂CH₂, NHCH₂, or CH₂NH; optionally, Y is substituted with 1, 2, 3, or 4 R^{Y}; each R^{Y} is independently deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, and 5- to 10-membered heteroaryl are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, Y is a bond.

In some embodiments, Z is NH, O, S, or CH₂; optionally, Z is substituted with 1 or 2 R^{Z}; each R^{Z} is independently hydrogen, deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; optionally, R^{Z} and R^{a}, or R^{Z} and R^{b}, are connected to form a 3- to 7-membered monocyclic carbocycle or a 3- to 7-membered monocyclic heterocycle; wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, 5- to 10-membered heteroaryl, and the 3- to 7-membered monocyclic carbocycle or 3- to 7-membered monocyclic heterocycle formed by the connection of R^{Z} and R^{a} or R^{Z} and R^{b} are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl; preferably, Z is NH, O, S, or CH₂; optionally, Z is substituted with 1 or 2 R^{Z}; each R^{Z} is independently hydrogen, deuterium, F, Cl, Br, cyano, hydroxyl, amino, C₁₋₄ alkyl, C₁₋₄ alkoxy, C₁₋₄ alkylamino, or C₃₋₆ cycloalkyl;

In some embodiments, R^{Z} is independently hydrogen or C₁₋₄ alkyl.

In some embodiments, R^{c} is hydrogen, deuterium, F, Cl, Br, cyano, hydroxyl, amino, C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl; preferably, R^{c} is hydrogen, deuterium, F, Cl, Br, cyano, C₁₋₄ alkyl, C₂₋₄ alkenyl, C₂₋₄ alkynyl, C₃₋₆ cycloalkyl, or 3- to 6-membered heterocyclyl.

In some embodiments, each R^{e} is independently hydrogen, deuterium, F, Cl, Br, cyano, hydroxyl, amino, oxo (O=), thio (S=), C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; optionally, two R^{e} attached to the same carbon atom, together with the atom to which they are attached, form a 3- to 7-membered monocyclic carbocycle; preferably, each R^{e} is independently hydrogen, deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, or C₃₋₆ cycloalkyl; optionally, two R^{e} attached to the same carbon atom, together with the atom to which they are attached, form a 3- to 7-membered monocyclic carbocycle.

In some embodiments, each R^{d} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₆ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-; optionally, two adjacent R^{d} are connected to form a 3- to 7-membered monocyclic carbocycle or a 3- to 7-membered monocyclic heterocycle; wherein the C₁₋₄ alkyl, C₂₋₄ alkenyl, C₂₋₄ alkynyl, C₆₋₁₀ aryl, 5-to 10-membered heteroaryl, C₃₋₆ cycloalkyl, 3- to 6-membered heterocyclyl, C₆₋₁₀ aryl C₁₋₄ alkyl, 5-to 10-membered heteroaryl C₁₋₄ alkyl, C₃₋₆ cycloalkyl C₁₋₄ alkyl, 3- to 6-membered heterocyclyl C₁₋₄ alkyl, C₁₋₆ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, C₆₋₁₀ aryl S(=O)₂NH-, and the 3- to 7-membered monocyclic carbocycle or 3- to 7-membered monocyclic heterocycle formed by the connection of two R^{d} are each independently optionally substituted with 1, 2, 3, 4, or 5 R^{d1}, each R^{d1} being independently selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, 3-to 6-membered heterocyclyl, and C₁₋₄ alkyl OC(=O)-; optionally, two R^{d1} are connected to form a 3-to 7-membered monocyclic carbocycle or a 3- to 7-membered monocyclic heterocycle, wherein the 3- to 7-membered monocyclic carbocycle or 3- to 7-membered monocyclic heterocycle formed by the connection of two R^{d1} is optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R^{d} is independently hydrogen, deuterium, F, Cl, Br, cyano, hydroxyl, sulfhydryl, nitro, amino, carboxyl, C₁₋₄ alkyl, C₂₋₄ alkynyl, 5- to 10-membered heteroaryl, 3- to 6-membered heterocyclyl, C₁₋₆ alkoxy, C₁₋₄ alkylamino, C₃₋₆ cycloalkyloxy, 3- to 6-membered heterocyclyloxy, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)₂-, C₁₋₄ alkyl OC(=O)-, or C₁₋₄ alkyl S(=O)₂NH-, wherein each R^{d} is independently optionally substituted with 1, 2, or 3 R^{d1}, each R^{d1} being independently selected from hydrogen, deuterium, F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, each R^{d} is independently hydrogen, deuterium, F, Cl, Br, cyano, hydroxyl, amino, or C₁₋₄ alkyl.

In some embodiments, each R^{d2} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

In some embodiments, R^{d2} is independently hydrogen, deuterium, F, Cl, Br, cyano, hydroxyl, sulfhydryl, nitro, amino, carboxyl, C₁₋₄ alkyl, C₆₋₁₀ aryl, 5- to 10-membered heteroaryl, C₃₋₆ cycloalkyl, or 3- to 6-membered heterocyclyl, wherein the C₁₋₄ alkyl, C₆₋₁₀ aryl, 5- to 10-membered heteroaryl, C₃₋₆ cycloalkyl, or 3- to 6-membered heterocyclyl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, and C₁₋₄ alkylamino.

In some embodiments, R^{d2} is independently hydrogen, F, Cl, Br, cyano, hydroxyl, amino, or C₁₋₄ alkyl, wherein the C₁₋₄ alkyl is optionally substituted with 1, 2, 3, 4, or 5 substituents selected from deuterium, F, Cl, Br, cyano, hydroxyl, amino, and C₁₋₄ alkyl.

In some embodiments, m3 is 0, 1, 2, 3, or 4.

In some embodiments, m3 is 0, 1, or 2.

In some embodiments, the compound has a structure of formula IV, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 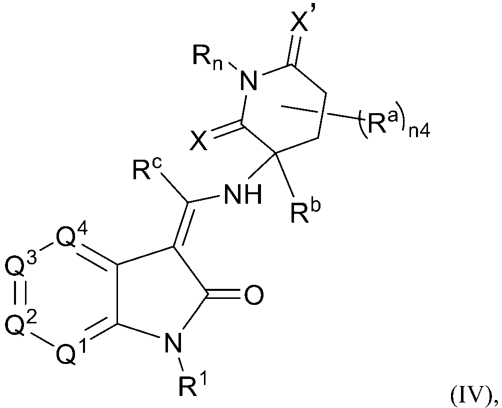 wherein X' and X are independently selected from O and S;
Q¹, Q², Q³, and Q⁴ are CR_{q} or N, each R_{q} being independently selected from H, deuterium, halogen, hydroxyl, cyano, carboxyl, amino, C₁₋₆ alkyl, C₁₋₆ alkoxy, C₁₋₆ haloalkyl, C₁₋₆ haloalkoxy, C₃₋₈ cycloalkyl, -NH(C₁₋₆ alkyl), -N(C₁₋₆ alkyl)(C₁₋₆ alkyl), -COR_{q1}, -C(O)O-R_{q1}, -C(O)NHR_{q2}, - C(O)NR_{q2}R_{q3}, C₆₋₁₀ aryl, 5- to 7-membered heterocyclyl, or 5- to 7-membered heteroaryl, wherein each R_{q1} is independently selected from H, C₁₋₆ alkyl, C₁₋₆ haloalkyl, and C₃₋₆ cycloalkyl, and R_{q2} and R_{q3} are each independently selected from H, C₁₋₆ alkyl, C₁₋₆ haloalkyl, and C₃₋₆ cycloalkyl;
R¹ is selected from H, deuterium, C₁₋₆ alkyl, C₁₋₆ haloalkyl, C₃₋₆ cycloalkyl, and -COR_{q1}, wherein R_{q1} is selected from H, C₁₋₆ alkyl, C₁₋₆ haloalkyl, and C₃₋₆ cycloalkyl;
R^{c} is selected from H, deuterium, C₁₋₆ alkyl, C₁₋₆ haloalkyl, and C₃₋₆ cycloalkyl;
Rn is selected from H, deuterium, C₁₋₆ alkyl, C₁₋₆ haloalkyl, C₃₋₆ cycloalkyl, and C₆₋₁₀ aryl-C₁₋₆ alkyl;
R^{a} is selected from deuterium, halogen, C₁₋₆ alkyl, C₁₋₆ haloalkyl, and C₃₋₆ cycloalkyl, or two R^{a} attached to the same carbon atom, together with the carbon atom to which they are attached, form C₃₋₆ cycloalkyl;
R^{b} is selected from H, deuterium, halogen, and C₁₋₆ alkyl, or one R^{a} is connected to R^{b} to form C₁₋₄ alkylene;
n4 is 0, 1, 2, 3, or 4.

In some embodiments, the compound has a structure of formula IV, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 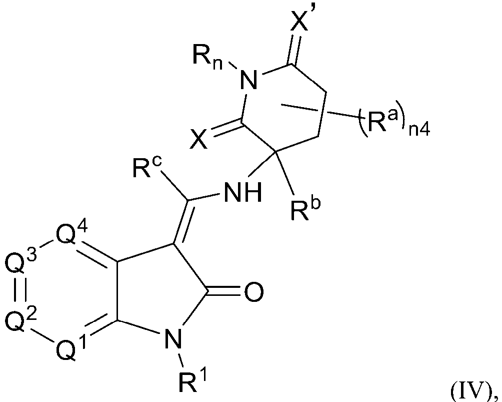 wherein X' and X are independently O or S;
at most one of Q¹, Q², Q³, and Q⁴ is N, and the others are CR_{q}, each R_{q} being independently selected from H, deuterium, halogen, hydroxyl, cyano, carboxyl, amino, C₁₋₆ alkyl, C₁₋₆ alkoxy, C₁₋₆ haloalkyl, C₁₋₆ haloalkoxy, C₃₋₈ cycloalkyl, -NH(C₁₋₆ alkyl), and -N(C₁₋₆ alkyl)(C₁₋₆ alkyl);
R¹ is selected from H, deuterium, C₁₋₆ alkyl, C₁₋₆ haloalkyl, C₃₋₆ cycloalkyl, and -COR_{q1}, wherein R_{q1} is selected from H and C₁₋₆ alkyl;
R^{c} is selected from H, deuterium, C₁₋₆ alkyl, and C₁₋₆ haloalkyl;
Rn is selected from H, deuterium, C₁₋₆ alkyl, and C₁₋₆ haloalkyl;
R^{a} is selected from deuterium, halogen, C₁₋₆ alkyl, and C₁₋₆ haloalkyl;
R^{b} is selected from H and deuterium;
n4 is 0, 1, 2, 3, or 4.

In some embodiments, the compound has a structure selected from the following, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof:

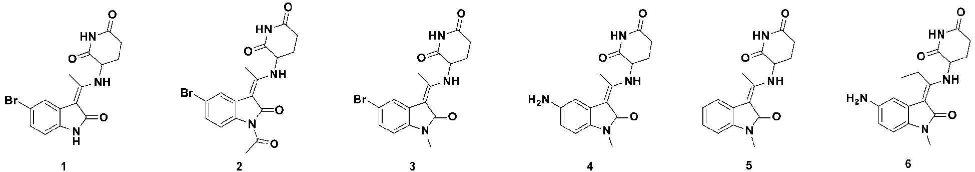

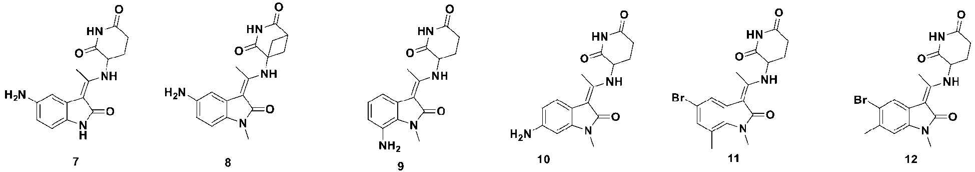

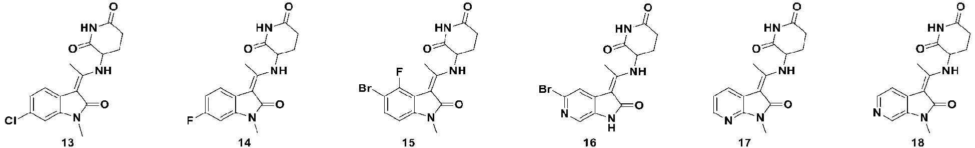

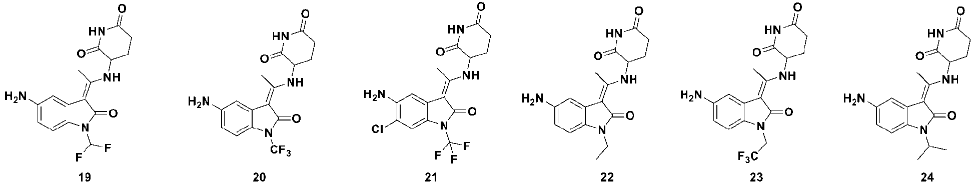

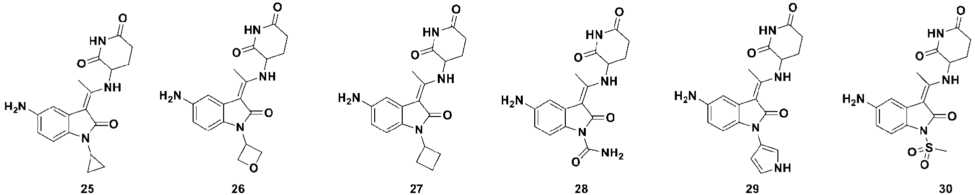

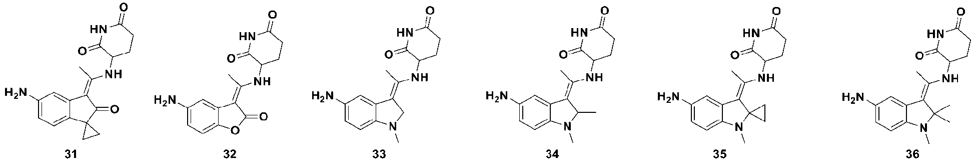

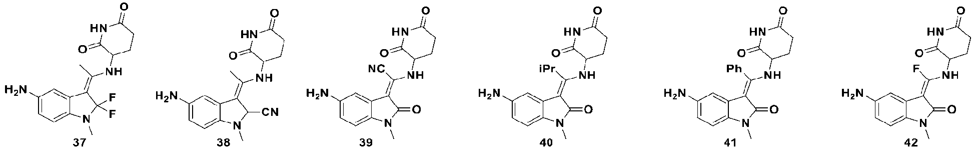

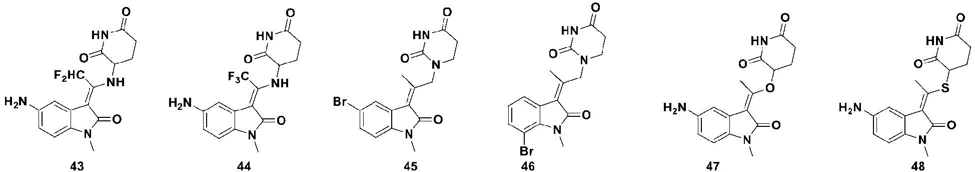

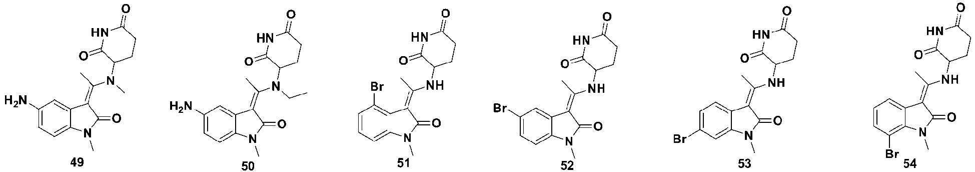

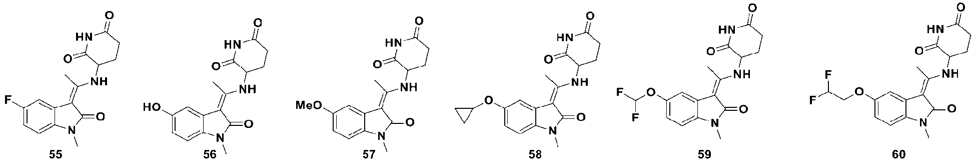

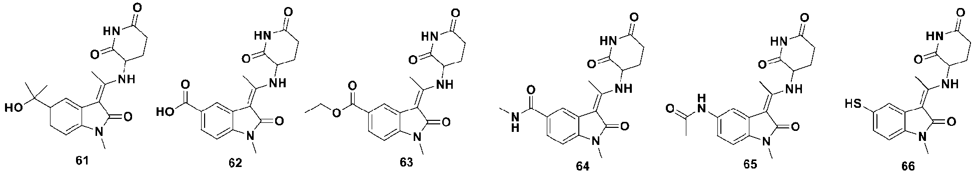

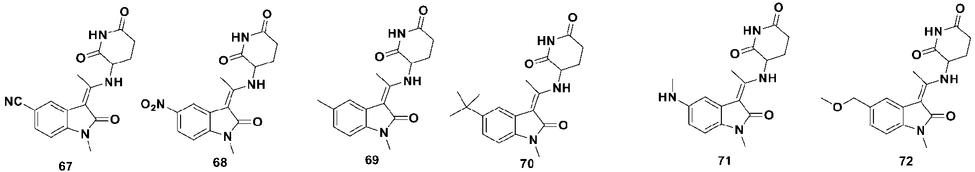

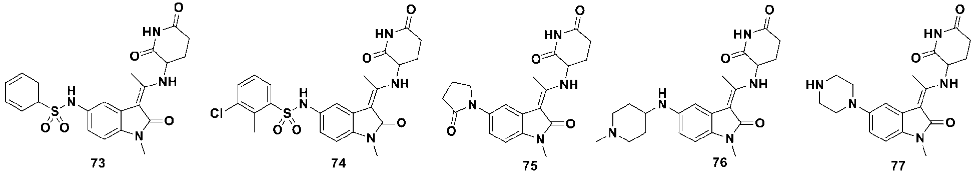

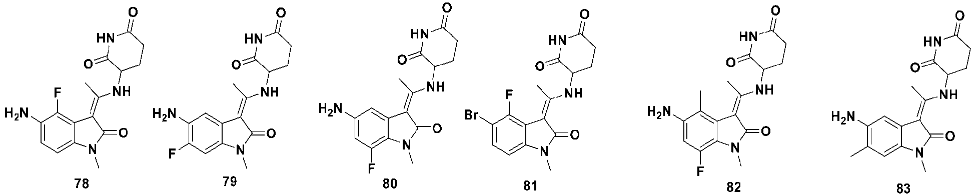

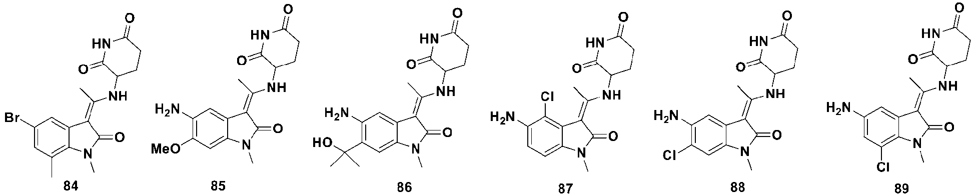

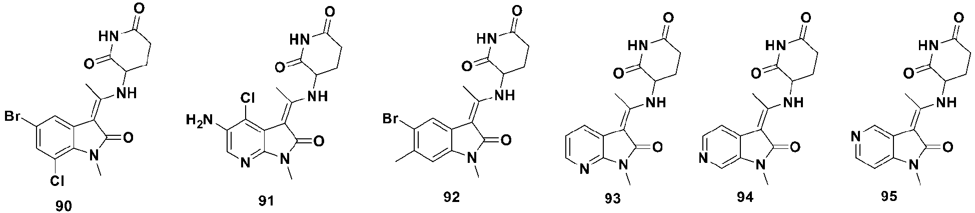

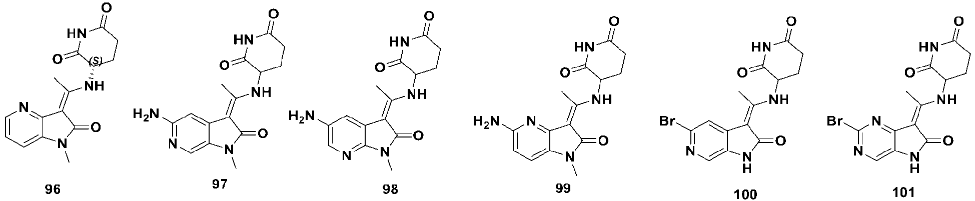

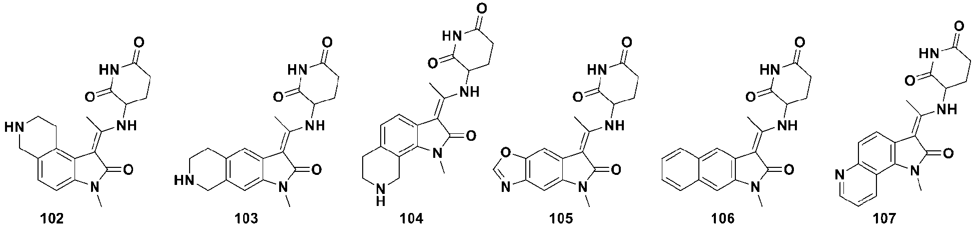

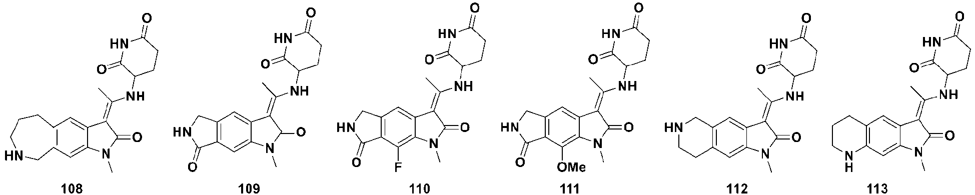

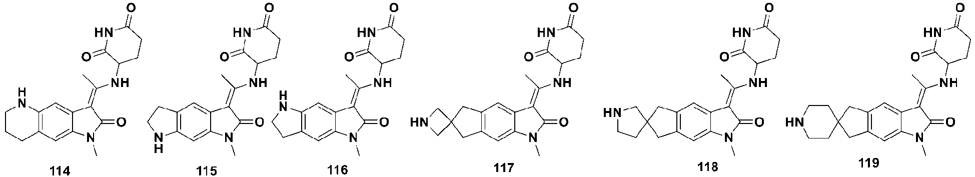

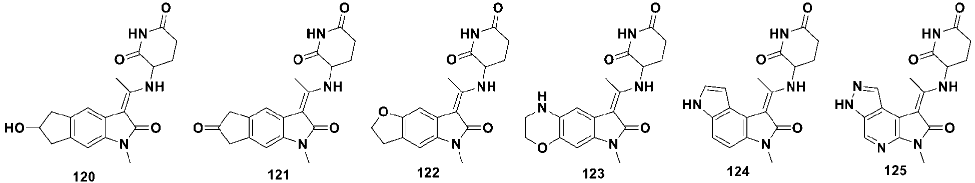

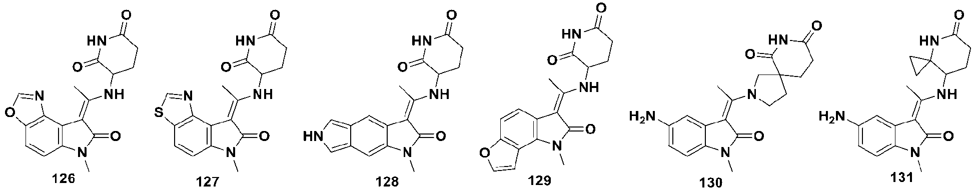

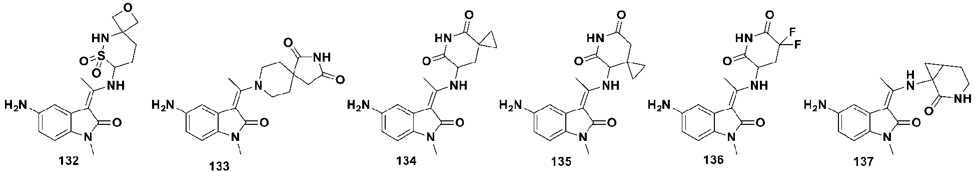

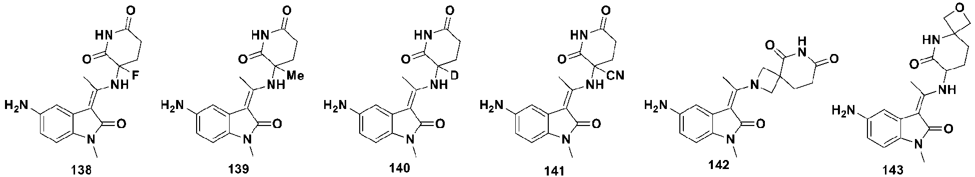

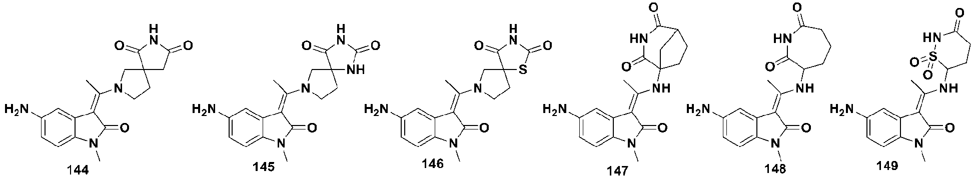

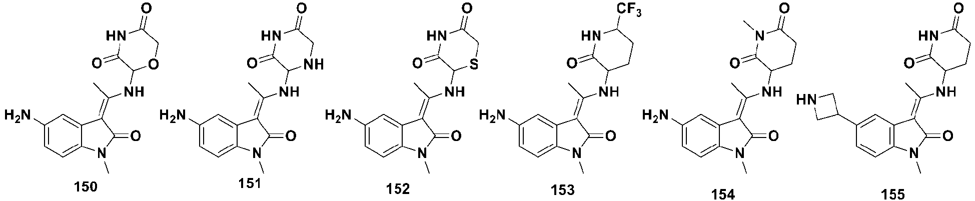

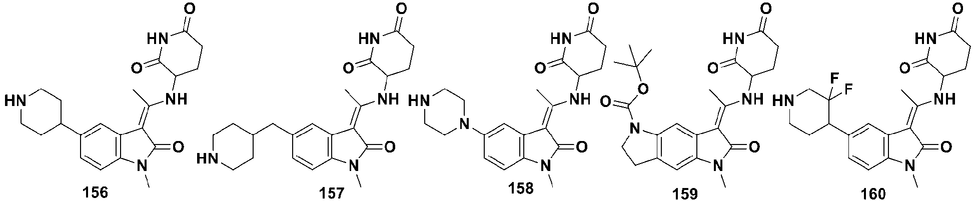

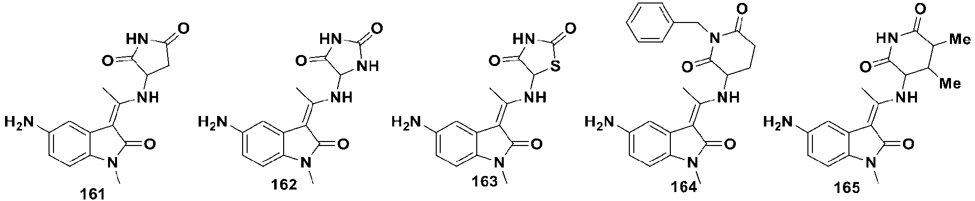

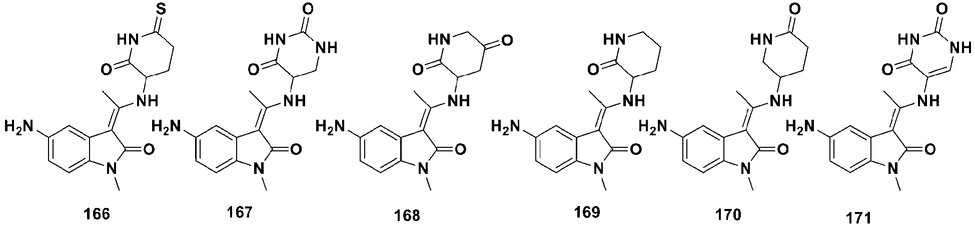

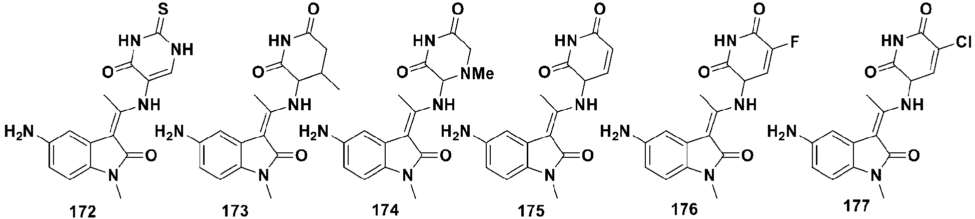

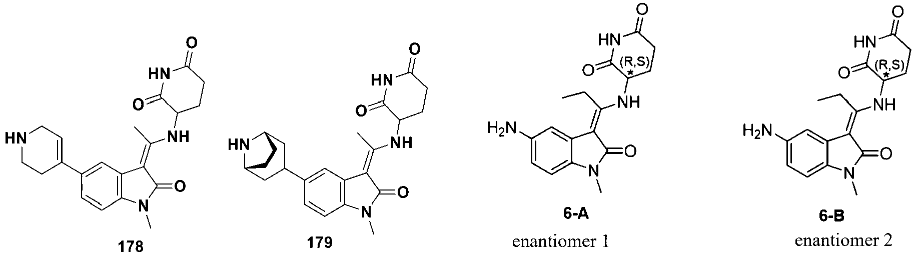

In another aspect, the present disclosure provides a PROTAC molecule, which comprises a ligand capable of binding to CRBN, wherein the ligand comprises or is based on any compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of the embodiments of the present disclosure.

In another aspect, the present disclosure provides a pharmaceutical composition, which comprises: the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure; and at least one pharmaceutically acceptable carrier.

In another aspect, the present disclosure provides the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure, or the PROTAC molecule described in the present disclosure, or the pharmaceutical composition of the present disclosure for use in treating a CRBN-mediated disease or disorder.

In another aspect, the present disclosure provides use of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof of the present disclosure, or the PROTAC molecule described in the present disclosure, or the pharmaceutical composition of the present disclosure in the preparation of a medicament, wherein the medicament is used for treating a CRBN-mediated disease or disorder.

In another aspect, the present disclosure provides a method for treating a CRBN-mediated disease or disorder, which comprises administering to a human in need thereof a therapeutically effective amount of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof of the present disclosure, or the PROTAC molecule described in the present disclosure, or the pharmaceutical composition of the present disclosure.

In some embodiments, the CRBN-mediated disease or disorder includes a proliferative disorder, a neurological disorder, and a transplantation-related disorder.

In some embodiments, the CRBN-mediated disorder or disease is a proliferative disease.

In some embodiments, the proliferative disease is leukemia; the leukemia is selected from acute leukemia, acute lymphocytic leukemia (ALL), chronic lymphocytic leukemia (CLL), acute myelogenous leukemia, acute myeloid leukemia (AML), adult acute basophilic leukemia, adult acute eosinophilic leukemia, adult acute megakaryocytic leukemia, adult acute leukemia, differentiated myeloid leukemia, adult acute monocytic leukemia, adult acute myeloid leukemia with maturation, adult acute myeloid leukemia without maturation, adult acute myeloid leukemia with abnormalities, adult acute myelomonocytic leukemia, adult erythroleukemia, adult pure erythroid leukemia, secondary acute myeloid leukemia, untreated adult acute myeloid leukemia, adult acute myeloid leukemia in remission, adult acute promyelocytic leukemia with PML-RARA, alkylating agent-related acute myeloid leukemia, prolymphocytic leukemia, or chronic myelomonocytic leukemia.

In some embodiments, the proliferative disease is lymphoma; the lymphoma is selected from adult grade III lymphomatoid granulomatosis, adult nasal-type extranodal NK/T-cell lymphoma, anaplastic large cell lymphoma, angioimmunoblastic T-cell lymphoma, cutaneous B-cell non-Hodgkin lymphoma, extranodal marginal zone lymphoma of mucosa-associated lymphoid tissue, hepatosplenic T-cell lymphoma, intraocular lymphoma, lymphomatous involvement of non-cutaneous extranodal site, mature T-cell and NK-cell non-Hodgkin lymphoma, nodal marginal zone lymphoma, post-transplant lymphoproliferative disorder, recurrent adult Burkitt lymphoma, recurrent adult diffuse large cell lymphoma, recurrent adult diffuse mixed cell lymphoma, recurrent adult diffuse small cleaved cell lymphoma, recurrent adult grade III lymphomatoid granulomatosis, recurrent adult immunoblastic lymphoma, recurrent adult lymphoblastic lymphoma, recurrent adult T-cell leukemia/lymphoma, recurrent cutaneous T-cell non-Hodgkin lymphoma, recurrent grade 1 follicular lymphoma, recurrent grade 2 follicular lymphoma, recurrent grade 3 follicular lymphoma, recurrent mantle cell lymphoma, recurrent marginal zone lymphoma, recurrent mycosis fungoides and Sézary syndrome, recurrent small lymphocytic lymphoma, refractory chronic lymphocytic leukemia, refractory hairy cell leukemia, Richter syndrome, small intestinal lymphoma, splenic marginal zone lymphoma, T-cell large granular lymphocyte leukemia, testicular lymphoma, Waldenstrom macroglobulinemia, adult T-cell leukemia-lymphoma, peripheral T-cell lymphoma, B-cell lymphoma, Hodgkin's disease, cutaneous T-cell lymphoma, diffuse large B-cell lymphoma, MALT lymphoma, mantle cell lymphoma, non-Hodgkin lymphoma, central nervous system lymphoma, refractory primary cutaneous large B-cell lymphoma (leg type), recurrent or refractory chronic lymphocytic leukemia, refractory anemia, refractory anemia with excess blasts, refractory anemia with ringed sideroblasts, refractory cytopenia with multilineage dysplasia, and secondary myelodysplastic syndrome.

In some embodiments, the CRBN-mediated disorder or disease is a neurological disease.

In some embodiments, the neurological disease is Alzheimer's disease.

In some embodiments, the CRBN-mediated disorder or disease is a transplantation-related disease, specifically graft-versus-host disease.

### Pharmaceutical Composition and Route of Administration

The present disclosure relates to a pharmaceutical composition, which comprises: the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof described in the present disclosure; and a pharmaceutically acceptable carrier.

The term "pharmaceutical composition" refers to a mixture of one or more of the compounds or the physiologically/pharmaceutically acceptable salts or prodrugs thereof described herein, and other chemical components. The other components are, for example, physiologically/pharmaceutically acceptable carriers and diluents, and may further include auxiliary materials such as excipients, binders, and fillers, as well as additional therapeutic agents such as antidiabetic agents, antihyperglycemic agents, antiobesity agents, antihypertensive agents, antiplatelet agents, antiatherosclerotic agents, or lipid-lowering agents. The purpose of the pharmaceutical composition is to facilitate the administration of the compound to an organism.

As used herein, the term "pharmaceutically acceptable carrier" refers to a substance that can be used in the preparation or administration of a pharmaceutical composition, and includes, for example, suitable diluents, solvents, dispersion media, surfactants, antioxidants, preservatives, isotonic agents, buffers, emulsifiers, absorption-delaying agents, salts, pharmaceutical stabilizers, binders, excipients, disintegrants, lubricants, wetting agents, sweeteners, flavoring agents, dyes, and combinations thereof, as known to those skilled in the art (see, for example, Remington: The Science and Practice of Pharmacy, 22nd Edition, Pharmaceutical Press, 2013, pp. 1049-1070).

The present disclosure further relates to a pharmaceutical composition comprising, as an active ingredient, a compound of formula I, II-1, II-2, or III, or a pharmaceutically acceptable salt thereof, which can be especially used for treating neoplastic diseases, particularly cancers, as described herein. The composition may be formulated for non-parenteral administration, such as nasal, oral, rectal, pulmonary, vaginal, sublingual, topical, transdermal, or ocular administration, or particularly for oral administration, for example, in the form of an oral solid dosage form, such as a granule, a pill, a powder, a tablet, a film-coated or sugar-coated tablet, an effervescent tablet, a hard or soft capsule, or a hydroxypropyl methylcellulose (HPMC) capsule (coated as applicable), an orally disintegrating tablet, an oral solution, a lipid emulsion, or a suspension; or it may be formulated for parenteral administration, such as intravenous, intramuscular or subcutaneous, intrathecal, intradermal, or epidural administration to mammals, particularly humans, for example, in the form of a solution, a lipid emulsion, or a suspension containing microparticles or nanoparticles. Such compositions may contain the active ingredient alone or, preferably, in combination with a pharmaceutically acceptable carrier.

The compound of formula I, II-1, II-2, or III, or the pharmaceutically acceptable salt thereof can be processed with a pharmaceutically inert inorganic or organic excipient to produce an oral solid dosage form, such as a granule, a pill, a powder, a tablet, a film-coated or sugar-coated tablet, an effervescent tablet, a hard capsule or an HPMC capsule, or an orally disintegrating tablet. Fillers (e.g., lactose, cellulose, mannitol, sorbitol, calcium phosphate, starch or derivatives thereof), binders (e.g., cellulose, starch, polyvinylpyrrolidone or derivatives thereof), glidants (e.g., talc or stearic acid or salts thereof), and flow agents (e.g., fumed silicon dioxide) may be used as such excipients for the formulation and manufacture of oral solid dosage forms, such as granules, pills, powders, tablets, film-coated or sugar-coated tablets, effervescent tablets, hard capsules or HPMC capsules, or orally disintegrating tablets. Suitable excipients for soft capsules are, for example, vegetable oils, waxes, fats, semi-solid and liquid polyols, etc.

Suitable excipients for the manufacture of oral solutions, lipid emulsions, or suspensions are, for example, water, alcohols, polyols, sucrose, invert sugar, glucose, etc.

Suitable excipients for parenteral formulations are, for example, water, alcohols, polyols, glycerol, vegetable oils, lecithin, surfactants, etc.

Furthermore, pharmaceutical formulations may contain preservatives, solubilizers, stabilizers, wetting agents, emulsifiers, sweeteners, colorants, flavoring agents, salts for altering osmotic pressure, buffers, masking agents, or antioxidants. Pharmaceutical preparations may also contain other therapeutically valuable substances.

The dose can vary within a wide range and will, of course, be adapted to suit individual requirements in each specific case. Usually, in the case of oral administration, a daily dose of about 1 to 1000 mg of the compound of general formula I per person should be appropriate, although it may fall below the lower limit or exceed the upper limit mentioned above when necessary.

The compound of formula I, II-1, II-2, or III may also be used in combination with one or more other pharmacologically active compounds that are also effective against the same disease, preferably with a different mode of action, or that reduce or prevent possible undesirable side effects of the compound of formula I, II-1, II-2, or III. The combination partners can be administered simultaneously in such treatment, for example, by incorporating them into a single pharmaceutical formulation, or administered sequentially by administering two or more different dosage forms (each containing one or more of the combination partner).

The term "therapeutically effective amount" of the compound of the present disclosure refers to an amount of the compound of the present disclosure that will elicit a biological or medical response (e.g., decrease or inhibition of enzyme or protein activity, or amelioration of symptoms, alleviation of disorders, slowing or delay of disease progression, or prevention of diseases, etc.) in a subject. In one non-limiting embodiment, the term "therapeutically effective amount" refers to an amount of the compound of the present disclosure that, when administered to a subject, is effective in at least partially alleviating, inhibiting, preventing, and/or ameliorating a CRBN-mediated disease, disorder, or condition.

The term "treatment" or "treating" as used herein in the context of treating a disease or disorder generally refers to treatment and therapy in humans or animals (e.g., in veterinary applications), where some desired therapeutic effects are achieved, for example, inhibiting the progression of the disease or disorder. This also includes reducing the rate of progression, halting the rate of progression, alleviating symptoms of the disease or disorder, ameliorating the disease or disorder, and curing the disease or disorder. Treatment as a preventative measure (i.e., prophylaxis) is also included. For example, use in patients who have not yet developed the disease or disorder but are at risk of developing the disease or disorder is encompassed by the term "treatment" or "treating". For example, treatment includes prevention of cancer, reduction of cancer incidence, alleviation of cancer symptoms, etc.

In some embodiments, the present disclosure provides use of compound I, II-1, II-2, or III, or the pharmaceutical composition or the pharmaceutically acceptable salt thereof in the preparation of a medicament for treating a CRBN-mediated disorder or disease.

In some embodiments, the present disclosure provides a method for treating a CRBN-mediated disorder or disease, which comprises administering a therapeutically effective amount of compound I, II-1, II-2, or III, or the pharmaceutically acceptable salt thereof.

In some embodiments, the present disclosure provides compound I, II-1, II-2, or III, or the pharmaceutical composition for use in treating a CRBN-mediated disorder or disease.

### Synthesis method

The compounds of formulas I, II-1, II-2, and III can be synthesized by the methods given below, by the methods provided in the experimental section below, or by analogous methods. The schemes described herein are not intended to present an exhaustive list of methods for preparing the compounds of formulas I, II-1, II-2, and III; instead, other techniques known to skilled chemists may also be used for compound synthesis.

The structures of the compounds were determined by nuclear magnetic resonance (NMR) spectroscopy or/and mass spectrometry (MS). NMR shifts (δ) are expressed in 10⁻⁶ (ppm). NMR analyses were performed using a Bruker Advance III-400M nuclear magnetic resonance spectrometer, with deuterated chloroform (CDCl₃), deuterated methanol (CD₃OD) or deuterated dimethyl sulfoxide (DMSO-d₆) as a solvent and tetramethylsilane (TMS) as an internal standard. When multiple peaks appear, the following abbreviations will be used: s (singlet), d (doublet), t (triplet), m (multiplet), br (broadened), dd (doublet of doublets), dt (doublet of triplets), td (triplet of doublets), and brs (broadened singlet). Coupling constants J are expressed in Hertz (Hz).

Liquid chromatography-mass spectrometry (LC-MS) analyses were performed using an Agilent mass spectrometer (Agilent 1260, Agilent 6125B). High-performance liquid chromatography (HPLC) analyses were performed using a Gilson high-pressure liquid chromatograph (Gilson GX-281), with a C18 column (10 µM, 19 mm × 250 mm), UV detection wavelengths at 220 nm and 254 nm, and an elution condition of gradient elution with 5%-95% acetonitrile (containing 0.05% v/v formic acid or ammonium bicarbonate) for 15 min.

A Biotage Isolera rapid purification system was used for reverse-phase purification.

Biotage Initiator+ (400 W, RT, ~300 °C) microwave reactor was used for microwave reactions.

The thin layer chromatography silica gel plates used were Qingdao GF254 silica gel plates. The specification of silica plates adopted for thin-layer chromatography (TLC) was 0.15-0.20 mm, while preparative thin-layer chromatography used 0.4-0.5 mm plates. Silica gel column chromatography generally used Qingdao 200- to 300-mesh silica gel as the carrier.

The starting materials in the examples of the present disclosure are all known and commercially available, or can be synthesized by adopting or according to the literature reported in the art.

Unless otherwise specified, all the reactions in the present disclosure are carried out under the protection of a dry inert gas (e.g., nitrogen or argon) with continuous magnetic stirring, and the reaction temperature is room temperature, ranging from 20 °C to 30 °C.

Those skilled in the art of organic synthesis will appreciate that optimal reaction conditions may vary depending on the specific reactants or solvents used, but such conditions can be determined by routine optimization procedures. In some cases, the order in which the following reaction schemes and/or reaction steps are performed may be altered to facilitate the reaction or to avoid the formation of undesirable side products. Furthermore, the functional groups present at various positions in the molecule must be compatible with the proposed reagents and reactions. Such restrictions on the substituents that are compatible with the reaction conditions are apparent to those skilled in the art, and alternative methods must then be employed. Furthermore, in some of the reactions mentioned herein, it may be necessary or desirable to protect any sensitive groups in the compound, and it is assumed that such protective groups (PG) are in place where necessary. Conventional protective groups can be used according to standard practices well known in the art (for an illustration, see Greene T.W., Wuts P.G.M., Protective Groups in Organic Synthesis, 5th Edition, Publisher: John Wiley & Sons, 2014). The protective groups may be removed at any convenient stage in the synthesis using conventional techniques well known in the art, or the protective groups may be removed during subsequent reaction steps or work-up.

The following abbreviations are used throughout the present disclosure:
LCMS: liquid chromatography-mass spectrometry;
M, mol/L: mole per liter;
ml, mL: milliliter;
g: gram;
mmol: millimole;
°C: degree Celsius;
HNO₃: nitric acid;
H₂SO₄: sulfuric acid;
Ac₂O: acetic anhydride;
SnCl₂: stannous chloride;
H₂O: water; and
DMSO: dimethyl sulfoxide.

The examples provided below can aid in understanding the present disclosure. However, it will be appreciated that these examples and accompanying drawings are merely intended to illustrate the present disclosure, but do not constitute any limitation thereon. The actual protection scope of the present disclosure is illustrated in the claims. It will be appreciated that any modifications and changes may be made without departing from the spirit of the present disclosure.

### Example 1

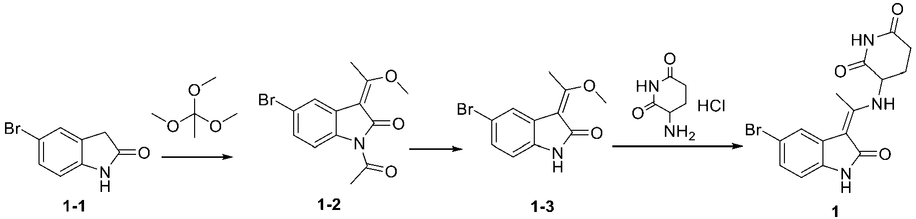

### Step 1: Preparation of compound 1-2

1,1,1-Trimethoxyethane (1.21 mL, 9.43 mmol) was added to a solution of **compound 1-1** (1.00 g, 4.72 mmol) in acetic anhydride (10 mL), and the mixture was stirred at 90 °C under a nitrogen atmosphere for 18 h. The reaction was monitored by LCMS. The mixture was concentrated to obtain a crude product. The crude product was separated and purified by column chromatography (eluent: petroleum ether/dichloromethane = 1/1 to 1/100) to obtain **compound 1-2.** LC-MS: m/z: 310.1 [M+H]⁺.

### Step 2: Preparation of compound 1-3

Potassium carbonate (103 mg, 0.75 mmol) was added to a solution of **compound 1-2** (230 mg, 0.74 mmol) in methanol (5 mL), and the mixture was stirred at 25 °C for 18 h. The mixture was concentrated to obtain a crude product. The crude product was purified by silica gel column chromatography (eluent: dichloromethane/methanol = 100/1 to 20/1) to obtain **compound 1-3.** LC-MS: m/z: 268.1 [M+H]⁺.

### Step 3: Preparation of compound 1

3-Aminohexahydropyridine-2,6-dione hydrochloride (50 mg, 0.30 mmol) and triethylamine (61 mg, 0.60 mmol) were added to a solution of **compound 1-3** (80 mg, 0.30 mmol) in 1,4-dioxane (2 mL), and the mixture was stirred at 100 °C for 4 h. The mixture was then poured into water (20 mL) to form a suspension. The suspension was filtered, and the filter cake was washed with water (20 mL). The filter cake was then dried under vacuum to obtain **compound 1.** LC-MS: m/z: 366.0 [M+H]⁺.

¹H NMR (400 MHz, DMSO-*d₆*) *δ* 11.01 (s, 1H), 10.69 (d, *J =* 8.4 Hz, 1H), 10.53 (s, 1H), 7.44 (d, *J =* 2.0 Hz, 1H), 7.08 (dd, *J=* 8.0, 2.0 Hz, 1H), 6.79 (d, *J=* 8.0 Hz, 1H), 4.97 - 4.85 (m, 1H), 2.85 - 2.73 (m, 1H), 2.62 - 2.54 (m, 1H), 2.45 (s, 3H), 2.23 - 2.11 (m, 2H).

### Example 2

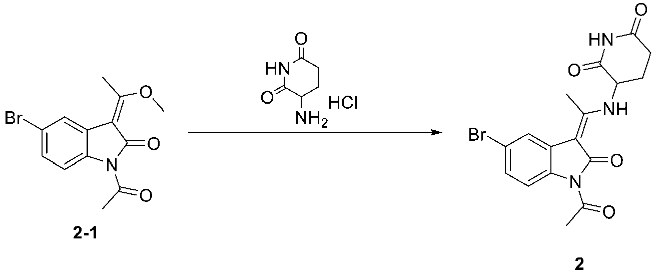

### Step: Preparation of compound 2

3-Aminohexahydropyridine-2,6-dione hydrochloride (167 mg, 1.01 mmol) and triethylamine (196 mg, 1.94 mmol) were added to a solution of **compound 2-1** (300 mg, 0.97 mmol) in 1,4-dioxane (5 mL), and the mixture was stirred at 100 °C under a nitrogen atmosphere for 18 h. The mixture was concentrated under reduced pressure to obtain a crude product, which was then purified by high-performance liquid chromatography (Gilson_306_1741, chromatographic column: Waters-SunFire-C18-10 µm-19 × 250 mm; mobile phase: water (containing 10 mmol/L formic acid) and acetonitrile, gradient ratio: acetonitrile 41%-95%, flow rate: 25 mL/min) to obtain **compound 2.** LC-MS: m/z: 406.0 [M+H]⁺.

¹H NMR (400 MHz, DMSO-*d₆*) *δ* 11.07 (s, 1H), 10.73 (d, *J =* 8.0 Hz, 1H), 8.12 (d, *J =* 8.4 Hz, 1H), 7.59 (d, *J =* 1.6 Hz, 1H), 7.24 (dd, *J =* 8.4, 1.6 Hz, 1H), 5.11 - 4.95 (m, 1H), 2.87 - 2.75 (m, 1H), 2.69 - 2.57 (m, 4H), 2.55 (s, 3H), 2.34 - 2.14 (m, 2H).

### Example 3

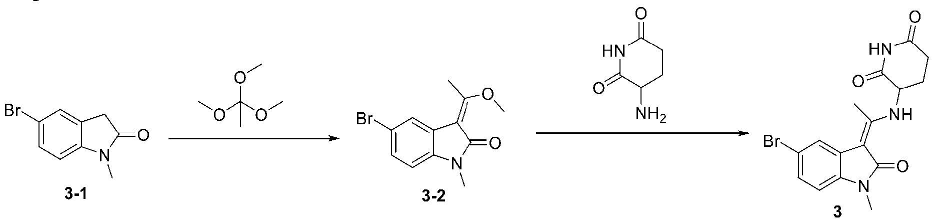

### Step 1: Preparation of compound 3-2

At room temperature, **compound 3-1** (500 mg, 2.21 mmol) and acetic anhydride (5 mL) were added to a single-neck flask, and trimethyl orthoacetate (565 µL, 4.42 mmol) was added under stirring. The reaction system was purged three times with nitrogen and allowed to react at 90 °C for 18 h. The reaction solution was concentrated to obtain a crude product. The crude product was purified by silica gel column chromatography (eluent: methanol/dichloromethane = 0%-5%) and concentrated under vacuum to obtain **compound 3-2.** LC-MS: m/z: 282.0 [M+H]⁺.

### Step 2: Preparation of compound 3

At room temperature, **compound 3-2** (55 mg, 0.19 mmol) and 3-amino-2,6-piperidinedione hydrochloride (35.29 mg, 0.21 mmol) were added to a single-neck flask, then dioxane (1.5 mL) was added, and triethylamine (81.07 µL, 0.58 mmol) was added under stirring. The mixture was allowed to react at 100 °C for 3 h. The reaction solution was filtered, and the filter cake was washed with dioxane (20 mL) to obtain the filter cake as a crude product. The crude product was slurried with acetonitrile (10 mL) to obtain **compound 3.** LC-MS: m/z: 378.0 [M+H]⁺.

¹H NMR (400 MHz, DMSO-*d₆*) *δ* 11.02 (s, 1H), 10.65 (d, *J =* 8.3 Hz, 1H), 7.50 (d, *J =* 1.5 Hz, 1H), 7.18 (dd, *J* = 8.2, 1.6 Hz, 1H), 6.97 (d, *J =* 8.3 Hz, 1H), 4.92 (dd, *J =* 17.8, 8.2 Hz, 1H), 3.24 (s, 3H), 2.87 - 2.72 (m, 1H), 2.60 (d, *J =* 17.5 Hz, 1H), 2.47 (s, 3H), 2.24 - 2.08 (m, 2H).

### Example 4

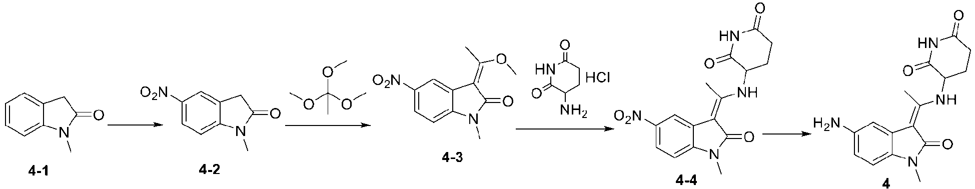

### Step 1: Preparation of compound 4-2

At 0 °C, under a nitrogen atmosphere, 70% nitric acid (1 mL) was slowly added to a solution of **compound 4-1** (2.00 g, 13.6 mmol) in concentrated sulfuric acid (8 mL). The mixture was stirred at 0 °C under the nitrogen atmosphere for 1 h. The mixture was then poured into water (200 mL) and filtered, and the filter cake was collected and dried under vacuum. The resulting crude product was purified by silica gel column chromatography (eluent: petroleum ether/dichloromethane = 1/1 to 1/500) to obtain **compound 4-2.** LC-MS: m/z :193.1 [M+H]⁺.

### Step 2: Preparation of compound 4-3

1,1,1-Trimethoxyethane (1.40 mL, 10.9 mmol) was added to a solution of **compound 4-2** (1.00 g, 5.20 mmol) in acetic anhydride (8 mL), and the mixture was stirred at 90 °C under a nitrogen atmosphere for 18 h. The mixture was then distilled under reduced pressure, and the crude product was purified by silica gel column chromatography (eluent: dichloromethane/methanol = 500/1 to 100/1) to obtain **compound 4-3.** LC-MS: m/z: 249.1 [M+H]⁺.

### Step 3: Preparation of compound 4-4

3-Aminohexahydropyridine-2,6-dione hydrochloride (219 mg, 1.33 mmol) and triethylamine (245 mg, 2.42 mmol) were added to a solution of **compound 4-3** (300 mg, 1.21 mmol) in 1,4-dioxane (8 mL), and the mixture was stirred at 100 °C for 4 h. The mixture was then poured into water (30 mL) and filtered, and the filter cake was washed with water (20 mL), collected, and dried under vacuum to obtain **compound 4-4.** LC-MS: m/z: 345.1 [M+H]⁺.

### Step 4: Preparation of compound 4

Stannous chloride (276 mg, 1.46 mmol) and water (0.1 mL) were added to a solution of **compound 4-4** (100 mg, 0.29 mmol) in dimethyl sulfoxide (3 mL), and the mixture was stirred at 65 °C for 6 h. After the reaction was completed, the mixture was filtered through celite, and the filtrate was concentrated. The crude product obtained after concentration was purified by high-performance liquid chromatography (Gilson_306_1741, chromatographic column: Waters-Xbridge-C18-10 µm-19 × 250 mm; mobile phase: water (containing 10 mmol/L ammonium bicarbonate) and acetonitrile, gradient ratio: acetonitrile 5%-95%, flow rate: 25 mL/min) to obtain **compound 4.** LC-MS: m/z: 315.1 [M+H]⁺.

¹H NMR (400 MHz, DMSO-*d₆*) *δ* 10.97 (s, 1H), 10.48 (d, *J* = 8.4 Hz, 1H), 6.77 (d, *J* = 1.6 Hz, 1H), 6.66 (d, *J* = 8.0 Hz, 1H), 6.32 (d, *J* = 8.0 Hz, 1H), 4.88 - 4.76 (m, 1H), 4.65 (s, 2H), 3.14 (s, 3H), 2.86 - 2.70 (m, 1H), 2.66 - 2.54 (m, 1H), 2.39 (s, 3H), 2.24 - 2.04 (m, 2H).

### Example 5

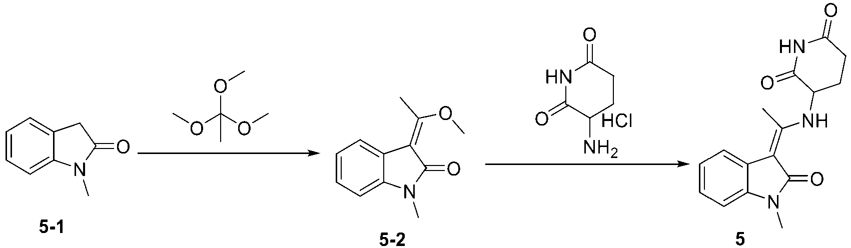

### Step 1: Preparation of compound 5-2

At room temperature, **compound 5-1** (2.00 g, 13.6 mmol) and acetic anhydride (20 mL) were added to a single-neck flask, and trimethyl orthoacetate (3.47 mL, 27.2 mmol) was added under stirring. The reaction system was purged three times with nitrogen and allowed to react at 90 °C for 18 h. The reaction solution was concentrated, and the resulting mixture was purified by silica gel column chromatography (eluent: methanol/dichloromethane = 0%-10%) and concentrated under vacuum to obtain **compound 5-2.** LC-MS: m/z: 204.2 [M+H]⁺.

### Step 2: Preparation of compound 5

At room temperature, **compound 5-2** (100 mg, 0.493 mmol) and 3-amino-2,6-piperidinedione hydrochloride (89.08 mg, 0.539 mmol) were added to a single-neck flask, then dioxane (1.5 mL) was added, and triethylamine (205 µL, 1.48 mmol) was added under stirring. The reaction system was purged three times with nitrogen and allowed to react at 90 °C for 18 h. The reaction solution was cooled and filtered, and the filter cake was washed with dioxane (3 mL), collected, and mixed with acetonitrile (10 mL). The resulting mixture was stirred for three hours and filtered, and the filter cake was washed with acetonitrile (5 mL) and dried under vacuum to obtain **compound 5.** LC-MS: m/z: 300.2 [M+H]⁺.

¹H NMR (400 MHz, DMSO-*d₆*) *δ* 11.02 (s, 1H), 10.53 (d, *J =* 8.4 Hz, 1H), 7.42 (d, *J =* 7.6 Hz, 1H), 7.07 - 6.94 (m, 3H), 4.94 - 4.83 (m, 1H), 3.25 (s, 3H), 2.86 - 2.73 (m, 1H), 2.65 - 2.55 (m, 1H), 2.47 (s, 3H), 2.24 - 2.09 (m, 2H).

### Example 6

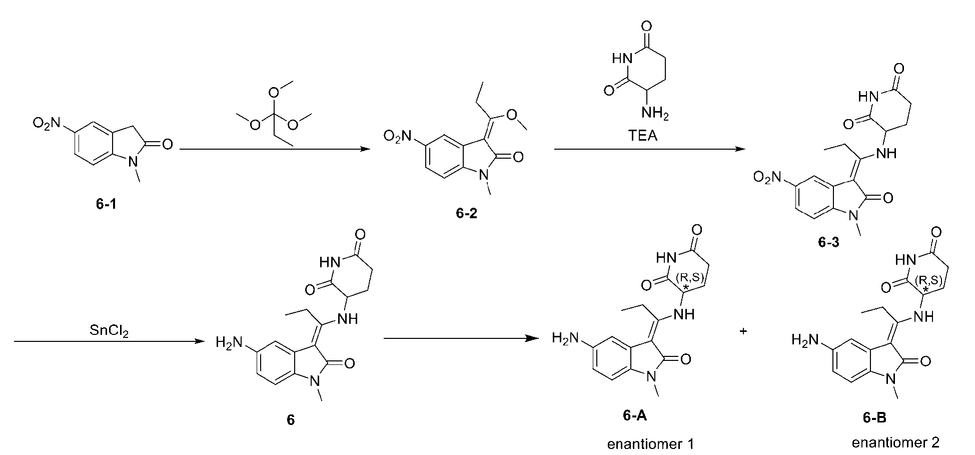

### Step 1: Preparation of compound 6-2

At room temperature, **compound 6-1** (690 mg, 3.59 mmol) and acetic anhydride (7 mL) were added to a single-neck flask, and trimethyl orthopropionate (1.03 mL, 7.18 mmol) was added under stirring. The reaction system was purged three times with nitrogen and allowed to react at 90 °C for 18 h. Water (10 mL) was added to the reaction solution, and the resulting mixture was filtered. The filter cake was washed with water (20 mL), collected, and dried under vacuum to obtain **compound 6-2.** LC-MS: m/z: 263.0 [M+H]⁺.

### Step 2: Preparation of compound 6-3

At room temperature, **compound 6-2** (300 mg, 1.14 mmol) and 3-amino-2,6-piperidinedione hydrochloride (207 mg, 1.26 mmol) were added to a single-neck flask, then dioxane (4.5 mL) was added, and triethylamine (476 µL, 3.43 mmol) was added under stirring. The reaction system was purged three times with nitrogen and allowed to react at 90 °C for 18 h. The reaction solution was filtered, and the filter cake was washed with dioxane (3 mL), collected, and mixed with water (20 mL). The resulting mixture was stirred for 3 h and filtered, and the filter cake was washed with water (5 mL), collected, and dried under vacuum to obtain **compound 6-3.** LC-MS: m/z: 359.2 [M+H]⁺.

### Step 3: Preparation of compound 6

At room temperature, **compound 6-3** (150 mg, 0.42 mmol) and stannous chloride (397 mg, 2.09 mmol) were added to a single-neck flask, and then DMSO (3 mL) and water (0.1 mL) were added. The reaction system was purged three times with nitrogen and allowed to react at 60 °C for 18 h. Saturated ammonium bicarbonate (20 mL) was added to the reaction solution, and the mixture was extracted with dichloromethane (30 mL × 3). The dichloromethane layers were collected, washed with saturated brine (10 mL), dried over anhydrous sodium sulfate, filtered, and concentrated. The crude product obtained after concentration was purified by high-performance liquid chromatography (Gilson_306_1741, chromatographic column: Waters-Xbridge-C18-10 µm-19 × 250 mm; mobile phase: water (containing 10 mmol/L ammonium bicarbonate) and acetonitrile, gradient ratio: acetonitrile 6%, flow rate: 25 mL/min) to obtain **compound 6.** LC-MS: m/z: 329.1 [M+H]⁺.

¹H NMR (400 MHz, DMSO-*d₆*) *δ* 10.97 (s, 1H), 10.46 (d, *J =* 9.0 Hz, 1H), 6.70 (d, *J =* 1.6 Hz, 1H), 6.67 (d, *J* = 8.2 Hz, 1H), 6.33 (dd, *J* = 8.0, 1.6 Hz, 1H), 4.86 - 4.73 (m, 1H), 4.56 (s, 2H), 3.14 (s, 3H), 2.91 - 2.65 (m, 3H), 2.58 - 2.53 (m, 1H), 2.20 - 2.08 (m, 2H), 2.14 - 2.07 (m, 1H), 1.22 (t, *J =* 7.6 Hz, 3H).

Compound 6 was separated to obtain compound 6-A (enantiomer 1) and compound 6-B (enantiomer 2).

Compound 6-A (enantiomer 1), retention time: 2.985 min (instrument: WATERS 150 preparative SFC (SFC-26); column: ChiralCel OJ, 250 × 30 mm I.D., 10 µm; mobile phase: A for CO₂ and B for ethanol (0.1% NH₃H₂O); gradient: B 45%; flow rate: 150 mL/min; back pressure: 100 bar).

Compound 6-B (enantiomer 2), retention time: 3.925 min (instrument: WATERS 150 preparative SFC (SFC-26); column: ChiralCel OJ, 250 × 30 mm I.D., 10 µm; mobile phase: A for CO₂ and B for ethanol (0.1% NH₃H₂O); gradient: B 45%; flow rate: 150 mL/min; back pressure: 100 bar).

Other compounds of the present disclosure can be prepared by methods analogous to those described in the examples above (with appropriate modification, if necessary).

### Biological Section:

### Biological Evaluation of CRBN Binding Assay (HTRF Method)

### I. Assay objective

In this experiment, the binding interaction between compounds and CRBN was detected, and based on the IC₅₀ values, the affinity of the compounds for the CRBN target was evaluated.

### II. Experimental method

The affinity of the compounds for CRBN was detected using CEREBLON BINDING KITS (CISBIO, 64 BDCRBNPEG) containing diluent #9, Human Cereblon WT GST-tagged protein, GST Eu Cryptate Antibody, and Thalidomide-Red.

20 nL of each of the compounds was transferred to a 384-well plate (PerkinElmer, 6007299) using an Ehco650 apparatus (Beckman, 650). 5 µL of 1× diluent #9 (CISBIO, 64BDCRBNPEG) was added to the 384-well plate, and then 5 µL of 1× Human Cereblon WT GST-tagged protein (CISBIO, 64BDCRBNPEG) was added. The plate was sealed with a plate sealer, shaken on a plate shaker (Kylin-Bell, MH-2) for 10 s, centrifuged at 1000 rpm for 1 min, and incubated at 25 °C for 5 min. 5 µL of 1× GST Eu Cryptate Antibody (CISBIO, 64BDCRBNPEG) and 5 µL of 1× Thalidomide-Red (CISBIO, 64BDCRBNPEG) were added to the 384-well plate. The plate was sealed with a plate sealer, shaken for 30 s, centrifuged at 1000 rpm for 1 min, and incubated at 25 °C for 1 h. The signals at 665 nm and 620 nm were measured using Envision (PerkinElmer, 2105-0020).

### III. Data analysis

The IC50 values for compound activity were calculated using Graphpad Prism9 software based on the compound concentrations and the ratio of the signals at 665 nm and 620 nm. Calculation formula: %Inhibition = (Signal compound [Ratio (665 nm/620 nm) × 10000] - SignalAve_PC)/(SignalAve_NC - SignalAve_PC) × 100.

The assay shows that the compounds of the present disclosure exhibited good affinity for the CRBN target. The activity assay results of some of the compounds of the present disclosure measured by the above method are shown in Table 1.

**Table 1**

| Compound No. | IC50 value (µM) | Compound No. | IC50 value (µM) |
|---|---|---|---|
| Compound 1 | A | Compound 4 | B |
| Compound 2 | C | Compound 5 | C |
| Compound 3 | C | Compound 6 | A |
| Compound 6-A | A | Compound 6-B | A |

In Table 1, A represents IC50 ≤ 2 µM, B represents 2 µM < IC50 ≤ 5 µM, C represents 5 µM < IC50 ≤ 10 µM, and D represents IC50 > 10 µM.

The embodiments of the present disclosure are not limited to the specific examples described above, and any technical modifications made according to the embodiments of the present disclosure shall fall within the protection scope of the present disclosure.

## Claims

1. A compound having a structure represented by formula I, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 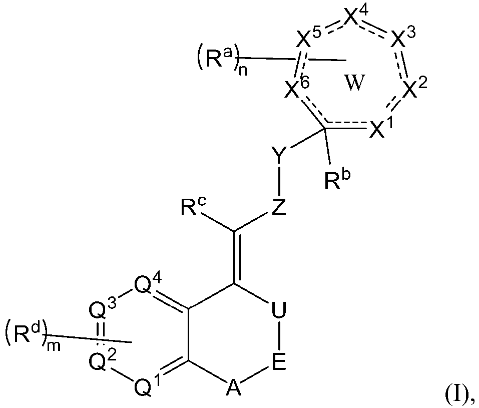 wherein
X¹, X², X³, X⁴, X⁵, and X⁶ are each independently a bond, carbon, nitrogen, oxygen, or sulfur; ring W is a 5-, 6-, or 7-membered ring;
the - - - - - - bonds connected to X¹, X², X³, X⁴, X⁵, and X⁶ each independently represent a single bond or a double bond; two - - - - - - bonds connected to the same atom are not simultaneously double bonds; when X¹, X², X³, X⁴, X⁵, or X⁶ is oxygen or sulfur, the - - - - - - bonds connected thereto are single bonds;
each R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3-to 6-membered heterocyclylamino; optionally, two R^{a} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, and the 3- to 7-membered monocyclic carbocycle or 3- to 7-membered monocyclic heterocycle formed by the connection of two R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
n is 0, 1, 2, 3, 4, 5, 6, 7, 8, 9, 10, 11, or 12;
R^{b} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, or 3- to 6-membered heterocyclyl; optionally, R^{b} and R^{a} are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, and the 3- to 7-membered monocyclic carbocycle or 3-to 7-membered monocyclic heterocycle formed by the connection of R^{b} and R^{a} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Y is a bond, NH, CH₂, CH₂CH₂, NHCH₂, or CH₂NH; optionally, Y is substituted with 1, 2, 3, or 4 R^{Y}; each R^{Y} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, and 5- to 10-membered heteroaryl are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
Z is NH, O, S, or CH₂; optionally, Z is substituted with 1 or 2 R^{Z}; each R^{Z} is independently hydrogen, deuterium, halogen, cyano, hydroxyl, amino, oxo (O=), thio (S=), C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, R^{Z} and R^{a}, or R^{Z} and R^{b}, are connected to form a 3- to 7-membered monocyclic carbocycle or a 3- to 7-membered monocyclic heterocycle; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, 5- to 10-membered heteroaryl, and the 3- to 7-membered monocyclic carbocycle or 3- to 7-membered monocyclic heterocycle formed by the connection of R^{Z} and R^{b} are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R^{c} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; wherein the C₁₋₆ alkyl, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
U is CH₂, NH, O, or S;
E and A are each independently a bond, CH₂, NH, O, or S, wherein E and A are not simultaneously a bond, NH, or O;
optionally, U, E, and A are each independently substituted with 1 or 2 R^{e}; each R^{e} is independently hydrogen, deuterium, halogen, cyano, hydroxyl, amino, oxo (O=), thio (S=), C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; optionally, two R^{e} attached to the same carbon atom, together with the atom to which they are attached, form a 3- to 7-membered monocyclic carbocycle or a 3- to 7-membered monocyclic heterocycle; the C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
Q¹, Q², Q³, and Q⁴ are each independently CH or N;
each R^{d} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-; optionally, two adjacent R^{d} are connected to form a 3- to 7-membered monocyclic carbocycle or a 3- to 7-membered monocyclic heterocycle; wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, C₆₋₁₀ aryl S(=O)₂NH-, and the 3- to 7-membered monocyclic carbocycle or 3- to 7-membered monocyclic heterocycle formed by the connection of two adjacent R^{d} are each independently optionally substituted with 1, 2, 3, 4, or 5 R^{d1}, each R^{d1} being independently selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, 3- to 6-membered heterocyclyl, and C₁₋₆ alkyl OC(=O)-; optionally, two R^{d1} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle formed by the connection of two R^{d1} is optionally substituted with 1, 2, 3, 4, or 5 substituents selected from hydrogen, deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
m is 0, 1, 2, 3, or 4.

2. The compound according to claim 1, wherein 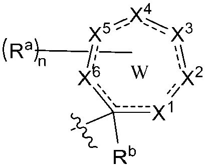 is 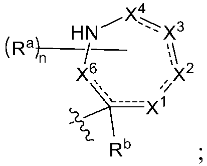 preferably, 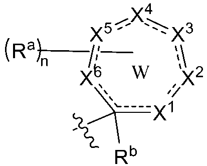 is selected from: 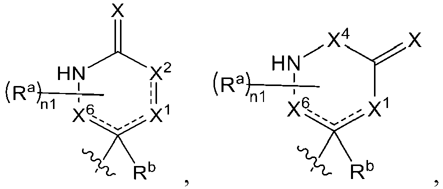 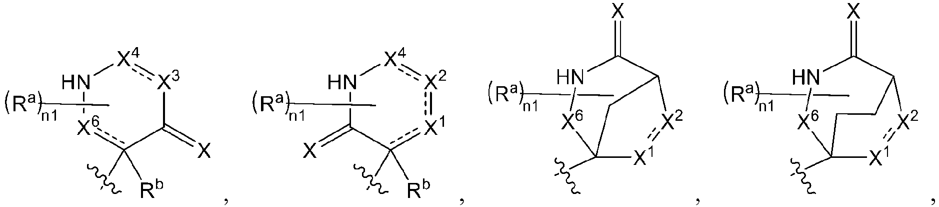 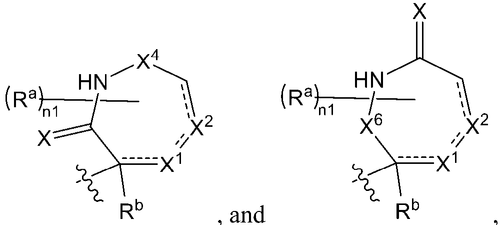 wherein X is O or S; n1 is 0, 1, 2, 3, 4, 5, or 6; preferably, 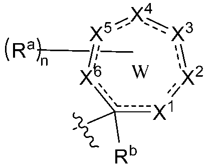 is selected from: 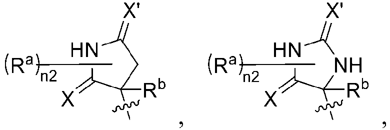 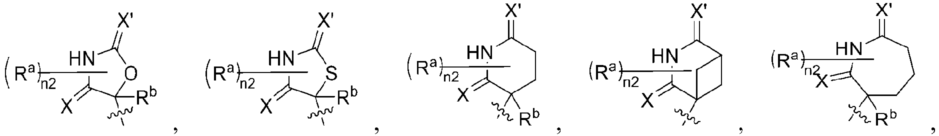 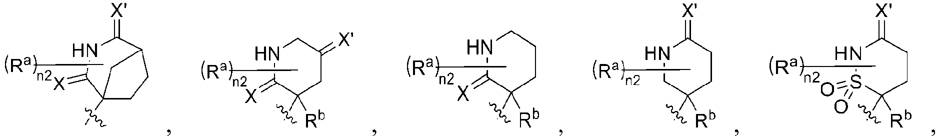 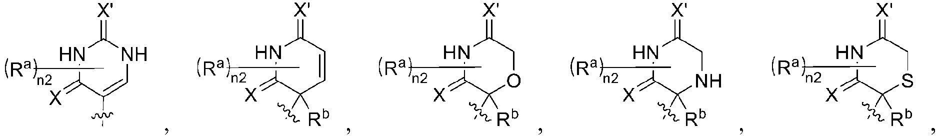 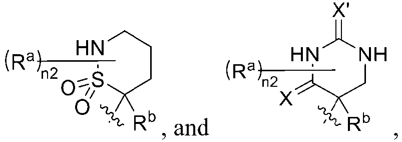 wherein X and X' are each independently O or S; n2 is 0, 1, 2, 3, or 4; preferably, 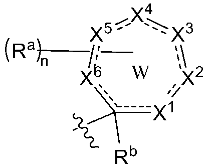 is selected from: 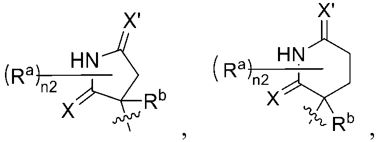 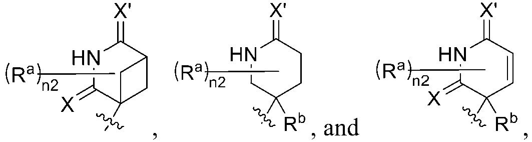 wherein X and X' are each independently O or S; n2 is 0, 1, 2, 3, or 4; preferably, 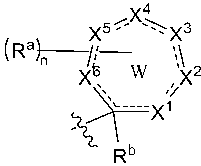 is selected from 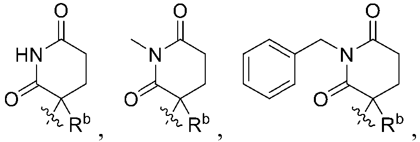 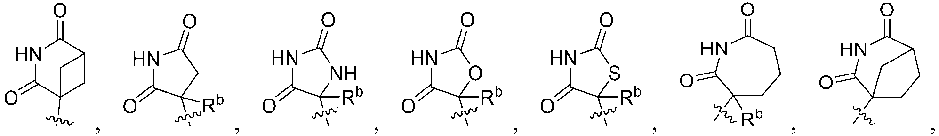 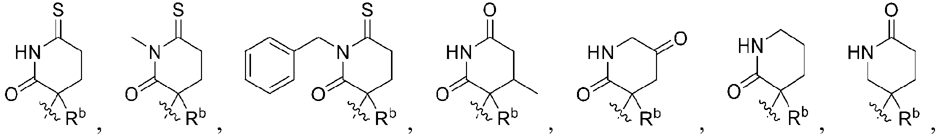 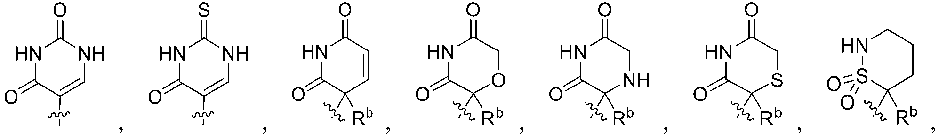 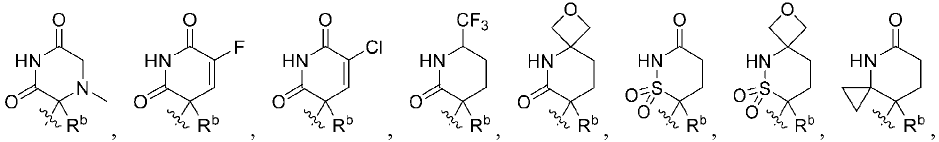 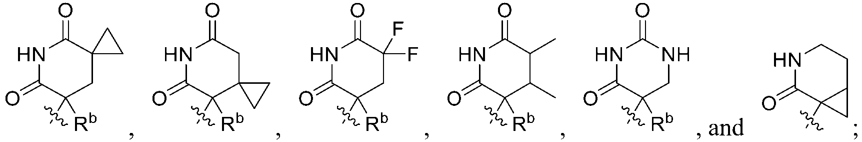 preferably, 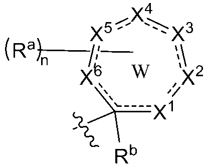 is selected from 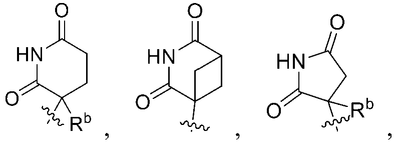 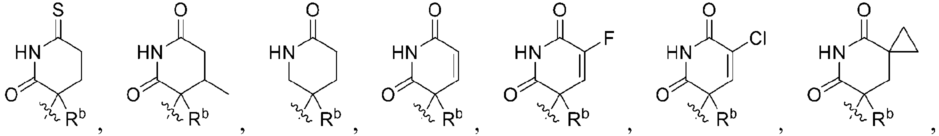 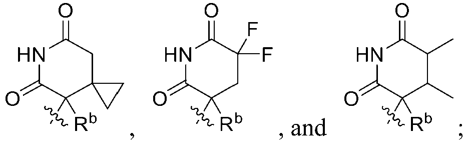 preferably, 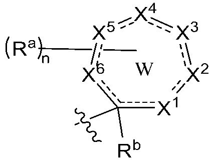 is selected from

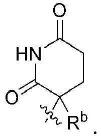

3. The compound according to any one of claims 1-2, wherein 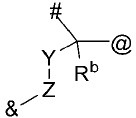 is selected from 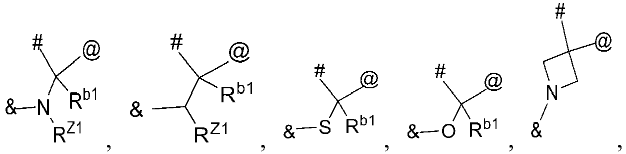 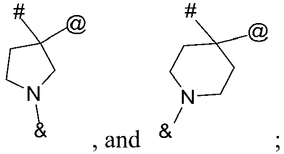 # is connected to X⁶, @ is connected to X¹, and & is connected to the double bond;
each R^{b1} is independently hydrogen, deuterium, halogen, cyano, hydroxyl, amino, or C₁₋₆ alkyl, wherein the C₁₋₆ alkyl is optionally substituted with 1, 2, 3, 4, or 5 substituents selected from deuterium, oxo (O=), halogen, cyano, hydroxyl, amino, C₁₋₄ alkyl, C₁₋₄ alkoxy, C₁₋₄ alkylamino, and C₃₋₆ cycloalkyl;
preferably, each R^{b1} is independently hydrogen, deuterium, F, Cl, Br, cyano, hydroxyl, or C₁₋₄ alkyl, wherein the C₁₋₄ alkyl is optionally substituted with 1, 2, 3, 4, or 5 substituents selected from deuterium, F, Cl, Br, cyano, hydroxyl, amino, C₁₋₄ alkyl, C₁₋₄ alkoxy, and C₁₋₄ alkylamino;
preferably, each R^{b1} is independently hydrogen, deuterium, F, Cl, Br, cyano, hydroxyl, methyl, ethyl, propyl, or isopropyl, wherein the methyl, ethyl, propyl, and isopropyl are each independently optionally substituted with 1, 2, or 3 substituents selected from deuterium, F, Cl, Br, cyano, hydroxyl, amino, methyl, ethyl, propyl, isopropyl, methoxy, ethoxy, isopropoxy, methylamino, and dimethylamino;
more preferably, each R^{b1} is independently hydrogen, deuterium, F, Cl, Br, cyano, or methyl;
most preferably, each R^{b1} is hydrogen;
each R^{Z1} is independently hydrogen, deuterium, halogen, cyano, hydroxyl, amino, or C₁₋₆ alkyl, wherein the C₁₋₆ alkyl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, and C₁₋₄ alkylamino;
preferably, each R^{Z1} is independently hydrogen, deuterium, or C₁₋₄ alkyl, wherein the C₁₋₄ alkyl is optionally substituted with 1, 2, or 3 substituents selected from deuterium, F, Cl, Br, cyano, hydroxyl, amino, C₁₋₄ alkyl, C₁₋₄ alkoxy, and C₁₋₄ alkylamino;
preferably, each R^{Z1} is independently hydrogen, deuterium, methyl, ethyl, propyl, or isopropyl;
preferably, 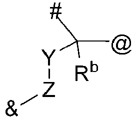
is selected from

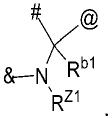

4. The compound according to any one of claims 1-3, wherein R^{c} is hydrogen, deuterium, halogen, cyano, hydroxyl, amino, C₁₋₆ alkyl, C₁₋₆ alkoxy, C₁₋₆ alkylamino, C₃₋₆ cycloalkyl, 3- to 6-membered heterocyclyl, C₆₋₁₀ aryl, or 5- to 10-membered heteroaryl, wherein the C₁₋₆ alkyl, C₁₋₆ alkoxy, C₁₋₆ alkylamino, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, halogen, cyano, hydroxyl, amino, C₁₋₄ alkyl, C₁₋₄ alkoxy, and C₁₋₄ alkylamino;
preferably, R^{c} is halogen, cyano, C₁₋₆ alkyl, C₆₋₁₀ aryl, or 5- to 10-membered heteroaryl, wherein the C₁₋₆ alkyl, C₆₋₁₀ aryl, or 5- to 10-membered heteroaryl is optionally substituted with 1, 2, 3, 4, or 5 substituents selected from deuterium, halogen, cyano, hydroxyl, amino, C₁₋₄ alkyl, C₁₋₄ alkoxy, and C₁₋₄ alkylamino;
preferably, R^{c} is halogen, cyano, C₁₋₆ alkyl, or C₆₋₁₀ aryl, wherein the C₁₋₆ alkyl or C₆₋₁₀ aryl is optionally substituted with 1, 2, 3, 4, or 5 substituents selected from deuterium, halogen, cyano, hydroxyl, amino, and C₁₋₄ alkyl;
preferably, R^{c} is halogen, cyano, C₁₋₄ alkyl, or phenyl, wherein the C₁₋₄ alkyl or phenyl is optionally substituted with 1, 2, 3, 4, or 5 substituents selected from deuterium, halogen, cyano, hydroxyl, amino, and C₁₋₄ alkyl;
preferably, R^{c} is F, Cl, Br, cyano, methyl, ethyl, propyl, isopropyl, difluoromethyl, trifluoromethyl, difluoroethyl, trifluoroethyl, or phenyl.

5. The compound according to any one of claims 1-4, wherein 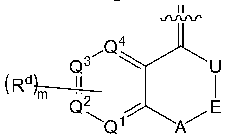 is selected from 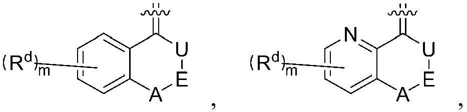 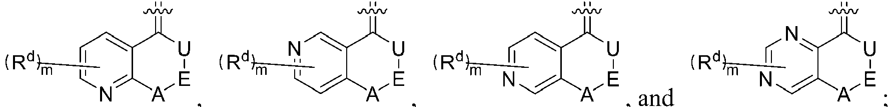 preferably, 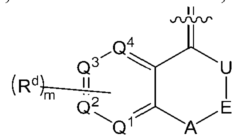 is selected from 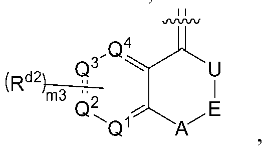 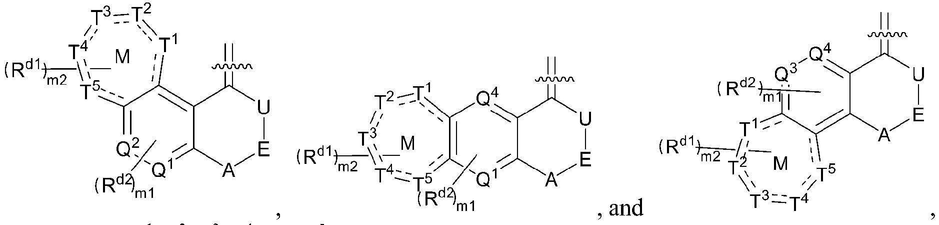 wherein T¹, T², T³, T⁴, and T³ are each independently a bond, carbon, nitrogen, oxygen, or sulfur; ring M is a 3-, 4-, 5-, 6-, or 7-membered ring;
the - - - - - - bonds connected to T¹, T², T³, T⁴, and T⁵ each independently represent a single bond or a double bond; two - - - - - - bonds connected to the same atom are not simultaneously double bonds; when T¹, T², T³, T⁴, and T⁵ are oxygen or sulfur, the - - - - - - bonds connected thereto are single bonds;
each R^{d2} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₆ alkyl, C₂₋₆ alkenyl, C₂₋₆ alkynyl, C₆₋₁₀ aryl, 5-to 10-membered heteroaryl, C₃₋₆ cycloalkyl, 3- to 6-membered heterocyclyl, C₆₋₁₀ aryl C₁₋₆ alkyl, 5-to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, each R^{d2} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, each R^{d2} is independently hydrogen, deuterium, F, Cl, Br, cyano, hydroxyl, sulfhydryl, nitro, amino, carboxyl, methyl, ethyl, propyl, isopropyl, tert-butyl, vinyl, ethynyl, phenyl, pyridinyl, pyrimidinyl, imidazolyl, oxazolyl, thiazolyl, cyclopropyl, cyclopentyl, cyclohexyl, oxetanyl, azetidinyl, pyrrolidinyl, piperidinyl, piperazinyl, 1,2,3,6-tetrahydropyridinyl, benzyl, 8-azabicyclo[3.2.1]octanyl, cyclopropylmethyl, piperidinylmethyl, methoxy, ethoxy, methylamino, dimethylamino, phenoxy, pyridinyloxy, cyclopropyloxy, phenylamino, cyclopropylamino, piperidinylamino, NH₂C(=O)-, methyl NHC(=O)-, ethyl NHC(=O)-, methyl C(=O)NH-, ethyl C(=O)NH-, methyl C(=O)-, ethyl C(=O)-, methyl S(=O)-, ethyl S(=O)-, methyl S(=O)₂-, methyl OC(=O)-, ethyl S(=O)₂-, ethyl OC(=O)-, methyl S(=O)NH-, ethyl S(=O)NH-, methyl S(=O)₂NH-, ethyl S(=O)₂NH-, phenyl C(=O)-, phenyl S(=O)-, phenyl S(=O)₂-, phenyl OC(=O)-, phenyl S(=O)NH-, or phenyl S(=O)₂NH-, wherein the methyl, ethyl, propyl, isopropyl, tert-butyl, vinyl, ethynyl, phenyl, pyridinyl, pyrimidinyl, imidazolyl, oxazolyl, thiazolyl, cyclopropyl, cyclopentyl, cyclohexyl, oxetanyl, azetidinyl, pyrrolidinyl, piperidinyl, piperazinyl, 1,2,3,6-tetrahydropyridinyl, benzyl, 8-azabicyclo[3.2.1]octanyl, cyclopropylmethyl, piperidinylmethyl, methoxy, ethoxy, methylamino, dimethylamino, phenoxy, pyridinyloxy, cyclopropyloxy, phenylamino, cyclopropylamino, piperidinylamino, NH₂C(=O)-, methyl NHC(=O)-, ethyl NHC(=O)-, methyl C(=O)NH-, ethyl C(=O)NH-, methyl C(=O)-, ethyl C(=O)-, methyl S(=O)-, ethyl S(=O)-, methyl S(=O)₂-, methyl OC(=O)-, ethyl S(=O)₂-, ethyl OC(=O)-, methyl S(=O)NH-, ethyl S(=O)NH-, methyl S(=O)₂NH-, ethyl S(=O)₂NH-, phenyl C(=O)-, phenyl S(=O)-, phenyl S(=O)₂-, phenyl OC(=O)-, phenyl S(=O)NH-, or phenyl S(=O)₂NH- is optionally substituted with 1, 2, 3, 4, or 5 substituents selected from deuterium, oxo (O=), thio (S=), F, Cl, Br, cyano, hydroxyl, amino, methyl, ethyl, methoxy, ethoxy, methylamino, and dimethylamino;
m1 is 0, 1, or 2;
m2 is 0, 1, 2, 3, 4, or 5;
m3 is 0, 1, 2, 3, or 4; preferably, m3 is 0, 1, or 2;
preferably, 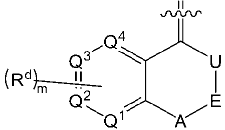 is selected from 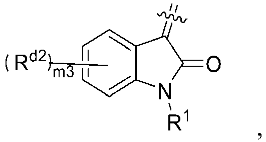 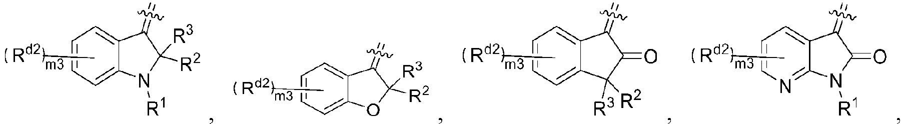 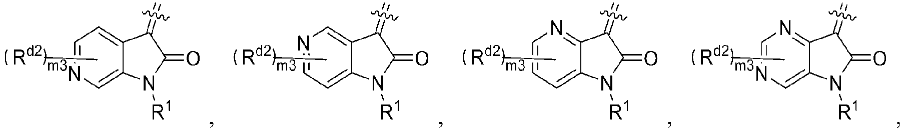 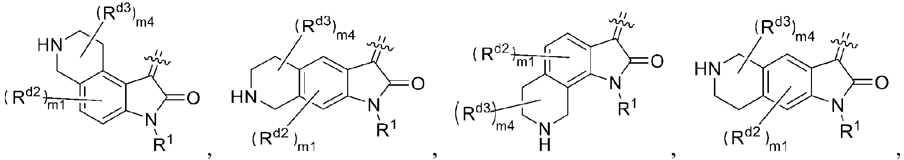 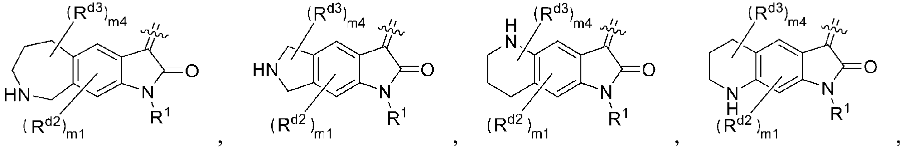 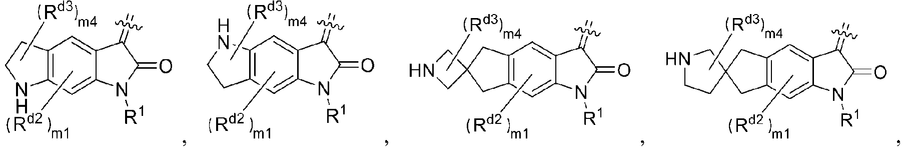 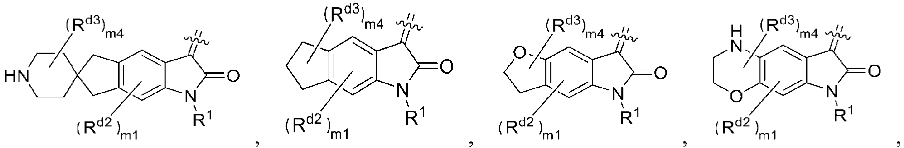 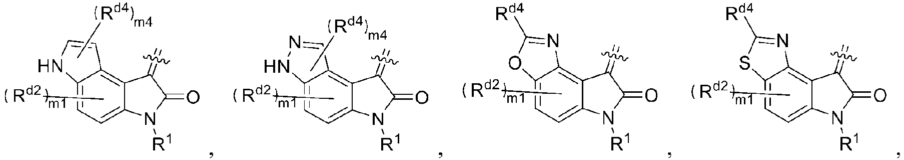 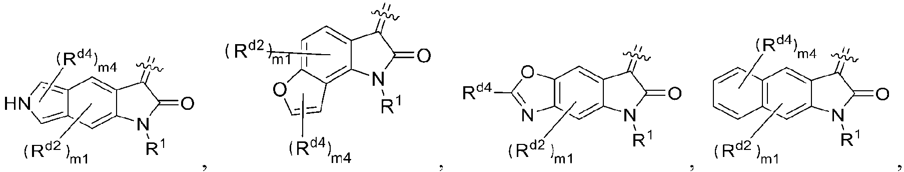 and 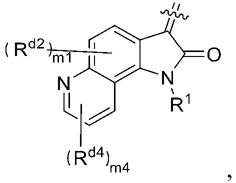
wherein
each R¹ is independently hydrogen, deuterium, C₁₋₆ alkyl, NH₂C(=O)-, C₁₋₆ alkyl C(=O)-, C₁₋₆ alkyl S(=O)-, C₁₋₆ alkyl S(=O)₂-, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl, wherein the C₁₋₆ alkyl, NH₂C(=O)-, C₁₋₆ alkyl C(=O)-, C₁₋₆ alkyl S(=O)-, C₁₋₆ alkyl S(=O)₂-, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, each R¹ is independently hydrogen, deuterium, C₁₋₄ alkyl, NH₂C(=O)-, C₁₋₄ alkyl C(=O)-, C₁₋₄ alkyl S(=O)-, C₁₋₄ alkyl S(=O)₂-, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, NH₂C(=O)-, C₁₋₄ alkyl C(=O)-, C₁₋₄ alkyl S(=O)-, C₁₋₄ alkyl S(=O)₂-, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, each R¹ is independently hydrogen, deuterium, methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, or imidazolyl, wherein the methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, or imidazolyl is optionally substituted with 1, 2, 3, 4, or 5 substituents selected from deuterium, oxo (O=), thio (S=), F, Cl, Br, cyano, hydroxyl, amino, methyl, ethyl, methoxy, ethoxy, methylamino, and dimethylamino;
preferably, each R¹ is independently hydrogen, deuterium, methyl, ethyl, isopropyl, NH₂C(=O)-, methyl C(=O)-, ethyl C(=O)-, methyl S(=O)-, methyl S(=O)₂-, ethyl S(=O)-, ethyl S(=O)₂-, cyclopropyl, cyclobutyl, oxetanyl, azetidinyl, pyrrolyl, or imidazolyl, wherein the methyl, ethyl, isopropyl, NH₂C(=O)-, methyl C(=O)-, ethyl C(=O)-, methyl S(=O)-, methyl S(=O)₂-, ethyl S(=O)-, ethyl S(=O)₂-, cyclopropyl, cyclobutyl, oxetanyl, azetidinyl, pyrrolyl, or imidazolyl is optionally substituted with 1, 2, 3, 4, or 5 substituents selected from deuterium, F, Cl, Br, cyano, hydroxyl, amino, methyl, ethyl, methoxy, ethoxy, methylamino, and dimethylamino;
R² and R³ are each independently hydrogen, deuterium, halogen, cyano, hydroxyl, amino, C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl; or R² and R³ are connected to form oxo (O=) or thio (S=); or R² and R³, together with the C atom to which they are attached, form a 3- to 6-membered monocyclic carbocycle or a 3- to 6-membered monocyclic heterocycle; wherein the C₁₋₆ alkyl, NH₂C(=O)-, C₁₋₆ alkyl C(=O)-, C₁₋₆ alkyl S(=O)-, C₁₋₆ alkyl S(=O)₂-, C₁₋₆ alkoxy, C₁₋₆ alkylamino, C₂₋₆ alkenyl, C₂₋₆ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, R² and R³ are each independently hydrogen, deuterium, F, Cl, Br, cyano, hydroxyl, amino, C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; or R² and R³ are connected to form oxo (O=) or thio (S=); or R² and R³, together with the C atom to which they are attached, form a 3- to 6-membered monocyclic carbocycle or a 3- to 6-membered monocyclic heterocycle; wherein the C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, R² and R³ are each independently hydrogen, deuterium, F, Cl, Br, cyano, hydroxyl, amino, methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, or imidazolyl; or R² and R³ are connected to form oxo (O=); or R² and R³, together with the C atom to which they are attached, form cyclopropyl; wherein the methyl, ethyl, isopropyl, tert-butyl, NH₂C(=O)-, methyl C(=O)-, ethyl C(=O)-, methyl S(=O)-, methyl S(=O)₂-, ethyl S(=O)-, ethyl S(=O)₂-, vinyl, ethynyl, cyclopropyl, cyclobutyl, oxetanyl, azetidinyl, pyrrolyl, and imidazolyl are each independently optionally substituted with 1, 2, 3, 4, or 5 substituents selected from deuterium, oxo (O=), thio (S=), F, Cl, Br, cyano, hydroxyl, amino, methyl, ethyl, methoxy, ethoxy, methylamino, and dimethylamino;
each R^{d3} is independently hydrogen, deuterium, halogen, oxo (O=), thio (S=), cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3-to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
optionally, each R^{d3} is independently hydrogen, deuterium, F, Cl, Br, oxo (O=), thio (S=), cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
optionally, each R^{d3} is independently hydrogen, deuterium, F, Cl, Br, oxo (O=), thio (S=), cyano, hydroxyl, sulfhydryl, nitro, amino, carboxyl, methyl, ethyl, vinyl, ethynyl, NH₂C(=O)-, methyl NHC(=O)-, methyl C(=O)NH-, methyl C(=O)-, methyl S(=O)-, methyl S(=O)₂-, methyl OC(=O)-, methyl S(=O)NH-, methyl S(=O)₂NH-, ethyl NHC(=O)-, ethyl C(=O)NH-, ethyl C(=O)-, ethyl S(=O)-, ethyl S(=O)₂-, ethyl OC(=O)-, ethyl S(=O)NH-, ethyl S(=O)₂NH-, isopropyl NHC(=O)-, isopropyl C(=O)NH-, isopropyl C(=O)-, isopropyl S(=O)-, isopropyl S(=O)₂-, isopropyl OC(=O)-, isopropyl S(=O)NH-, isopropyl S(=O)₂NH-, tert-butyl NHC(=O)-, tert-butyl C(=O)NH-, tert-butyl C(=O)-, tert-butyl S(=O)-, tert-butyl S(=O)₂-, tert-butyl OC(=O)-, tert-butyl S(=O)NH-, or tert-butyl S(=O)₂NH-;
each R^{d4} is independently hydrogen, deuterium, halogen, cyano, hydroxyl, sulfhydryl, nitro, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₆ alkyl NHC(=O)-, C₁₋₆ alkyl C(=O)NH-, C₁₋₆ alkyl C(=O)-, C₁₋₆ alkyl S(=O)-, C₁₋₆ alkyl S(=O)₂-, C₁₋₆ alkyl OC(=O)-, C₁₋₆ alkyl S(=O)NH-, C₁₋₆ alkyl S(=O)₂NH-, C₁₋₆ alkyl NHC(=O)-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
optionally, each R^{d4} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3-to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-;
optionally, each R^{d4} is independently hydrogen, deuterium, F, Cl, Br, cyano, hydroxyl, sulfhydryl, nitro, amino, carboxyl, methyl, ethyl, vinyl, ethynyl, NH₂C(=O)-, methyl NHC(=O)-, methyl C(=O)NH-, methyl C(=O)-, methyl S(=O)-, methyl S(=O)₂-, methyl OC(=O)-, methyl S(=O)NH-, methyl S(=O)₂NH-, ethyl NHC(=O)-, ethyl C(=O)NH-, ethyl C(=O)-, ethyl S(=O)-, ethyl S(=O)₂-, ethyl OC(=O)-, ethyl S(=O)NH-, ethyl S(=O)₂NH-, isopropyl NHC(=O)-, isopropyl C(=O)NH-, isopropyl C(=O)-, isopropyl S(=O)-, isopropyl S(=O)₂-, isopropyl OC(=O)-, isopropyl S(=O)NH-, isopropyl S(=O)₂NH-, tert-butyl NHC(=O)-, tert-butyl C(=O)NH-, tert-butyl C(=O)-, tert-butyl S(=O)-, tert-butyl S(=O)₂-, tert-butyl OC(=O)-, tert-butyl S(=O)NH-, or tert-butyl S(=O)₂NH-;
m4 is 0 1 or 2
preferably, 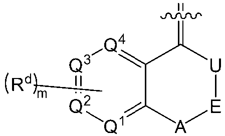 is selected from 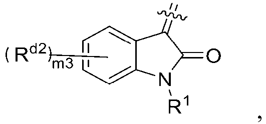 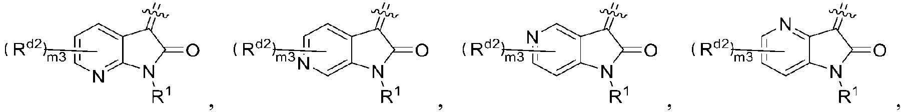 and 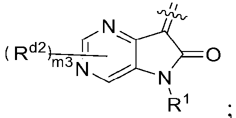
preferably, 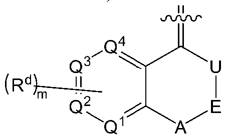
is selected from

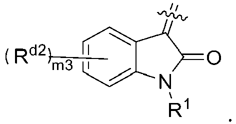

6. The compound according to any one of claims 1-5 having a structure of formula I-1, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof:

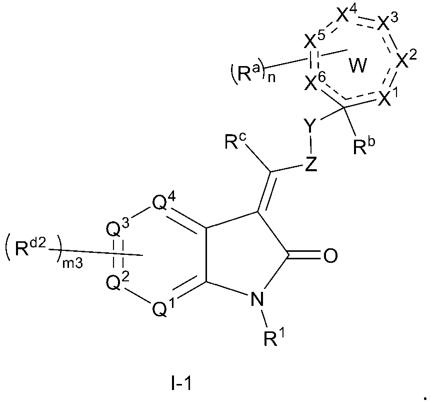

7. The compound according to claim 6 having a structure of formula II-1 or II-2, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof:

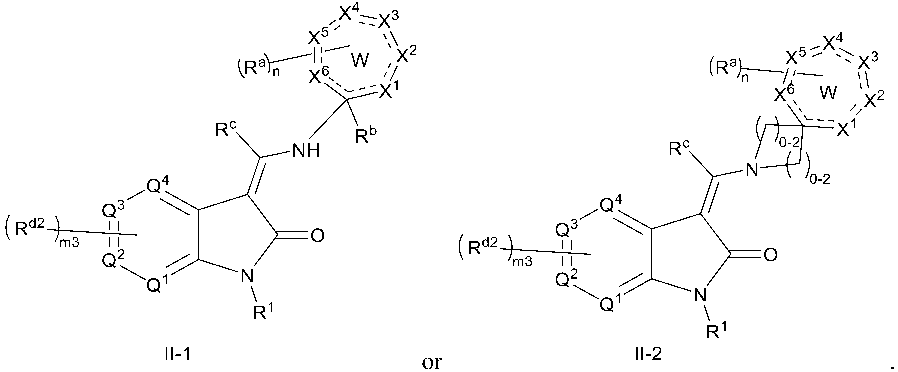

8. The compound according to claim 7 having a structure of formula II-1-1, II-1-2, II-1-3, II-1-4, II-1-5, 11-2-1, II-2-2, II-2-3, or II-2-4, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 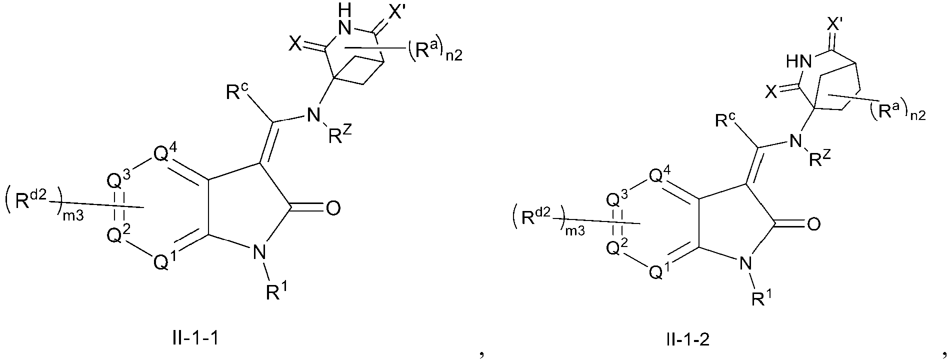 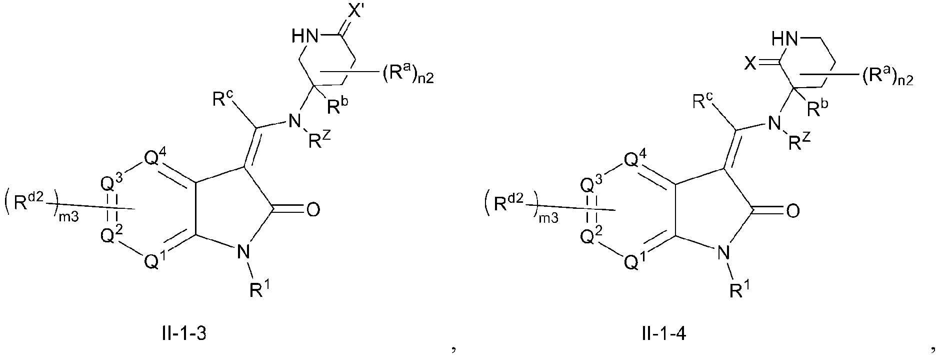 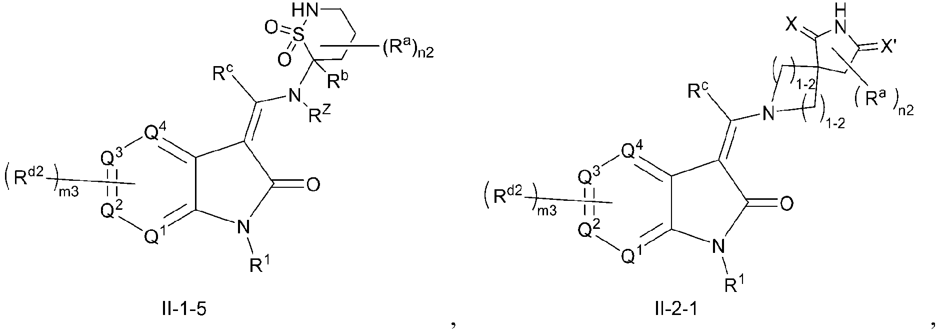 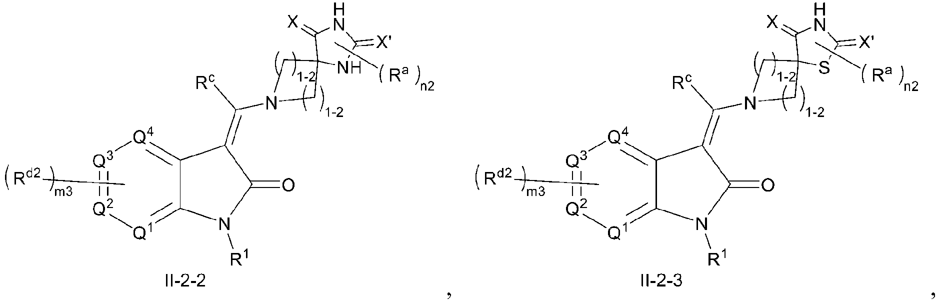 or 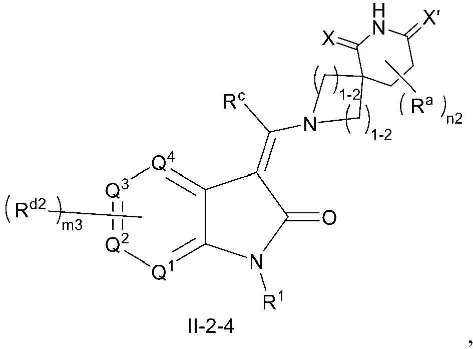 wherein Q¹, Q², Q³, Q⁴, R^{c}, and R^{Z} are as defined in claim 1; X, X', and n₂ are as defined in claim 2; R^{d2}, m3, and R¹ are as defined in claim 5;
in general formulas II-1-1, II-1-2, II-2-1, II-2-2, II-2-3, and II-2-4, each R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
in general formulas II-1-3, II-1-4, and II-1-5, there is at least one occurrence where two R^{a} on the same carbon atom are connected to form a 3- to 7-membered monocyclic carbocycle or a 3- to 7-membered monocyclic heterocycle, or there is at least one occurrence where R^{b} is connected to one R^{a} on an adjacent carbon atom to form a 3- to 7-membered monocyclic carbocycle or a 3- to 7-membered monocyclic heterocycle;
when two R^{a} on the same carbon atom are connected to form a 3- to 7-membered monocyclic carbocycle or a 3- to 7-membered monocyclic heterocycle, each remaining R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
R^{b} is hydrogen, deuterium, halogen, cyano, hydroxyl, amino, C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, or 3- to 6-membered heterocyclyl, wherein the C₁₋₆ alkyl, C₁₋₆ alkoxy, C₁₋₆ alkylamino, C₂₋₆ alkenyl, C₂₋₆ alkynyl, C₆₋₁₀ aryl, 5- to 10-membered heteroaryl, C₃₋₆ cycloalkyl, and 3- to 6-membered heterocyclyl are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
when R^{b} is connected to one R^{a} on an adjacent carbon atom to form a 3- to 7-membered monocyclic carbocycle or a 3- to 7-membered monocyclic heterocycle, each remaining R^{a} is independently hydrogen, deuterium, oxo (O=), thio (S=), halogen, cyano, hydroxyl, amino, carboxyl, C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3- to 6-membered heterocyclylamino, wherein the C₁₋₆ alkyl, C₂₋₆ alkenyl, C₂₋₆ alkynyl, C₆₋₁₀ aryl, 5- to 10-membered heteroaryl, C₃₋₆ cycloalkyl, 3- to 6-membered heterocyclyl, C₆₋₁₀ aryl C₁₋₆ alkyl, 5- to 10-membered heteroaryl C₁₋₆ alkyl, C₃₋₆ cycloalkyl C₁₋₆ alkyl, 3- to 6-membered heterocyclyl C₁₋₆ alkyl, C₁₋₆ alkoxy, C₁₋₆ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, and 3- to 6-membered heterocyclylamino are each independently optionally substituted with 1, 2, 3, 4, or 5 substituents selected from deuterium, oxo (O=), thio (S=), halogen, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl.

9. The compound according to any one of claims 1-7 having a structure of formula III, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 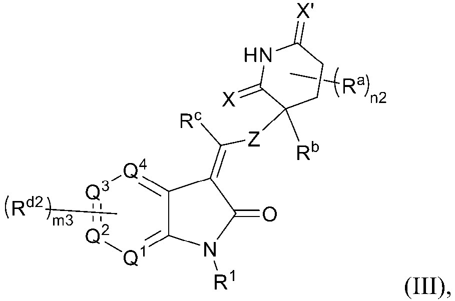 wherein Q¹, Q², Q³, Q⁴, R^{a}, R^{b}, R^{c}, and Z are as defined in claim 1; X, X', and n2 are as defined in claim 2; R^{d2}, m3, and R¹ are as defined in claim 5.

10. The compound according to claim 9 having a structure of formula III-1, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 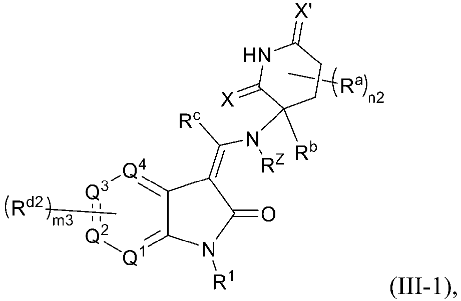 wherein Q¹, Q², Q³, Q⁴, R^{a}, R^{b}, R^{c}, and R^{Z} are as defined in claim 1; X, X', and n2 are as defined in claim 2; R^{d2}, m3, and R¹ are as defined in claim 5.

11. The compound according to claim 1 having a structure represented by formula IV-1, IV-2, IV-3, IV-4, IV-5, or IV-6, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof: 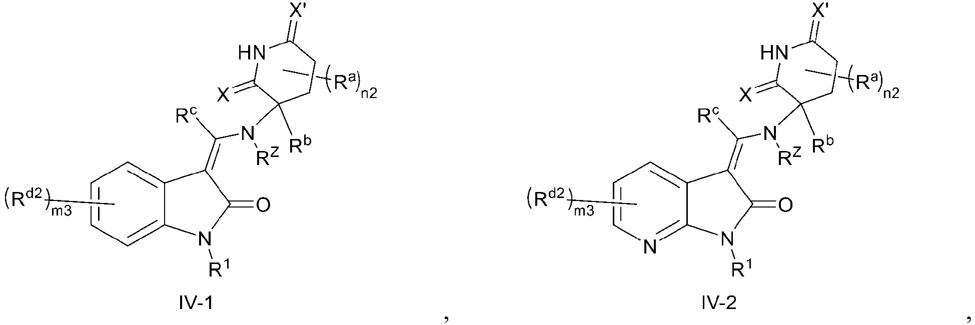 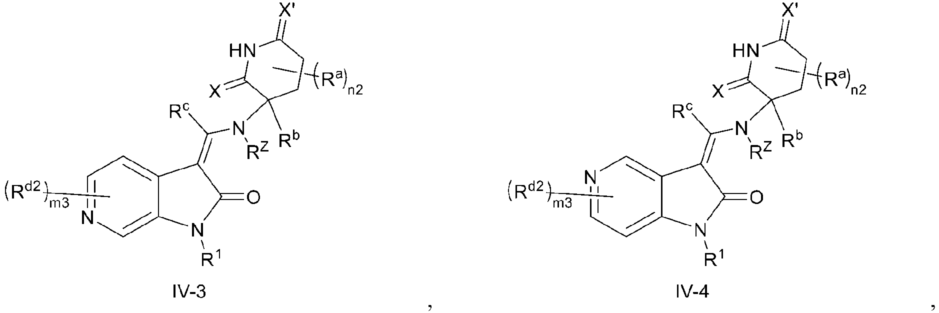 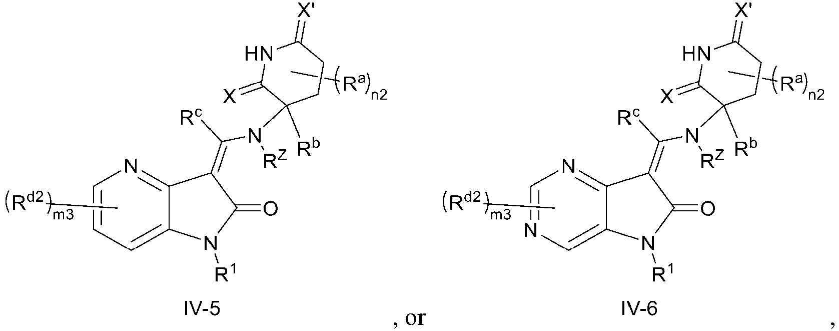 wherein R^{a}, R^{b}, R^{c}, and R^{Z} are as defined in claim 1; X, X', and n2 are as defined in claim 2; R^{d2}, m3, and R¹ are as defined in claim 5.

12. The compound according to any one of claims 1-11, wherein
each R^{a} is independently hydrogen, deuterium, oxo (O=), thio (S=), F, Cl, Br, cyano, hydroxyl, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, or 3-to 6-membered heterocyclylamino; optionally, two R^{a} are connected to form a 3- to 7-membered monocyclic carbocycle or a 3- to 7-membered monocyclic heterocycle; wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, and the 3- to 7-membered monocyclic carbocycle or 3- to 7-membered monocyclic heterocycle formed by the connection of two R^{a} are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl;
preferably, each R^{a} is independently hydrogen, deuterium, F, Cl, Br, -NH₂, -NH(C₁₋₆ alkyl), or -N(C₁₋₆ alkyl)(C₁₋₆ alkyl), or two R^{a} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle is optionally substituted with 1-3 substituents selected from deuterium, F, Cl, Br, and C₁₋₄ alkyl;
n is 0, 1, 2, 3, 4, 5, or 6; n2 is 0, 1, 2, 3, or 4;
preferably, n is 0, 1, 2, or 3; n2 is 0, 1, or 2;
X and X' are each independently O or S;
preferably, X and X' are each independently O;
R^{b} is hydrogen, deuterium, F, Cl, Br, cyano, hydroxyl, amino, C₁₋₄ alkyl, C₁₋₄ alkoxy, C₁₋₄ alkylamino, C₂₋₄ alkenyl, C₂₋₄ alkynyl, C₆₋₁₀ aryl, 5- to 10-membered heteroaryl, C₃₋₆ cycloalkyl, or 3- to 6-membered heterocyclyl; optionally, R^{b} and R^{a} are connected to form a 3- to 7-membered monocyclic carbocycle or a 3- to 7-membered monocyclic heterocycle; wherein the C₁₋₄ alkyl, C₁₋₄ alkoxy, C₁₋₄ alkylamino, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, and the 3- to 7-membered monocyclic carbocycle or 3-to 7-membered monocyclic heterocycle formed by the connection of R^{b} and R^{a} are each independently optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, R^{b} is H, deuterium, F, Cl, Br, or C₁₋₄ alkyl; optionally, R^{b} and R^{a} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle is optionally substituted with 1, 2, or 3 substituents selected from D and C₁₋₄ alkyl;
Y is a bond, NH, CH₂, CH₂CH₂, NHCH₂, or CH₂NH; optionally, Y is substituted with 1, 2, 3, or 4 R^{Y}; each R^{Y} is independently deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, and 5- to 10-membered heteroaryl are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3-to 6-membered heterocyclyl; preferably, Y is a bond;
Z is NH, O, S, or CH₂; optionally, Z is substituted with 1 or 2 R^{Z}; each R^{Z} is independently hydrogen, deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; optionally, R^{Z} and R^{a}, or R^{Z} and R^{b}, are connected to form a 3- to 7-membered monocyclic carbocycle or a 3- to 7-membered monocyclic heterocycle; wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, 5- to 10-membered heteroaryl, and the 3- to 7-membered monocyclic carbocycle or 3- to 7-membered monocyclic heterocycle formed by the connection of R^{Z} and R^{a} or R^{Z} and R^{b} are each independently optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl; preferably, Z is NH, O, S, or CH₂; optionally, Z is substituted with 1 or 2 R^{Z}; each R^{Z} is independently hydrogen, deuterium, F, Cl, Br, cyano, hydroxyl, amino, C₁₋₄ alkyl, C₁₋₄ alkoxy, C₁₋₄ alkylamino, or C₃₋₆ cycloalkyl; preferably, R^{Z} is independently hydrogen or C₁₋₄ alkyl;
R^{c} is hydrogen, deuterium, F, Cl, Br, cyano, hydroxyl, amino, C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl, wherein the C₁₋₄ alkyl, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl is optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl; preferably, R^{c} is hydrogen, deuterium, F, Cl, Br, cyano, C₁₋₄ alkyl, C₂₋₄ alkenyl, C₂₋₄ alkynyl, C₃₋₆ cycloalkyl, or 3- to 6-membered heterocyclyl;
each R^{e} is independently hydrogen, deuterium, F, Cl, Br, cyano, hydroxyl, amino, oxo (O=), thio (S=), C₁₋₄ alkyl, NH₂C(=O)-, C₁₋₄ alkyl C(=O)-, C₁₋₄ alkyl S(=O)-, C₁₋₄ alkyl S(=O)₂-, C₁₋₄ alkoxy, C₁₋₄ alkylamino, C₂₋₄ alkenyl, C₂₋₄ alkynyl, C₃₋₆ cycloalkyl, 3- to 6-membered heterocyclyl, C₆₋₁₀ aryl, or 5- to 10-membered heteroaryl; optionally, two R^{e} attached to the same carbon atom, together with the atom to which they are attached, form a 3- to 7-membered monocyclic carbocycle; preferably, each R^{e} is independently hydrogen, deuterium, F, Cl, Br, cyano, hydroxyl, amino, oxo (O=), thio (S=), C₁₋₄ alkyl, C₁₋₄ alkoxy, C₁₋₄ alkylamino, C₂₋₄ alkenyl, C₂₋₄ alkynyl, or C₃₋₆ cycloalkyl; optionally, two R^{e} attached to the same carbon atom, together with the atom to which they are attached, form a 3- to 7-membered monocyclic carbocycle;
each R^{d} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-; optionally, two adjacent R^{d} are connected to form a 3- to 7-membered monocyclic carbocycle or a 3- to 7-membered monocyclic heterocycle; wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, C₆₋₁₀ aryl C₁₋₄ alkyl, 5- to 10-membered heteroaryl C₁₋₄ alkyl, C₃₋₆ cycloalkyl C₁₋₄ alkyl, 3- to 6-membered heterocyclyl C₁₋₄ alkyl, C₁₋₄ alkoxy, C₁₋₄ alkylamino, C₆₋₁₀ aryloxy, 5- to 10-membered heteroaryloxy, C₃₋₆ cycloalkyloxy, 3- to 6-membered heterocyclyloxy, C₆₋₁₀ arylamino, 5- to 10-membered heteroarylamino, C₃₋₆ cycloalkylamino, 3- to 6-membered heterocyclylamino, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, C₆₋₁₀ aryl S(=O)₂NH-, and the 3- to 7-membered monocyclic carbocycle or 3- to 7-membered monocyclic heterocycle formed by the connection of two R^{d} are each independently optionally substituted with 1, 2, 3, 4, or 5 R^{d1}, each R^{d1} being independently selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, 3- to 6-membered heterocyclyl, and C₁₋₄ alkyl OC(=O)-; optionally, two R^{d1} are connected to form a 3- to 7-membered monocyclic carbocycle or a 3- to 7-membered monocyclic heterocycle, wherein the 3- to 7-membered monocyclic carbocycle or 3- to 7-membered monocyclic heterocycle formed by the connection of two R^{d1} is optionally substituted with 1, 2, 3, 4, or 5 substituents selected from hydrogen, deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, each R^{d} is independently hydrogen, deuterium, F, Cl, Br, cyano, hydroxyl, sulfhydryl, nitro, amino, carboxyl, C₁₋₄ alkyl, C₂₋₄ alkynyl, 5- to 10-membered heteroaryl, 3- to 6-membered heterocyclyl, C₁₋₆ alkoxy, C₁₋₄ alkylamino, C₃₋₆ cycloalkyloxy, 3- to 6-membered heterocyclyloxy, C₃₋₆ cycloalkylamino, 3- to 6-membered heterocyclylamino, NH₂C(=O)-, C₁₋₄ alkyl NHC(=O)-, C_{1- 4} alkyl C(=O)NH-, C₁₋₄ alkyl C(=O)-, C₁₋₄ alkyl S(=O)₂-, C₁₋₄ alkyl OC(=O)-, or C₁₋₄ alkyl S(=O)₂NH-, wherein each R^{d} is independently optionally substituted with 1, 2, or 3 R^{d1}, each R^{d1} being independently selected from hydrogen, deuterium, F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
more preferably, each R^{d} is independently hydrogen, deuterium, F, Cl, Br, cyano, hydroxyl, amino, or C₁₋₄ alkyl;
each R^{d2} is independently hydrogen, deuterium, F, Cl, Br, cyano, hydroxyl, sulfhydryl, nitro, amino, carboxyl, C₁₋₄ alkyl, C₂₋₄ alkenyl, C₂₋₄ alkynyl, C₆₋₁₀ aryl, 5- to 10-membered heteroaryl, C_{3- 6} cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH-, wherein the C₁₋₄ alkyl, C₂₋₄ alkenyl, C₂₋₄ alkynyl, C₆₋₁₀ aryl, 5- to 10-membered heteroaryl, C₃₋₆ cycloalkyl, 3- to 6-membered heterocyclyl, NH₂C(=O)-, C₁₋₄ alkyl NHC(=O)-, C₁₋₄ alkyl C(=O)NH-, C₁₋₄ alkyl C(=O)-, C₁₋₄ alkyl S(=O)-, C₁₋₄ alkyl S(=O)₂-, C₁₋₄ alkyl OC(=O)-, C₁₋₄ alkyl S(=O)NH-, C₁₋₄ alkyl S(=O)₂NH-, C₆₋₁₀ aryl C(=O)-, C₆₋₁₀ aryl S(=O)-, C₆₋₁₀ aryl S(=O)₂-, C₆₋₁₀ aryl OC(=O)-, C₆₋₁₀ aryl S(=O)NH-, or C₆₋₁₀ aryl S(=O)₂NH- is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, C₁₋₄ alkylamino, C₆₋₁₀ aryl, 5- to 10-membered heteroaryl, C₃₋₆ cycloalkyl, and 3- to 6-membered heterocyclyl;
preferably, R^{d2} is independently hydrogen, deuterium, F, Cl, Br, cyano, hydroxyl, sulfhydryl, nitro, amino, carboxyl, C₁₋₄ alkyl, C₆₋₁₀ aryl, 5- to 10-membered heteroaryl, C₃₋₆ cycloalkyl, or 3- to 6-membered heterocyclyl, wherein the C₁₋₄ alkyl, C₆₋₁₀ aryl, 5- to 10-membered heteroaryl, C₃₋₆ cycloalkyl, or 3- to 6-membered heterocyclyl is optionally substituted with 1, 2, 3, 4, or 5 substituents selected from deuterium, oxo (O=), thio (S=), F, Cl, Br, cyano, hydroxyl, amino, C₁₋₄ alkyl, C₁₋₄ alkoxy, and C₁₋₄ alkylamino;
preferably, R^{d2} is independently hydrogen, F, Cl, Br, cyano, hydroxyl, amino, or C₁₋₄ alkyl, wherein the C₁₋₄ alkyl is optionally substituted with 1, 2, 3, 4, or 5 substituents selected from deuterium, F, Cl, Br, cyano, hydroxyl, amino, and C₁₋₄ alkyl;
m3 is 0, 1, 2, 3, or 4; preferably, m3 is 0, 1, or 2.

13. The compound according to any one of claims 1-12 having a structure selected from the following, or an enantiomer, a diastereoisomer, a racemate, a tautomer, a stereoisomer, a geometric isomer, a nitrogen oxide, a metabolite, a pharmaceutically acceptable salt, an ester, a solvate, a hydrate, an isotope-labeled compound, or a prodrug thereof:

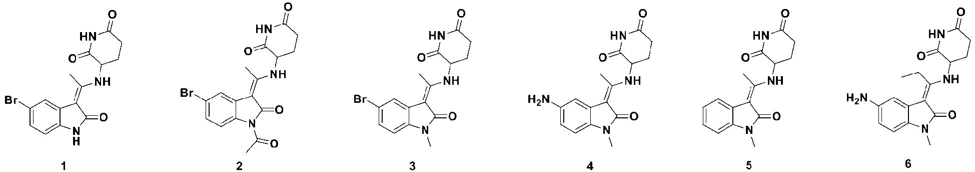

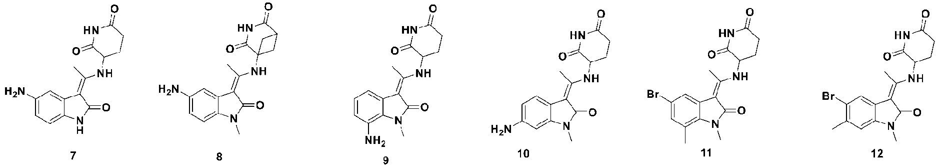

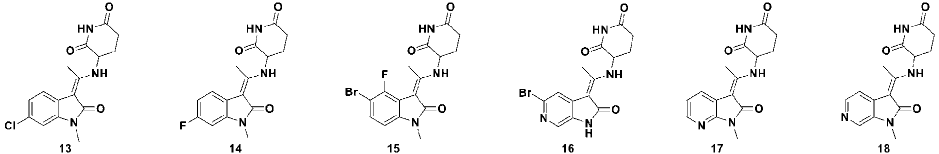

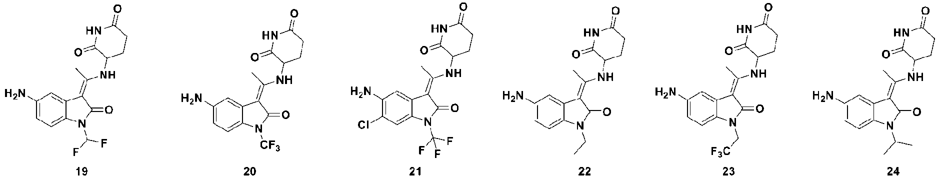

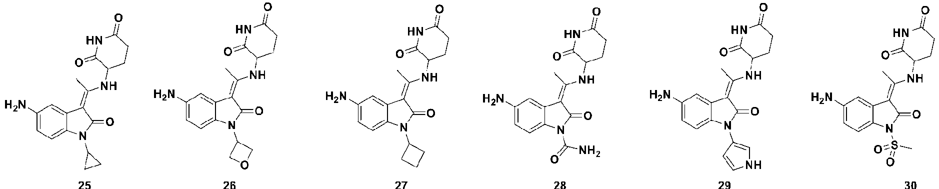

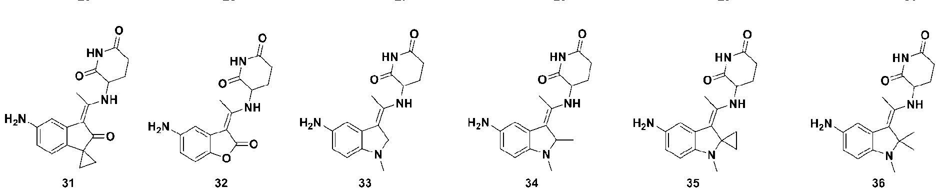

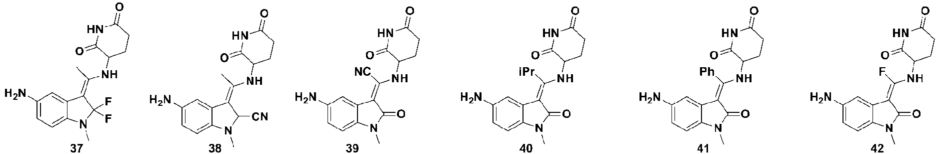

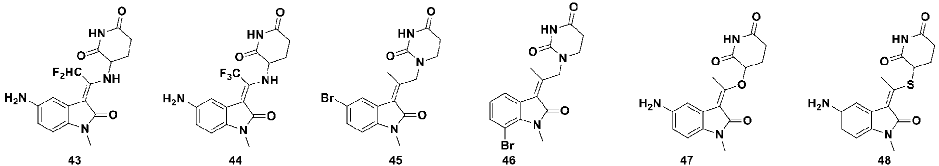

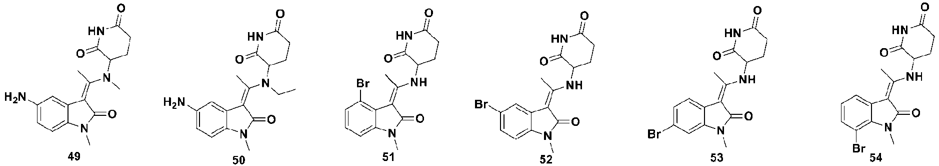

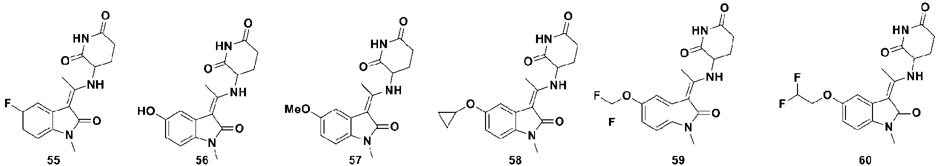

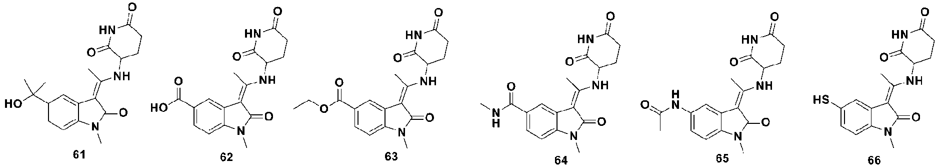

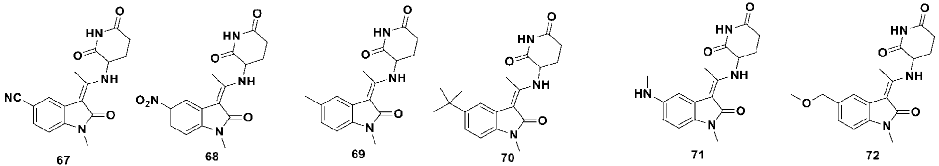

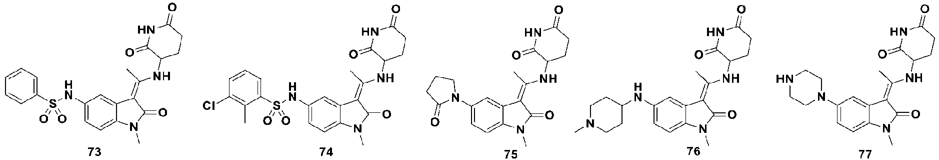

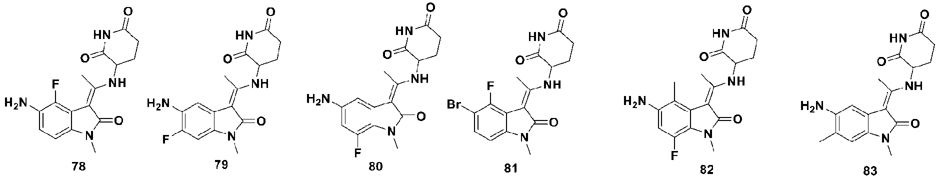

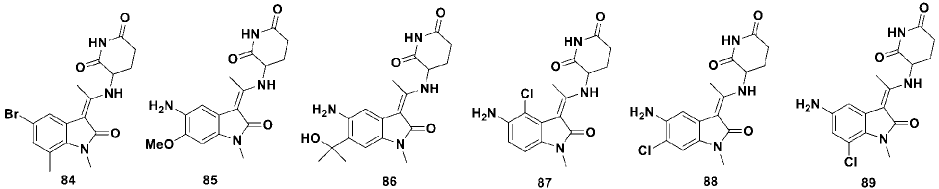

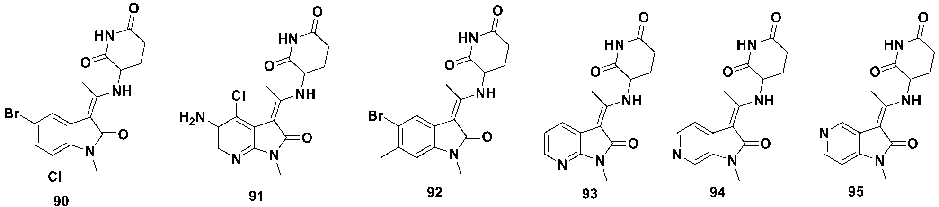

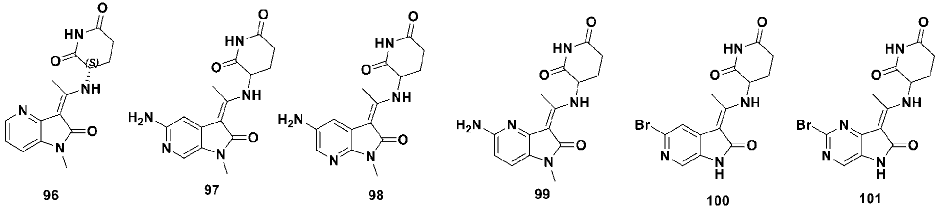

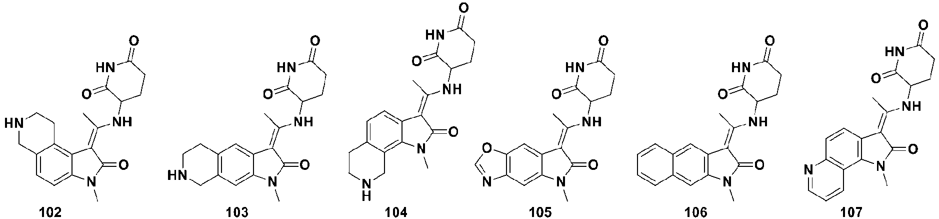

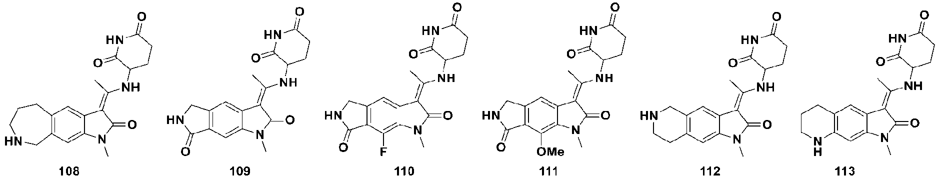

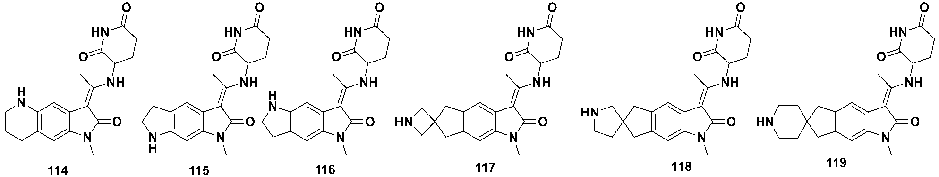

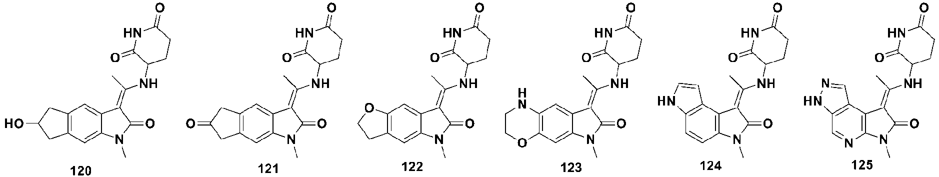

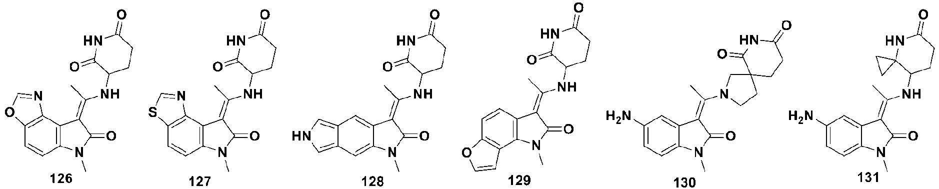

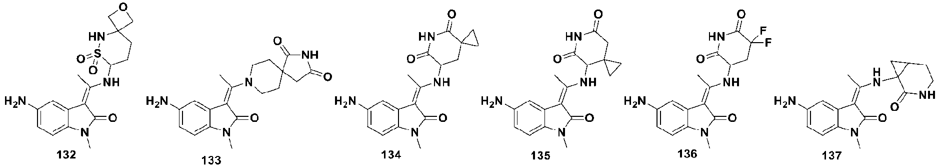

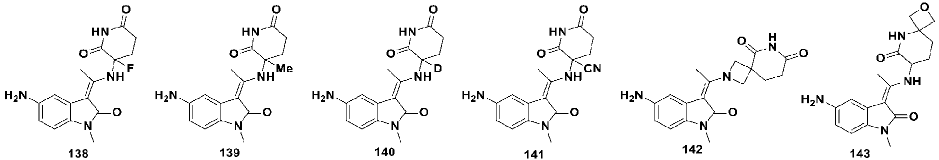

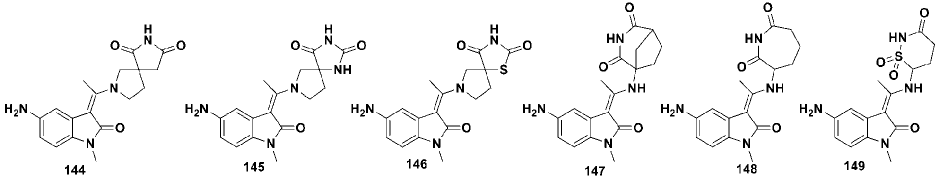

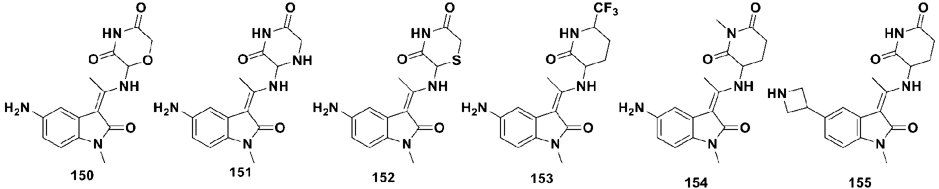

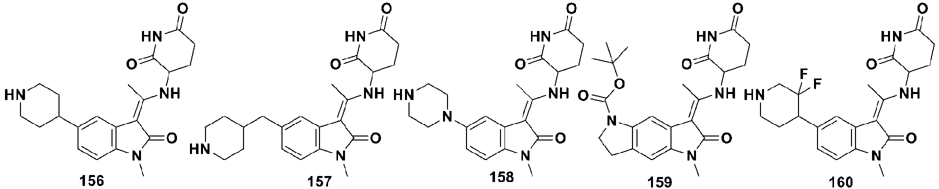

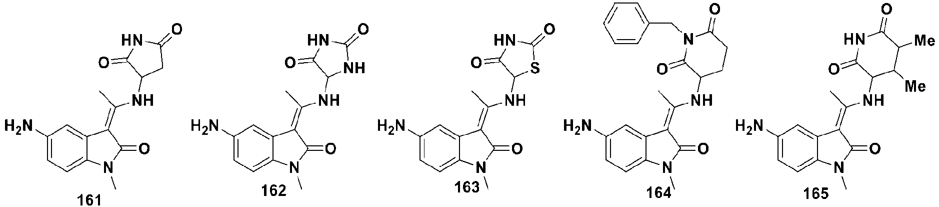

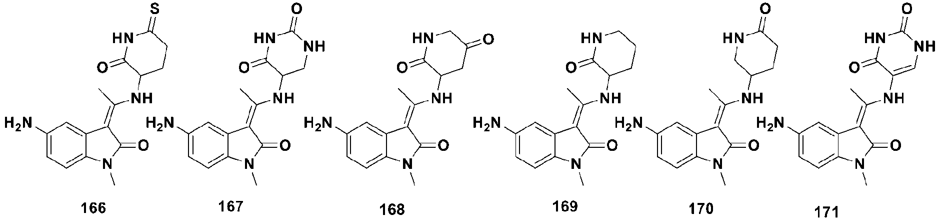

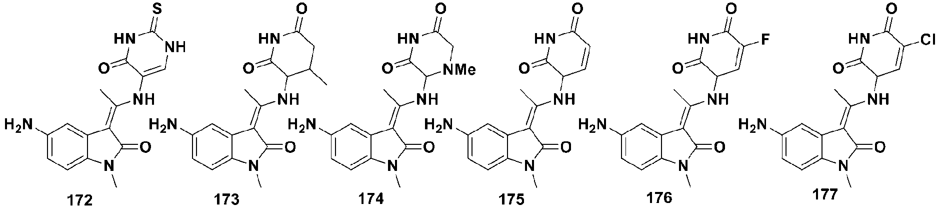

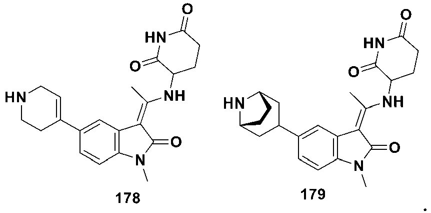

14. A PROTAC molecule, comprising a ligand capable of binding to CRBN, wherein the ligand comprises or is based on any compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of claims 1-13.

15. A pharmaceutical composition, comprising: the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of claims 1-13, or the PROTAC molecule according to claim 14; and at least one pharmaceutically acceptable carrier.

16. The compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of claims 1-13, or the PROTAC molecule according to claim 14, or the pharmaceutical composition according to claim 15 for use in treating a CRBN-mediated disease or disorder.

17. Use of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of claims 1-13, or the PROTAC molecule according to claim 14, or the pharmaceutical composition according to claim 15 in the preparation of a medicament, wherein the medicament is used for treating a CRBN-mediated disease or disorder.

18. A method for treating a CRBN-mediated disease or disorder, comprising administering to a human in need thereof a therapeutically effective amount of the compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to any one of claims 1-13, or the PROTAC molecule according to claim 14, or the pharmaceutical composition according to claim 15.

19. The compound, or the enantiomer, the diastereoisomer, the racemate, the tautomer, the stereoisomer, the geometric isomer, the nitrogen oxide, the metabolite, the pharmaceutically acceptable salt, the ester, the solvate, the hydrate, the isotope-labeled compound, or the prodrug thereof according to claim 16, or the use in the preparation of the medicament according to claim 17, or the method for treating the CRBN-mediated disease or disorder according to claim 18, wherein the CRBN-mediated disease or disorder comprises a proliferative disorder, a neurological disorder, and a transplantation-related disorder.
